(19) Europäisches Patentamt
European Patent Office
Office européen des brevets

(11) EP 3 380 496 B1

(12) **EUROPEAN PATENT SPECIFICATION**

(45) Date of publication and mention of the grant of the patent:
**20.05.2020 Bulletin 2020/21**

(21) Application number: **16831675.0**

(22) Date of filing: **21.12.2016**

(51) Int Cl.:
***C07K 14/32*** (2006.01) ***C12P 1/04*** (2006.01)

(86) International application number:
**PCT/US2016/067936**

(87) International publication number:
**WO 2017/112733 (29.06.2017 Gazette 2017/26)**

# (54) ENHANCED PROTEIN PRODUCTION AND METHODS THEREOF

ERHÖHTE PROTEINPRODUKTION UND VERFAHREN DAFÜR

PRODUCTION DE PROTÉINE AMÉLIORÉE ET PROCÉDÉS ASSOCIÉS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.12.2015 US 201562387578 P**
**15.03.2016 US 201662308440 P**

(43) Date of publication of application:
**03.10.2018 Bulletin 2018/40**

(73) Proprietor: **Danisco US Inc.**
**Palo Alto, California 94304 (US)**

(72) Inventors:
- **BONGIORNI, Cristina**
  **Palo Alto, CA 94304 (US)**
- **NEEF, Jolanda**
  **Palo Alto, CA 94304 (US)**
- **SCHMIDT, Brian F.**
  **Palo Alto, CA 94304 (US)**
- **VAN KIMMENADE, Anita**
  **Palo Alto, CA 94304 (US)**
- **VAN DIJL, Jan Maarten**
  **Palo Alto, CA 94304 (US)**

(74) Representative: **Mewburn Ellis LLP**
**Aurora Building**
**Counterslip**
**Bristol BS1 6BX (GB)**

(56) References cited:
**US-A1- 2002 031 807**

- **J. HEINRICH ET AL: "The Bacillus subtilis ABC transporter EcsAB influences intramembrane proteolysis through RasP", MICROBIOLOGY, vol. 154, no. 7, 1 July 2008 (2008-07-01), pages 1989-1997, XP055351767, GB ISSN: 1350-0872, DOI: 10.1099/mic.0.2008/018648-0**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 380 496 B1

## Description

### CROSS REFERENCE TO RELATED APPLICATIONS

This application claims the benefit of U.S. Provisional Application No. 62/387,578, filed December 23, 2015 and U.S. Provisional Application No. 62/308,440, filed March 15, 2016.

### FIELD

The present disclosure is generally related to the fields of bacteriology, microbiology, genetics, molecular biology, enzymology and the like. Certain embodiments are directed to modified Gram-positive bacterial cells expressing and producing increased amounts of one or more protein(s) of interest.

### REFERENCE TO THE SEQUENCE LISTING

The contents of the electronic submission of the text file Sequence Listing, named "NB40883WOPCT_SequenceListing.txt" was created on November 16, 2016 and is 39 KB in size.

### BACKGROUND

The production of proteins (*e.g.,* enzymes, antibodies, receptors, *etc.*) in microbial hosts is of particular interest in the biotechnological arts. Likewise, optimization of microbial host cells for the production and secretion of one or more protein(s) of interest is of high relevance, particularly in the industrial biotechnology setting, wherein small improvements in protein yield are quite significant when the protein is produced in large industrial quantities.

Gram-positive bacteria such as *Bacillus subtilis, Bacillus licheniformis* and *Bacillus amyloliquefaciens* are frequently used as microbial factories for the production of industrial relevant proteins, due to their excellent fermentation properties and high yields (up to 25 grams per liter culture; Van Dijl and Hecker, 2013). For example, *B. subtilis* is well known for its production of $\alpha$-amylases (Jensen *et al.,* 2000; Raul *et al.,* 2014) and proteases (Brode *et al.,* 1996) necessary for food, textile, laundry, pharmaceutical industries and the like (Westers *et al.,* 2004). Because these non-pathogenic Gram-positive bacteria produce proteins that completely lack toxic by-products (*e.g.*, lipopolysaccharides; LPS, also known as endotoxins) they have obtained the "Qualified Presumption of Safety" (QPS) status of the European Food Safety Authority, and many of their products gained a "Generally Recognized As Safe" (GRAS) status from the US Food and Drug Administration (Olempska-Beer *et al.,* 2006; Earl *et al.,* 2008; Caspers *et al.,* 2010).

The Gram-positive bacterium *B. subtilis* uses various pathways to secrete proteins, of which the Tat-machinery (Raul *et al.,* 2014; Goosens *et al.,* 2014; Tjalsma *et al.,* 2004) and the Sec-machinery (Tjalsma *et al.,* 2004) are the two best studied. The majority of proteins are secreted *via* the Sec-pathway (Harwood and Cranenburgh, 2007) in an unfolded (non-native) conformation, wherein the hydrophobic signal peptide directs the protein to the translocation machinery of the cell. Shortly after translocation *via* the SecYEG translocon, promoted by the ATPase SecA, the signal peptide of the protein is liberated (cleaved) by one of the signal peptidases SipS-SipW (Tjalsma *et al.,* 1997) and subsequently degraded by the signal peptide peptidases TepA and SppA (Bolhuis *et al.,* 1999).

In the past 30 years, numerous studies have been performed to improve the secretion of heterologous proteins by *B. subtilis, B. licheniformis, B. amyloliquefaciens* and the like. The major targets for this optimization have been the composition of the signal peptides, the SRP, the translocation machinery, the signal peptidases and regulatory factors (Kang *et al.,* 2014). Recently it was shown that overexpression of the essential protein PrsA (involved in post-translocational folding) and the chaperone DnaK resulted in improved secretion of amylases in *B. subtilis* (Chen *et al.,* 2015), demonstrating the impact of proper folding on efficient protein secretion, wherein PrsA was postulated to be the limiting factor for efficient protein secretion (Kontinen and Sarvas, 1993). Furthermore, it was recently demonstrated that PrsA can be degraded by WprA and other proteases located in the cell wall (Krishnappa *et al.,* 2014), rendering it less suitable for industrial use.

Thus, due to the high commercial/industrial use of these microbial cell factories, new targets for the improvement of secretion and/or production of industrially relevant proteins in Gram-positive bacteria are highly desirable in the biotechnological arts. The present disclosure addresses and fulfils the unmet need for increased production and/or secretion of proteins of interest in Gram-positive bacterial cells. More particularly, as set forth below in the Detailed Description, the instant disclosure is directed to the surprising and unexpected findings that Gram-positive bacterial cells modified to express, or over-express, the *rasP* gene, encoding the "regulating anti-sigma factor protease" or "RasP" (formerly known as YluC), results in increased production of one or more protein(s) of interest from the modified Gram-positive bacterial cells. Heinrich et al. discloses that over-expression of RasP can restore RasP activity, but does not disclose a method for increasing the production of one or more protein(s) of interest, nor the modified cell produced by these methods (Heinrich et al., 2008).

## SUMMARY

In certain embodiments the present disclosure is directed to modified Gram-positive bacterial cells producing an increased amount of a protein of interest (hereinafter, a "POI") relative to an unmodified (parental) Gram-positive bacterial cell, wherein the modified bacterial cell comprises a modification which increases *rasP* gene expression. In certain embodiments, the modification which increases *rasP* gene expression is a modification to an endogenous chromosomal *rasP* gene. In other embodiments, the native promoter of the endogenous chromosomal *rasP* gene is substituted with any promoter having a higher activity than the native *rasP* promoter. In certain other embodiments, the native promoter of the endogenous chromosomal *rasP* gene is substituted with a *spoVG* promoter or an *aprE* promoter. In yet other embodiments, the *spoVG* promoter comprises a nucleotide sequence comprising 95% sequence identity to SEQ ID NO: 3. In other embodiments, the *aprE* promoter comprises a nucleotide sequence comprising 95% sequence identity to SEQ ID NO: 4.

In certain other embodiments, the modification to an endogenous chromosomal *rasP* gene is a modification of the native 5'-untranslated region (5'-UTR) of the endogenous chromosomal *rasP* gene. In other embodiments, the native *rasP* chromosomal 5'-UTR is replaced with a 5'-UTR comprising 95% sequence identity to the *aprE* 5'-UTR of SEQ ID NO: 5. In yet other embodiments, the modification to an endogenous chromosomal *rasP* gene is a modification of both the native promoter and the native 5'-UTR of the endogenous chromosomal *rasP* gene.

In certain other embodiments, the modification which increases *rasP* gene expression is an exogenous polynucleotide comprising a *rasP* gene. In particular embodiments, the exogenous polynucleotide comprising the *rasP* gene is comprised within an extrachromosomal plasmid. In another embodiment, the extrachromosomal plasmid is an expression cassette. In other embodiments, the extrachromosomal plasmid is an integration plasmid. In certain other embodiments, the plasmid stably integrates into the chromosome of the modified cell.

In other embodiments, the genetic modification increasing *rasP* expression is a polynucleotide comprising an exogenous *rasP* open reading frame (ORF), wherein the ORF is operably linked and under the control of a constitutive promoter, an inducible promoter or a conditional promoter. In certain embodiments, the exogenous polynucleotide comprising the *rasP* ORF is comprised within an extrachromosomal plasmid. In certain embodiments, the extrachromosomal plasmid is an expression cassette. In certain other embodiments, the extrachromosomal plasmid is an integration plasmid. In particular embodiments, plasmid integrates into the chromosome of the modified cell.

In other embodiments, the *rasP* gene comprises a nucleic acid sequence comprising at least 60% sequence identity to open reading frame (ORF) nucleic acid sequence of SEQ ID NO: 1. In other embodiments, the ORF of SEQ ID NO: 1 encodes a RasP polypeptide, wherein the RasP polypeptide is further defined as a $Zn^{2+}$ metalloprotease having site-2 protease (S2P) activity. In another embodiment, the *rasP* gene encodes a RasP polypeptide comprising 60% amino acid sequence identity to a RasP polypeptide of SEQ ID NO: 2 and comprises an active site consensus sequence of SEQ ID NO: 6 (LVFFHELGHLL), which aligns with amino acid residues 16 to 26 of the RasP polypeptide of SEQ ID NO: 2. In yet other embodiments, the *rasP* gene encodes a RasP polypeptide comprising 80% amino acid sequence identity to a RasP polypeptide of SEQ ID NO: 2 and comprises an active site consensus sequence of SEQ ID NO: 7 (HEXXH), which aligns with amino acid residues 20 to 24 of the RasP polypeptide of SEQ ID NO: 2.

In other embodiments, the increased amount of the POI produced, relative to the unmodified (parental) Gram-positive cell, is at least a 5% increase. In another embodiment, the increased amount of the POI produced, relative to the unmodified (parental) Gram-positive cell, is at least a 10% increase.

In certain other embodiments, the Gram-positive bacterial cell is a member of the *Bacillus* genus. In yet other embodiments, the *Bacillus* is selected from *B. subtilis, B. licheniformis, B. lentus, B. brevis, B. stearothermophilus, B. alkalophilus, B. amyloliquefaciens, B. clausii, B. sonorensis, B. halodurans, B. pumilus, B. lautus, B. pabuli, B. cereus, B. agaradhaerens, B akibai, B. clarkii, B. pseudofirmus, B. lehensis, B. megaterium, B. coagulans, B. circulans, B. gibsonii, B. marmaresis,* and *B. thuringiensis.* In another embodiment, the *Bacillus* is *B. subtilis* or *B. licheniformis.*

In certain other embodiments, the POI is encoded by a gene exogenous to the modified bacterial cell or a gene endogenous to the modified bacterial cell. In other instances, the POI is secreted or transported extracellularly. As described herein, the POI secreted or transported extracellularly may be further isolated and purified.

In certain other embodiments, the POI is an enzyme. In other embodiments, the enzyme is selected from the group consisting of acetyl esterases, aryl esterases, aminopeptidases, amylases, arabinases, arabinofuranosidases, carboxypeptidases, catalases, cellulases, chitinases, chymosin, cutinase, deoxyribonucleases, epimerases, esterases, α-galactosidases, α-glucanases, glucan lysases, endo-β-glucanases, glucoamylases, glucose oxidases, α-glucosidases, β-glucosidases, glucuronidases, hemicellulases, hexose oxidases, hydrolases, invertases, isomerases, laccases, lipases, lyases, mannosidases, oxidases, oxidoreductases, pectate lyases, pectin acetyl esterases, pectin depolymerases, pectin methyl esterases, pectinolytic enzymes, perhydrolases, polyol oxidases, peroxidases, phenoloxidases, phytases, polygalacturonases, proteases, rhamno-galacturonases, ribonucleases, thaumatin, transferases, transport proteins, transglutaminases, xylanases, hexose oxidases, and combinations thereof.

In certain other embodiments, the disclosure is directed to a method for increasing the production of a POI in a Gram-positive bacterial cell comprising (a) obtaining a modified Gram-positive bacterial cell producing an increased amount of a POI, wherein the modified bacterial cell comprises a modification which increases *rasP* gene expression, and (b) culturing the modified cell under conditions such that the POI is expressed, wherein the modified bacterial cell producing an increased amount of a POI is relative to the production of the same POI in an unmodified (parental) Gram-positive bacterial cell.

In other embodiments, the disclosure is directed to a method for obtaining a modified Gram-positive bacterial cell producing an increased amount of a POI comprising (a) introducing into a parental Gram-positive bacterial cell at least one gene modification which increases *rasP* gene expression, and (b) selecting one or more daughter cells expressing an increased amount of a POI, wherein the one or more daughter cells selected for producing an increased amount of the POI are defined as modified (daughter) Gram-positive bacterial cells.

## BRIEF DESCRIPTION OF DRAWINGS

**Figure 1** compares the expression of AmyE amylase, AmyL amylase and protease BPN'-Y217L secreted into the culture medium from modified *B. subtilis* cells comprising either the deleted *tepA* gene (Δ*tepA*) or the deleted *rasP* gene (Δ*rasP*) relative to unmodified (parental; wild-type) *B. subtilis* cells. **FIG. 1A** shows the LDS-PAGE gels of the secreted enzymes (AmyE, AmyL and BPN'-Y217L) from the *B. subtilis* cells lacking the *rasP* or *tepA* genes compared to the *B. subtilis* cells (parental) cells. **FIG. 1B** shows the quantification of the secreted AmyE, AmyL and BPN'-Y217L proteins using ImageJ, presented as the ratio of protein secreted from *B. subtilis* cells comprising a deleted rasP (Δ*rasP*) or tepA (Δ*tepA*) gene relative to the ratio of protein secreted from unmodified (parental) wild-type *B. subtilis* cells (*i.e.,* Δ*tepA* cells/wt cells and Δ*rasP*/wt cells).

**Figure 2** shows the cell densities measured at OD600 **(FIG. 2A)** and production **(FIG. 2B)** of AmyE from wild-type (parental) *B. subtilis* cells, modified *B. subtilis* cells comprising and expressing/over-expressing the *rasP* gene under the control of the *spoVG* promoter (*i.e.,* the "PspoVG-rasP" expression cassette) and modified *B. subtilis* cells comprising and expressing/over-expressing the *rasP* gene under the control of the *spoVG* promoter and the *aprE* 5'UTR (*i.e.,* the "PspoVG-UTR-rasP" expression cassette).

**Figure 3** shows shake flask production of amylase PcuAmy1-v6 from wild-type (parental) *B. subtilis* cells and modified *B. subtilis* cells comprising and expressing/over-expressing the *rasP* gene under the control of the *spoVG* promoter (*i.e.,* the "PspoVG-rasP" expression cassette).

**Figure 4** shows the cell densities **(FIG. 4A)** and production **(FIG. 4B)** of amylase PcuAmy1-v6 from wild-type (parental) *B. subtilis* cells and modified *B. subtilis* cells comprising and expressing/over-expressing the *tepA* gene under the control of the *spoVG* promoter *(i.e.,* the "PspoVG-tepA" expression cassette).

**Figure 5** shows the production of Beta-D-glucanase (BglC) from wild-type (parental) *B. subtilis* cells and modified *B. subtilis* cells comprising and expressing/over-expressing the *rasP* gene under the control of the *spoVG* promoter *(i.e.,* the "PspoVG-rasP" expression cassette).

**Figure 6** shows the cell densities **(FIG. 6A)** and production **(FIG. 6B)** of Properase from wild-type (parental) *B. subtilis* cells and modified *B. subtilis* cells comprising and expressing/over-expressing the *rasP* gene under the control of the *spoVG* promoter *(i.e.,* the "PspoVG-rasP" expression cassette).

**Figure 7** shows the cell densities **(FIG. 7A)** and production **(FIG. 7B)** of the "AmyAc family" α-amylase expressed from wild-type (parental) *B. subtilis* cells and modified (daughter) *B. subtilis* comprising and expressing/over-expressing the *rasP gene* under the control of the *spoVG* promoter *(i.e.,* the "PspoVG-rasP" expression cassette). The parental and modified (daughter) *B. subtilis* cells used in this experiment were grown in deep well microtiter plates (DWMTP) using 5SM12 growth medium.

## DETAILED DESCRIPTION

The present disclosure is generally related to modified Gram-positive bacterial cells producing increased amounts of one or more protein(s) of interest (hereinafter, a "POI"). Thus, certain embodiments of the instant disclosure are directed to modified Gram-positive bacterial cells producing an increased amount of a POI relative to unmodified (parental) Gram-positive bacterial cells producing the same POI, wherein the modified bacterial cells comprise a modification which increases *rasP* gene expression.

As set forth herein, the *rasP* gene encodes the "regulating anti-sigma factor protease" or "RasP" (formerly known as YluC). RasP belongs to a group of zinc-dependent intramembrane cleaving proteases (or "iClips) and is further defined herein as a site-2 protease (S2P) or site-2 zinc metalloprotease *(see, e.g.,* Saito *et al.,* 2011; Heinrich *et al.,*

2008). In certain embodiments, a RasP polypeptide of the disclosure is further defined as a hydrolase (*e.g.,* a peptidase), and more particularly a metalloendopeptidase, belonging to Enzyme Commission (EC) class 3.4.24 (EC 3.4.24).

In view of the modified bacterial cells, compositions thereof and methods thereof described herein, the following terms and phrases are defined. Terms not defined herein should be accorded their ordinary meaning as used in the art.

Unless defined otherwise, all technical and scientific terms used herein have the same meaning as commonly understood by one of ordinary skill in the art to which the present compositions and methods apply. Although any methods and materials similar or equivalent to those described herein can also be used in the practice or testing of the present compositions and methods, representative illustrative methods and materials are now described.

It is further noted that the claims may be drafted to exclude any optional element. As such, this statement is intended to serve as antecedent basis for use of such exclusive terminology as "solely," "only", "excluding" and the like, in connection with the recitation of claim elements, or use of a "negative" limitation.

As will be apparent to those of skill in the art upon reading this disclosure, each of the individual embodiments described and illustrated herein has discrete components and features which may be readily separated from or combined with the features of any of the other several embodiments without departing from the scope or spirit of the present compositions and methods described herein. Any recited method can be carried out in the order of events recited or in any other order which is logically possible.

As defined herein, a "modified cell", a "modified bacterial cell" or a "modified host cell" may be used interchangeably and refer to recombinant Gram-positive bacterial (host) cells that comprise a modification (*e.g.,* a genetic modification) which increases *rasP* gene expression. For example, a "modified" Gram-positive bacterial cell of the instant disclosure may be further defined as a "modified (host) cell" which is derived from a parental bacterial cell, wherein the modified (daughter) cell comprises a modification which increases *rasP* gene expression.

As defined herein, an "unmodified cell", an "unmodified bacterial cell" or an "unmodified host cell" may be used interchangeably and refer to "unmodified" (parental) Gram-positive bacterial cells that do not comprise a modification which increases *rasP gene* expression.

As used herein, when the expression and/or production and/or secretion of a POI in an "unmodified" (parental) cell is being compared to the expression and/or production and/or secretion of the same POI in a "modified" (daughter) cell, it will be understood that the "modified" and "unmodified" cells are grown/cultured/fermented under essentially the same conditions (*e.g.,* the same conditions such as media, temperature, pH and the like).

Likewise, as defined herein, the terms "increased production", "enhanced production", "increased production of a POI", "enhanced production of a POI", and the like refer to a "modified" (daughter) cell comprising a modification which increases *rasP* gene expression, wherein the "increase" is always relative (vis-à-vis) to an "unmodified" (parental) cell expressing the same POI.

As used herein, "nucleic acid" refers to a nucleotide or polynucleotide sequence, and fragments or portions thereof, as well as to DNA, cDNA, and RNA of genomic or synthetic origin, which may be double-stranded or single-stranded, whether representing the sense or antisense strand. It will be understood that as a result of the degeneracy of the genetic code, a multitude of nucleotide sequences may encode a given protein.

It is understood that the polynucleotides (or nucleic acid molecules) described herein include "genes", "vectors" and "plasmids". Accordingly, the term "gene", refers to a polynucleotide that codes for a particular sequence of amino acids, which comprise all, or part of a protein coding sequence, and may include regulatory (non-transcribed) DNA sequences, such as promoter sequences, which determine for example the conditions under which the gene is expressed. The transcribed region of the gene may include untranslated regions (UTRs), including introns, 5'-untranslated region (UTR), and 3'-UTR, as well as the coding sequence.

As used herein, the term "coding sequence" refers to a nucleotide sequence, which directly specifies the amino acid sequence of its (encoded) protein product. The boundaries of the coding sequence are generally determined by an open reading frame, which usually begins with an ATG start codon. The coding sequence typically includes DNA, cDNA, and recombinant nucleotide sequences.

As defined herein, the term "open reading frame" (hereinafter, "ORF") means a nucleic acid or nucleic acid sequence (whether naturally occurring, non-naturally occurring, or synthetic) comprising an uninterrupted reading frame consisting of (i) an initiation codon, (ii) a series of two (2) or more codons representing amino acids, and (iii) a termination codon, the ORF being read (or translated) in the 5' to 3' direction. For example, in certain embodiments, a modified cell, a modified bacterial cell or a modified host cell that comprises a modification which "increases *rasP* gene expression" encompasses recombinant Gram-positive bacterial cells that comprise an ORF encoding a RasP polypeptide, wherein the expression of the ORF sequence encoding the RasP polypeptide is operably linked and under the control of a constitutive promoter, an inducible promoter, a conditional promoter, and the like; and optionally comprise 5' and 3' regulatory (non-transcribed) nucleic acid sequences.

The term "promoter" as used herein refers to a nucleic acid sequence capable of controlling the expression of a coding sequence or functional RNA. In general, a coding sequence is located 3' (downstream) to a promoter sequence. Promoters may be derived in their entirety from a native gene, or be composed of different elements derived from different promoters found in nature, or even comprise synthetic nucleic acid segments. It is understood by those skilled in the art that different promoters may direct the expression of a gene in different cell types, or at different stages of development, or in response to different environmental or physiological conditions. Promoters which cause a gene to be expressed in most cell types at most times are commonly referred to as "constitutive promoters". It is further recognized that since in most cases the exact boundaries of regulatory sequences have not been completely defined, DNA fragments of different lengths may have identical promoter activity.

The term "operably linked" as used herein refers to the association of nucleic acid sequences on a single nucleic acid fragment so that the function of one is affected by the other. For example, a promoter is operably linked with a coding sequence (*e.g.,* an ORF) when it is capable of affecting the expression of that coding sequence *(i.e.,* that the coding sequence is under the transcriptional control of the promoter). Coding sequences can be operably linked to regulatory sequences in sense or antisense orientation.

As defined herein, "suitable regulatory sequences" refer to nucleotide sequences located upstream (5' non-coding sequences), within, or downstream (3' non-coding sequences) of a coding sequence, and which influence the transcription, RNA processing or stability, or translation of the associated coding sequence. Regulatory sequences may include promoters, translation leader sequences, RNA processing sites, effector binding sites, stem-loop structures and the like.

As defined herein, the term "introducing", as used in phrases such as "introducing into the bacterial cell" at least one polynucleotide open reading frame (ORF), or a gene thereof, or a vector thereof, includes methods known in the art for introducing polynucleotides into a cell, including, but not limited to protoplast fusion, transformation (*e.g.,* calcium chloride, electroporation), transduction, transfection, conjugation and the like *(e.g., see* Ferrari *et al.,* 1989).

As used herein, "transformed" or "transformation" mean a cell has been transformed by use of recombinant DNA techniques. Transformation typically occurs by insertion of one or more nucleotide sequences (*e.g.,* a polynucleotide, an ORF or gene) into a cell. The inserted nucleotide sequence may be a heterologous nucleotide sequence (*i.e.,* a sequence that is not naturally occurring in the cell that is to be transformed. As used herein, "transformation" refers to the transfer of a nucleic acid molecule into the genome of a host organism, resulting in genetically stable inheritance of the transferred nucleic acid molecule.

As defined herein, a host cell "genome", a bacterial (host) cell "genome", or a Gram-positive bacterial (host) cell "genome" includes chromosomal and extrachromosomal genes.

As used herein, the terms "plasmid", "vector" and "cassette" refer to extrachromosomal elements, often carrying genes which are typically not part of the central metabolism of the cell, and usually in the form of circular double-stranded DNA molecules. Such elements may be autonomously replicating sequences, genome integrating sequences, phage or nucleotide sequences, linear or circular, of a single-stranded or double-stranded DNA or RNA, derived from any source, in which a number of nucleotide sequences have been joined or recombined into a unique construction which is capable of introducing a promoter fragment and DNA sequence for a selected gene product along with appropriate 3' untranslated sequence into a cell.

A "transformation cassette" refers to a specific vector comprising a foreign gene (or an ORF thereof), and having elements in addition to the foreign gene that facilitate transformation of a particular host cell.

An "expression cassette" refers to a specific vector comprising a foreign gene (or an ORF thereof), and having elements in addition to the foreign gene that allow for "increased" expression of the foreign (heterologous) gene in a host cell.

Many prokaryotic and eukaryotic expression vectors are commercially available and well known in the art. Selection of appropriate expression vectors is within the knowledge of those having skill in the art.

As used herein, the term "vector" is any means by which a nucleic acid can be propagated and/or transferred between organisms, cells, or cellular components. Vectors include viruses, bacteriophage, pro-viruses, plasmids, phagemids, transposons, and artificial chromosomes such as YACs (yeast artificial chromosomes), BACs (bacterial artificial chromosomes), and PLACs (plant artificial chromosomes), and the like, that are "episomes", that is, that replicate autonomously or can integrate into a chromosome of a host microorganism.

An "expression vector" refers to a vector that has the ability to incorporate and express heterologous DNA in a foreign cell. Many prokaryotic and eukaryotic expression vectors are commercially available.

A "targeting vector" is a vector that includes polynucleotide sequences that are homologous to a region in the chromosome of a host cell into which it is transformed and that can drive homologous recombination at that region. Targeting vectors find use in introducing mutations or exogenous gene sequences into the chromosome of a cell through homologous recombination. In some embodiments, the targeting vector comprises other non-homologous sequences, *e.g.,* added to the ends *(i.e.,* stuffer sequences or flanking sequences). The ends can be closed such that the targeting vector forms a closed circle, such as, for example, insertion into a vector. Selection and/or construction of appropriate vectors is within the knowledge of those having skill in the art.

As used herein, the term "plasmid" refers to a circular double-stranded (ds) DNA construct used as a cloning vector, and which forms an extrachromosomal self-replicating genetic element in many bacteria and some eukaryotes.

In some embodiments, plasmids become incorporated into the genome of the host cell.

As used herein, the term "protein of interest" or "POI" refers to a polypeptide of interest that is desired to be expressed in a modified Gram-positive bacterial cell (*e.g.,* a "host" cell), wherein the POI is produced at increased levels (*i.e.,* relative to an unmodified (parental) Gram-positive bacterial cell). Thus, as used herein, a POI may be an enzyme, a substrate-binding protein, a surface-active protein, a structural protein, a receptor protein, an antibody and the like.

Similarly, as defined herein, a "gene of interest" or "GOI" refers to a nucleic acid sequence (*e.g.*, a polynucleotide, a gene or an open reading frame) which encodes a POI. A GOI encoding a POI may be a naturally occurring gene, a mutated gene or a synthetic gene.

As used herein, the terms "polypeptide" and "protein" are used interchangeably, and refer to polymers of any length comprising amino acid residues linked by peptide bonds. The conventional one (1) letter or three (3) letter codes for amino acid residues are used herein. The polypeptide may be linear or branched, it may comprise modified amino acids, and it may be interrupted by non-amino acids.

The term polypeptide also encompasses an amino acid polymer that has been modified naturally or by intervention; for example, disulfide bond formation, glycosylation, lipidation, acetylation, phosphorylation, or any other manipulation or modification, such as conjugation with a labeling component. Also included within the definition are, for example, polypeptides containing one or more analogs of an amino acid (including, for example, unnatural amino acids, *etc*.), as well as other modifications known in the art.

In certain embodiments, a gene of the instant disclosure encodes a commercially relevant industrial protein of interest, such as an enzyme (*e.g*., a acetyl esterases, aryl esterases, aminopeptidases, amylases, arabinases, arabinofuranosidases, carbonic anhydrases, carboxypeptidases, catalases, cellulases, chitinases, chymosins, cutinases, deoxyribonucleases, epimerases, esterases, α-galactosidases, α-glucanases, glucan lysases, endo-β-glucanases, glucoamylases, glucose oxidases, α-glucosidases, β-glucosidases, glucuronidases, glycosyl hydrolases, hemicellulases, hexose oxidases, hydrolases, invertases, isomerases, laccases, lipases, lyases, mannosidases, oxidases, oxidoreductases, pectate lyases, pectin acetyl esterases, pectin depolymerases, pectin methyl esterases, pectinolytic enzymes, perhydrolases, polyol oxidases, peroxidases, phenoloxidases, phytases, polygalacturonases, proteases, peptidases, rhamno-galacturonases, ribonucleases, transferases, transport proteins, transglutaminases, xylanases, hexose oxidases, and combinations thereof.

As defined herein, an "endogenous gene" refers to a gene in its natural location in the genome of an organism.

As defined herein, a "heterologous" gene, a "non-endogenous" gene, or a "foreign" gene refer to a gene (or ORF) not normally found in the host organism, but that is introduced into the host organism by gene transfer. Foreign (heterologous) genes comprise native genes (or ORFs) inserted into a non-native organism and/or chimeric genes inserted into a native or non-native organism.

As used herein, the term "expression" refers to the transcription and stable accumulation of sense (mRNA) or anti-sense RNA, derived from a nucleic acid molecule of the disclosure. Expression may also refer to translation of mRNA into a polypeptide. Thus, the term "expression" includes any step involved in the production of the polypeptide including, but not limited to, transcription, post- transcriptional modification, translation, post-translational modification, secretion and the like.

As used herein, a "variant" polypeptide refers to a polypeptide that is derived from a parent (or reference) polypeptide by the substitution, addition, or deletion of one or more amino acids, typically by recombinant DNA techniques. Variant polypeptides may differ from a parent polypeptide by a small number of amino acid residues and may be defined by their level of primary amino acid sequence homology/identity with a parent (reference) polypeptide.

Preferably, variant polypeptides have at least 70%, at least 75%, at least 80%, at least 85%, at least 90%, at least 91%, at least 92%, at least 93%, at least 94%, at least 95%, at least 96%, at least 97%, at least 98%, or even at least 99% amino acid sequence identity with a parent (reference) polypeptide sequence. As used herein, a "variant" polynucleotide refers to a polynucleotide encoding a variant polypeptide, wherein the "variant polynucleotide" has a specified degree of sequence homology/identity with a parent polynucleotide, or hybridizes with a parent polynucleotide (or a complement thereof) under stringent hybridization conditions. Preferably, a variant polynucleotide has at least 70%, at least 75%, at least 80%, at least 85%, at least 90%, at least 91%, at least 92%, at least 93%, at least 94%, at least 95%, at least 96%, at least 97%, at least 98%, or even at least 99% nucleotide sequence identity with a parent (reference) polynucleotide sequence.

As used herein, a "mutation" refers to any change or alteration in a nucleic acid sequence. Several types of mutations exist, including point mutations, deletion mutations, silent mutations, frame shift mutations, splicing mutations and the like. Mutations may be performed specifically (*e.g., via* site directed mutagenesis) or randomly (*e.g., via* chemical agents, passage through repair minus bacterial strains).

As used herein, in the context of a polypeptide or an amino acid sequence thereof, the term "substitution" means the replacement (*i.e.,* substitution) of one amino acid with another amino acid.

As defined herein, a "heterologous" nucleic acid construct or a "heterologous" nucleic acid sequence has a portion of the sequence which is not native to the cell in which it is expressed.

As defined herein, a "heterologous control sequence", refers to a gene expression control sequence (*e.g.,* a promoter or enhancer) which does not function in nature to regulate (control) the expression of the GOI. Generally, heterologous nucleic acid sequences are not endogenous (native) to the cell, or a part of the genome in which they are present, and have been added to the cell, by infection, transfection, transformation, microinjection, electroporation, protoplast fusion and the like. A "heterologous" nucleic acid construct may contain a control sequence/DNA coding (ORF) sequence combination that is the same as, or different, from a control sequence/DNA coding sequence combination found in the native host cell.

As used herein, the terms "signal sequence" and "signal peptide" refer to a sequence of amino acid residues that may participate in the secretion or direct transport of a mature protein or precursor form of a protein. The signal sequence is typically located N-terminal to the precursor or mature protein sequence. The signal sequence may be endogenous or exogenous. A signal sequence is normally absent from the mature protein. A signal sequence is typically cleaved from the protein by a signal peptidase after the protein is transported.

As used herein, a "parental" cell refers to any cell or strain of microorganism in which the genome of the "parental" cell is modified (*e.g.,* one or more mutations introduced into the parental cell or one or more genes or ORFs introduced into the parental cell) to generate a modified "daughter" cell.

The term "derived" encompasses the terms "originated" "obtained," "obtainable," and "created," and generally indicates that one specified material or composition finds its origin in another specified material or composition, or has features that can be described with reference to the another specified material or composition.

As used herein, "increasing" protein production is meant an increased amount of protein produced. The protein may be produced inside the host cell, or secreted (or transported) into the culture medium. In certain embodiments, the protein of interest is produced into the culture medium.

Increased protein production may be detected for example, as higher maximal level of protein or enzymatic activity, such as protease activity, amylase activity, cellulase activity, hemicellulase activity and the like, or total extracellular protein produced by the modified (daughter) cell as compared to the unmodified (parental) cell.

As used herein, the term "homology" relates to homologous polynucleotides or polypeptides. If two or more polynucleotides or two or more polypeptides are homologous, this means that the homologous polynucleotides or polypeptides have a "degree of identity" of at least 60%, more preferably at least 70%, even more preferably at least 85%, still more preferably at least 90%, more preferably at least 95%, and most preferably at least 98%. Whether two polynucleotide or polypeptide sequences have a sufficiently high degree of identity to be homologous as defined herein, can suitably be investigated by aligning the two sequences using a computer program known in the art, such as "GAP" provided in the GCG program package (Program Manual for the Wisconsin Package, Version 8, August 1994, Genetics Computer Group, 575 Science Drive, Madison, Wisconsin, USA 53711) (Needleman and Wunsch, (1970).

As used herein, the term "percent (%) identity" refers to the level of nucleic acid or amino acid sequence identity between the nucleic acid sequences that encode a polypeptide or the polypeptide's amino acid sequences, when aligned using a sequence alignment program.

As used herein, "specific productivity" is total amount of protein produced per cell per time over a given time period.

As defined herein, the terms "purified", "isolated" or "enriched" are meant that a biomolecule (*e.g.,* a polypeptide or polynucleotide) is altered from its natural state by virtue of separating it from some, or all of, the naturally occurring constituents with which it is associated in nature. Such isolation or purification may be accomplished by art-recognized separation techniques such as ion exchange chromatography, affinity chromatography, hydrophobic separation, dialysis, protease treatment, ammonium sulphate precipitation or other protein salt precipitation, centrifugation, size exclusion chromatography, filtration, microfiltration, gel electrophoresis or separation on a gradient to remove whole cells, cell debris, impurities, extraneous proteins, or enzymes undesired in the final composition. It is further possible to then add constituents to a purified or isolated biomolecule composition which provide additional benefits, for example, activating agents, anti-inhibition agents, desirable ions, compounds to control pH or other enzymes or chemicals.

## I. RasP POLYPEPTIDES AND GENES ENCODING THE SAME

As set forth previously above, the *rasP* gene encodes the "regulating anti-sigma factor protease" or "RasP" (formerly known as YluC); which belongs to a group of zinc-dependent intramembrane cleaving proteases (or "iClips) and is further defined herein as a site-2 protease (S2P) or site-2 zinc metalloprotease *(see, e.g.,* Saito *et al.,* 2011; Heinrich *et al.,* 2008). As defined herein, a "*rasP*" gene (or an ORF thereof) encodes a "RasP" polypeptide, and is not meant to be limited to a specific RasP polypeptide sequence (or *rasP* gene or ORF sequence encoding the same).

Thus, in certain embodiments, a *rasP* gene (or ORF thereof) of the instant disclosure may be any nucleic acid (polynucleotide) or any homologue or orthologue nucleic acid sequence thereof encoding a polypeptide characterized as having S2P $Zn^{2+}$ metalloprotease activity, as long as such encoded polypeptide (having S2P $Zn^{2+}$ metalloprotease activity) increases the production of a POI when the *rasP* nucleic acid or homologue or orthologue nucleic acid sequence thereof is expressed or over-expressed in a modified Gram-positive of the instant disclosure.

For example, the RasP polypeptide isolated from *B. subtilis* strain 168 (*e.g., see,* UniProtKB; RASP_BACSU; Accession No. 031754) comprises 422 amino acids (~46.7 kDa) set forth in SEQ ID NO: 2, wherein histidine amino acid residues at positions 20 and 24 are the putative $Zn^{2+}$ coordination/binding sites and the glutamic acid residue at position 21 is the proteolytic active site.

Thus, in certain embodiments, a gene (or ORF thereof) encoding a RasP polypeptide of the instant disclosure is a gene or ORF encoding a polypeptide comprising about 60% sequence identity to a RasP polypeptide set forth in SEQ ID NO: 2. For example, a BLAST search in the UniProtKB database (using *B. subtilis* RasP polypeptide sequence of SEQ ID NO: 2 as the subject sequence and the following search parameters: E-threshold = 10; Matrix = auto; gapped = yes) identified a range of RasP polypeptides comprising 100% to about 60% sequence identity to SEQ ID NO: 2.

In certain other embodiments, a gene (or ORF thereof) encoding a RasP polypeptide of the instant disclosure is a polypeptide comprising a contiguous active site consensus sequence of SEQ ID NO: 6 (LVFFH**E**LGHLL; wherein the underlined histidine amino acids are the $Zn^{2+}$ binding sites and the bold glutamic acid residue is the active site residue), wherein the amino acid sequence of the RasP polypeptide consensus sequence of SEQ ID NO: 6 can be aligned with the amino acid residues 16 to 26 of the RasP polypeptide of SEQ ID NO: 2 and result in at least 60% sequence homology.

In other embodiments, a gene (or ORF thereof) encoding a RasP polypeptide of the instant disclosure is a polypeptide comprising an active site consensus sequence of SEQ ID NO: 7 (H**E**XXH; wherein the underlined histidine amino acids are the $Zn^{2+}$ binding sites, the bold glutamic acid residue is the active site residue and X is any amino acid), wherein the amino acid sequence of the RasP polypeptide consensus sequence of SEQ ID NO: 7 can be aligned with the amino acid residues 20 to 24 of the RasP polypeptide of SEQ ID NO: 2 and result in at least 80% sequence homology.

In certain embodiments, a RasP polypeptide of the disclosure is further defined as a metalloendopeptidase belonging to EC class 3.4.24 (EC 3.4.24).

## II. BACTERIAL CELLS AND METHODS OF USE

The present disclosure is generally related to modified Gram-positive bacterial cells producing increased amounts of one or more protein(s) of interest. Thus, certain embodiments the instant disclosure are directed to modified Gram-positive bacterial cells expressing an increased amount of a POI relative to unmodified (*i.e.,* parental) Gram-positive bacterial cells, wherein the modified (*i.e.,* daughter) bacterial cells comprise a modification which increases *rasP* gene expression.

In certain embodiments, the disclosure pertains to methods of modifying bacterial cells such that the modified (daughter) cells produce an increased level of a protein of interest. As described herein, the disclosure pertains to a protein of interest produced by fermenting a modified bacterial cell of the instant disclosure. The disclosure may be directed to one or more proteinaceous compositions comprising one or more protein(s) of interest thus made. In other instances the present disclosure pertains to methods of producing one or more protein(s) of interest employing modified bacterial cells set forth herein, as well as to methods of producing and using one or more compositions comprising one or more protein(s) of interest.

In certain embodiments, a modification of a bacterial cell which increases *rasP* gene expression can be any type of genetic modification which enhances or increases the expression of a *rasP* gene (or ORF thereof) in the modified host. For example, in certain embodiments, a modification of a bacterial cell which increases *rasP* gene expression comprises codon optimization of the *rasP* gene (or ORF thereof) for enhanced expression in a desired host cell. In other embodiments, a modification of a host cell which increases *rasP* gene expression is an expression cassette encoding a RasP polypeptide, wherein the expression cassette is introduced into the (modified) cell. In certain other embodiments, an expression cassette comprising a gene or ORF encoding a RasP polypeptide is under the control of an inducible promoter, a constitutive promoter, a conditional promoter and the like.

In certain embodiments, a promoter for directing the transcription of a polynucleotide sequence encoding a POI or a RasP polypeptide is a wild-type *aprE* promoter, a mutant *aprE* promoter or a consensus *aprE* promoter set forth in PCT International Publication WO2001/51643. In certain other embodiments, a promoter for directing the transcription of a polynucleotide sequence encoding a POI or a RasP polypeptide is a wild-type *spoVG* promoter, a mutant *spoVG* promoter or a consensus *spoVG* promoter (Frisby and Zuber, 1991).

In certain embodiments, a modified bacterial cell of the disclosure is a *Bacillaceae* family member. In other embodiments, a modified bacterial cell of the disclosure is a member of the *Bacillus* genus. In certain embodiments, a modified bacterial cell of the disclosure is a *Bacillus* cell selected from *B. subtilis, B. licheniformis, B. lentus, B. brevis, B. stearothermophilus, B. alkalophilus, B. amyloliquefaciens, B. clausii, B. sonorensis, B. halodurans, B. pumilus, B. lautus, B. pabuli, B. cereus, B. agaradhaerens, B akibai, B. clarkii, B. pseudofirmus, B. lehensis, B. megaterium, B. coagulans, B. circulans, B. gibsonii* and *B. thuringiensis, B. marmarensis.* In other embodiments, the *Bacillus* cell is *Bacillus subtilis* or *Bacillus licheniformis.*

It is recognized in the art that the genus *Bacillus* continues to undergo taxonomical reorganization. Thus, it is intended that the genus include species that have been reclassified, including, but not limited to, such organisms as *B. stearothermophilus,* which is now named *"Geobacillus stearothermophilus",* or *B. polymyxa,* which is now *"Paenibacillus polymyxa".*

**III. MODIFIED CELLS PRODUCING AN INCREASED LEVEL OF A PROTEIN OF INTEREST**

To confirm that a modified Gram-positive bacterial cell produces an increased level of a POI, various methods of screening can be applied. For instance, an expression vector may encode a polypeptide fusion to the target protein and serves as a detectable label, or alternatively, the target protein itself may serve as the selectable or screenable marker. The labeled protein can also be detected using Western blotting, dot blotting (detailed descriptions of such methods are available at the website of the Cold Spring Harbor Protocols), ELISA, or, if the label is a GFP, whole cell fluorescence or FACS.

For example, a 6-histidine tag can be included to make a fusion to the target protein, and Western blots can be used to detect such a tag. Moreover, if the target protein expresses at sufficiently high levels, SDS-PAGE combined with Coomassie/silver staining, may be performed to adequately detect increases in mutant expression over wild type; and in such a case, no labeling of any molecules would be necessary.

In other embodiments, the expression of the POI in a modified (host) cell versus an unmodified (parental) cell is correlated with mRNA transcript levels. For example, certain embodiments are related to the molecular characterization of a gene or ORF encoding a POI, which usually includes a thorough analysis of the temporal and spatial distribution of RNA expression. A number of widely used procedures exist and are known in the art for detecting and determining the abundance of a particular mRNA in a total or poly(A) RNA sample. Non-limiting examples include such methods as Northern blot analysis, nuclease protection assays (NPA), in situ hybridization, and reverse transcription-polymerase chain reaction (RT-PCR).

Other methods can be employed to confirm the improved level of a protein of interest, including, for example, the detection of the increase of protein activity or amount per cell, protein activity or amount per milliliter of medium, allowing cultures or fermentations to continue efficiently for longer periods of time, or through a combination of these methods.

The detection of specific productivity is another suitable method for evaluating protein production. Specific productivity (Qp) can be determined using the following equation:

$$Qp = gP/gDCW \bullet hr$$

herein, "gP" is grams of protein produced in the tank; "gDCW' is grams of dry cell weight (DCW) in the tank and "hr" is fermentation time in hours from the time of inoculation, which includes the time of production as well as growth time.

In certain embodiments, a modified bacterial cell of the disclosure produces at least about 5%, at least about 6%, at least about 7%, at least about 8%, at least about 9%, or at least about 10% or more of a POI, as compared to its unmodified (parental) cell.

**IV. CULTURING MODIFIED CELLS FOR PRODUCTION OF A PROTEIN OF INTEREST**

In other embodiments, the present disclosure provides methods for increasing the protein productivity of a modified bacterial cell, as compared (*i.e.,* relative) to an unmodified (parental) cell. More particularly, in certain embodiments, methods for increasing the protein productivity of a modified bacterial cell comprises culturing the modified bacterial cells under suitable fermentation conditions, wherein the modified cell comprises a modification which increases *rasP* gene expression.

Thus, host cells and transformed cells can be cultured in conventional nutrient media. The culture media for transformed host cells may be modified as appropriate for activating promoters and selecting transformants. The specific culture conditions, such as temperature, pH and the like, may be those that are used for the host cell selected for expression, and will be apparent to those skilled in the art. In addition, culture conditions may be found in the scientific literature such as Sambrook (1982; 2001), Harwood et al. (1990) and from the American Type Culture Collection (ATCC).

Thus, as describe herein the instant disclosure may be directed to methods of producing a POI comprising fermenting a modified bacterial cell, wherein the modified cell secretes the POI into the culture medium. Fermentation methods well known in the art can be applied to ferment the modified and unmodified bacterial cells.

In some instances, the bacterial cells may be cultured under batch or continuous fermentation conditions. A classical batch fermentation is a closed system, where the composition of the medium is set at the beginning of the fermentation and is not altered during the fermentation. At the beginning of the fermentation, the medium is inoculated with the desired organism(s). In this method, fermentation is permitted to occur without the addition of any components to the system. Typically, a batch fermentation qualifies as a "batch" with respect to the addition of the carbon source, and attempts are often made to control factors such as pH and oxygen concentration. The metabolite and biomass compositions of the batch system change constantly up to the time the fermentation is stopped. Within typical batch cultures, cells can progress through a static lag phase to a high growth log phase, and finally to a stationary phase, where growth rate is diminished or halted. If untreated, cells in the stationary phase eventually die. In general, cells in log phase are responsible for the bulk of production of product.

A suitable variation on the standard batch system is the "fed-batch fermentation" system. In this variation of a typical batch system, the substrate is added in increments as the fermentation progresses. Fed-batch systems are useful when catabolite repression likely inhibits the metabolism of the cells and where it is desirable to have limited amounts of substrate in the medium. Measurement of the actual substrate concentration in fed-batch systems is difficult and is therefore estimated on the basis of the changes of measurable factors, such as pH, dissolved oxygen and the partial pressure of waste gases, such as $CO_2$. Batch and fed-batch fermentations are common and known in the art.

Continuous fermentation is an open system where a defined fermentation medium is added continuously to a bioreactor, and an equal amount of conditioned medium is removed simultaneously for processing. Continuous fermentation generally maintains the cultures at a constant high density, where cells are primarily in log phase growth. Continuous fermentation allows for the modulation of one or more factors that affect cell growth and/or product concentration. For instance in one example a limiting nutrient, such as the carbon source or nitrogen source, is maintained at a fixed rate and all other parameters are allowed to moderate. In other systems, a number of factors affecting growth can be altered continuously while the cell concentration, measured by media turbidity, is kept constant. Continuous systems strive to maintain steady state growth conditions. Thus, cell loss due to medium being drawn off should be balanced against the cell growth rate in the fermentation. Methods of modulating nutrients and growth factors for continuous fermentation processes, as well as techniques for maximizing the rate of product formation, are well known in the art of industrial microbiology.

Thus as described herein, a POI produced by a transformed (modified) host cell may be recovered from the culture medium by conventional procedures including separating the host cells from the medium by centrifugation or filtration, or if necessary, disrupting the cells and removing the supernatant from the cellular fraction and debris. Typically, after clarification, the proteinaceous components of the supernatant or filtrate are precipitated by means of a salt, *e.g.*, ammonium sulfate. The precipitated proteins are then solubilized and may be purified by a variety of chromatographic procedures, *e.g.*, ion exchange chromatography, gel filtration.

## V. TRANSFORMATION

A polynucleotide construct comprising a nucleic acid encoding a RasP polypeptide or a POI of the disclosure can be constructed such that it is expressed by a host cell. Because of the known degeneracies in the genetic code, different polynucleotides encoding an identical amino acid sequence can be designed and made with routine skills in the art. For example, codon optimizations can be applied to optimize production in a particular host cell.

Nucleic acids encoding proteins of interest can be incorporated into a vector, wherein the vector can be transferred into a host cell using well-known transformation techniques, such as those disclosed herein.

The vector may be any vector that can be transformed into and replicated within a host cell. For example, a vector comprising a nucleic acid encoding a POI can be transformed and replicated in a bacterial host cell as a means of propagating and amplifying the vector. The vector also may be transformed into an expression host, so that the protein encoding nucleic acids (*i.e.,* ORFs) can be expressed as a functional protein. Bacterial cells that serve as expression hosts (*i.e,* a modified bacterial "host" cell) include members of the *Bacillaceae* family and members of the *Bacillus* genus.

A representative vector which can be modified with routine skill to comprise and express a nucleic acid encoding a POI is vector p2JM103BBI (*see*, Vogtentanz, 2007).

As stated briefly above, a polynucleotide encoding a RasP polypeptide or a POI can be operably linked to a suitable promoter, which allows transcription in the host cell. The promoter may be any nucleic acid sequence that shows transcriptional activity in the host cell of choice and may be derived from genes encoding proteins either homologous or heterologous to the host cell. For example, in certain embodiments, a modification which increases the expression of a *rasP* gene comprises substituting the native *rasP* gene promoter with any promoter having a higher activity than the native *rasP* promoter. Means of assessing promoter activity/strength are routine for the skilled artisan.

Examples of suitable promoters for directing the transcription of a polynucleotide sequence encoding RasP polypeptide or a POI of the disclosure, especially in a bacterial host, include the promoter of the lac operon of *E. coli,* the *Streptomyces coelicolor* agarase gene *dagA* or *celA* promoters, the promoters of the *Bacillus licheniformis* alpha-amylase gene (*amyL*), the promoters of the *Bacillus stearothermophilus* maltogenic amylase gene (*amyM*), the promoters of the *Bacillus amyloliquefaciens* alpha-amylase (amyQ), the promoters of the *Bacillus subtilis xylA* and *xylB* genes, and the like.

In certain embodiments, a promoter for directing the transcription of a polynucleotide sequence encoding a POI or a RasP polypeptide is a wild-type *aprE* promoter, a mutant *aprE* promoter or a consensus *aprE* promoter set forth in PCT International Publication WO2001/51643 (PCT International Application PCT/US2000/035312). In certain other embodiments, a promoter for directing the transcription of a polynucleotide sequence encoding a POI or a RasP polypeptide is a wild-type *spoVG* promoter, a mutant *spoVG* promoter or a consensus *spoVG* promoter (Frisby and Zuber, 1991).

In certain embodiments, an *aprE* promoter comprises a nucleic acid sequence comprising about 90-95% sequence identity to SEQ ID NO: 4.

In other embodiments, a *spoVG* promoter comprises a nucleic acid sequence comprising about 90-95% sequence identity SEQ ID NO: 3.

In some instances, a promoter for directing the transcription of the polynucleotide sequence encoding RasP polypeptide or a POI is a ribosomal promoter such as a ribosomal RNA promoter or a ribosomal protein promoter. More particularly, in certain instances, the ribosomal RNA promoter is a *rrn* promoter derived from *B. subtilis,* more particularly, the *rrn* promoter is a *rrnB, rrnI* or *rrnE* ribosomal promoter from *B. subtilis.* In certain instances, the ribosomal RNA promoter is a P2 *rrnI* promoter from *B. subtilis* set forth in PCT International Publication No. WO2013/086219.

The RasP or POI coding sequence can be operably linked to a signal sequence. The nucleic acid sequence encoding the signal sequence may be the DNA sequence naturally associated with the *rasP gene* or the GOI (encoding the POI) to be expressed, or may be from a different genus or species. A signal sequence and a promoter sequence comprising a polynucleotide construct or vector can be introduced into a bacterial host cell, and those sequences may be derived from the same source or different sources. For example, in certain embodiments, the signal sequence is an *aprE* signal sequence (*see, e.g*., Vogtentanz *et al.,* 2007; Wang *et al.,* 1988) that is operably linked to an *aprE* promoter set forth in PCT International Publication WO2001/51643.

In certain embodiments, a modification to an endogenous chromosomal *rasP* gene is a modification of the native 5'-untranslated region (5'-UTR) of the endogenous chromosomal *rasP* gene. In another embodiment, native *rasP* chromosomal 5'-UTR is replaced with a 5'-UTR comprising about 90-95% sequence identity to the *aprE* 5'-UTR of SEQ ID NO: 5.

An expression vector may also comprise a suitable transcription terminator and, in eukaryotes, certain polyadenylation sequences operably linked to the DNA sequence encoding the protein of interest. Termination and polyadenylation sequences may suitably be derived from the same sources as the promoter, but as described herein, the termination and polyadenylation sequences may well be derived from different sources as each other and/or as the promoter.

A suitable vector may further comprise a nucleic acid sequence enabling the vector to replicate in the host cell. Examples of such enabling sequences include the origins of replication of plasmids pUC19, pACYC177, pUB110, pE194, pAMB1, pIJ702, and the like.

A suitable vector may also comprise a selectable marker, *e.g*., a gene the product of which complements a defect in the isolated host cell, such as the *dal* genes from *B. subtilis* or *B. licheniformis*; or a gene that confers antibiotic resistance such as, *e.g.*, ampicillin resistance, kanamycin resistance, chloramphenicol resistance, tetracycline resistance and the like.

A suitable expression vector typically includes components of a cloning vector, such as, for example, an element that permits autonomous replication of the vector in the selected host organism and one or more phenotypically detectable markers for selection purposes. Expression vectors typically also comprise control nucleotide sequences such as, for example, promoter, operator, ribosome binding site, translation initiation signal and optionally, a repressor gene, one or more activator genes sequences, or the like.

Additionally, a suitable expression vector may further comprise a sequence coding for an amino acid sequence capable of targeting the protein of interest to a host cell organelle such as a peroxisome, or to a particular host cell compartment. Such a targeting sequence may be, for example, the amino acid sequence "SKL". For expression under the direction of control sequences, the nucleic acid sequence of the protein of interest can be operably linked to the control sequences in a suitable manner such that the expression takes place.

Protocols, such as described herein, used to ligate the DNA construct encoding a protein of interest, promoters, terminators and/or other elements, and to insert them into suitable vectors containing the information necessary for replication, are well known to persons skilled in the art *(see, e.g.,* Sambrook *et al*., 1989, and 3rd edition 2001).

An isolated cell, either comprising a polynucleotide construct or an expression vector, is advantageously used as a host cell in the recombinant production of a POI. The cell may be transformed with the DNA construct encoding the POI, conveniently by integrating the construct (in one or more copies) into the host chromosome. Integration is generally deemed an advantage, as the DNA sequence thus introduced is more likely to be stably maintained in the cell. Integration of the DNA constructs into the host chromosome may be performed applying conventional methods, for example, by homologous or heterologous recombination. Alternatively, the cell may be transformed with an expression vector as described above in connection with the different types of host cells.

It is, in other instances, advantageous to delete genes from expression hosts, where the gene deficiency can be cured by an expression vector. Known methods may be used to obtain a bacterial host cell having one or more inactivated genes. Gene inactivation may be accomplished by complete or partial deletion, by insertional inactivation or by any other means that renders a gene nonfunctional for its intended purpose, such that the gene is prevented from expression of a functional protein.

Techniques for transformation of bacteria and culturing the bacteria are standard and well known in the art. They can be used to transform the improved hosts of the present disclosure for the production of recombinant proteins of interest. Introduction of a DNA construct or vector into a host cell includes techniques such as transformation; electroporation; nuclear microinjection; transduction; transfection, e.g., lipofection mediated and DEAE-Dextrin mediated transfection; incubation with calcium phosphate DNA precipitate; high velocity bombardment with DNA-coated microprojectiles; gene gun or biolistic transformation and protoplast fusion, and the like. Transformation and expression methods for bacteria are also disclosed in Brigidi *et al*. (1990). A preferred general transformation and expression protocol for protease deleted Bacillus strains is provided in Ferrari et al., U.S. Pat. No. 5, 264, 366.

## VI. PROTEINS OF INTEREST PRODUCED BY MODIFIED CELLS

In another embodiment, the present disclosure provides methods for producing increased levels of a POI comprising obtaining a modified Gram-positive bacterial cell expressing an increased amount of a POI, wherein the modified bacterial cell comprises modification which increase expression of a *rasP* gene (or ORF thereof), and culturing the modified cell under conditions such that the POI is expressed, wherein the modified bacterial cell expressing an increased amount of a POI is relative to the expression of the POI in an unmodified Gram-positive bacterial cell.

The POI can be any endogenous or heterologous protein, and it may be a variant of such a POI. The protein can contain one or more disulfide bridges or is a protein whose functional form is a monomer or a multimer, *i.e.,* the protein has a quaternary structure and is composed of a plurality of identical (homologous) or non-identical (heterologous) subunits, wherein the POI or a variant POI thereof is preferably one with properties of interest.

In certain embodiments, a POI or a variant POI thereof is selected from the group consisting of acetyl esterases, aryl esterases, aminopeptidases, amylases, arabinases, arabinofuranosidases, carbonic anhydrases, carboxypeptidases, catalases, cellulases, chitinases, chymosins, cutinases, deoxyribonucleases, epimerases, esterases, $\alpha$-galactosidases, $\alpha$-glucanases, glucan lysases, endo-$\beta$-glucanases, glucoamylases, glucose oxidases, $\alpha$-glucosidases, $\beta$-glucosidases, glucuronidases, glycosyl hydrolases, hemicellulases, hexose oxidases, hydrolases, invertases, isomerases, laccases, lipases, lyases, mannosidases, oxidases, oxidoreductases, pectate lyases, pectin acetyl esterases, pectin depolymerases, pectin methyl esterases, pectinolytic enzymes, perhydrolases, polyol oxidases, peroxidases, phenoloxidases, phytases, polygalacturonases, proteases, peptidases, rhamno-galacturonases, ribonucleases, transferases, transport proteins, transglutaminases, xylanases, hexose oxidases, and combinations thereof.

The POI or variant POI may also be a peptide, a peptide hormone, a growth factor, a clotting factor, a chemokine, a cytokine, a lymphokine, an antibody, a receptor, an adhesion molecule, a microbial antigen (*e.g.,* HBV surface antigen, HPV E7, *etc*.), and variants thereof or fragments thereof.

Other types of proteins (or variants) of interest may be those that are capable of providing nutritional value to a food or to a crop. Non-limiting examples include plant proteins that can inhibit the formation of anti-nutritive factors and plant proteins that have a more desirable amino acid composition (*e.g*., a higher lysine content than a non-transgenic plant).

## VII. USE OF THE PROTEINACEOUS COMPOSITIONS

The present disclosure provides a proteinaceous composition comprising one or more protein(s) of interest. The proteinaceous composition is suitably produced using the methods provided herein. The proteinaceous composition comprises a protein of interest, encoded by a gene of interest, expressed using a method described herein. The composition may be used in various useful industrial applications such as, for example, in biomass hydrolysis, cleaning applications, grain processing, animal nutrition, food composition, textile treatment, personal care products and the like.

For example, a proteinaceous composition thus produced can be used in cleaning application. Enzymatic cleaning components are popular because of their ability to break down soils, stains, and other debris that are otherwise not readily removed by conventional chemical detergents. Well-known enzymes useful for cleaning include proteases and amylases, with other enzymes such as lipases, pectinases, mannanases, even certain cellulases, each providing a set of different functionalities. Proteases combat protein-based stains; amylases work on carbohydrates and starches; and lipases break down lipids or fats, for example. The disclosure provides modified bacterial cells, which have demonstrated improved protein production, suitable and advantageous as a producer of industrial enzymes, variants, and mixtures of interest to such use in cleaning applications.

In another example, the proteinaceous composition thus made can be used in grain procession. Starch is the most common storage carbohydrate in plants, used by the plants themselves as well as by microbes and by higher organisms. A great variety of enzymes are able to catalyze starch hydrolysis. Starch from all plant sources occurs in the form of granules, but depending on the species of the plant source, starch presents in markedly different size and physical characteristics. Acid hydrolysis of starch had widespread use in the past, however this process has now largely been replaced by enzymatic processes, which are known to demand less corrosion-resistant materials and other benefits, need less energy for heating and are relatively easier to control than the acid process. The disclosure provides an engineered, transformed, or derived eubacterial cell, which has demonstrated improved protein production, suitable and advantageous as a producer of industrial enzymes, variants, and mixtures of interest to such use in starch degradation and grain processing.

In another example, the proteinaceous composition thus made can be used in food application. Enzymes produced by bacteria, yeasts and moulds have been used in food application to make foods such as bread, cheese, beer and wine for many thousands of years. Today enzymes are used in bakery, cheese making, starch processing and production of fruit juices and other drinks, providing various benefits such improved texture, appearance and nutritional value, generate desirable flavors and aromas, and the like. Food enzymes typically originate in animals and plants (for example, a starch-digesting enzyme, amylase, can be obtained from germinating barley seeds) as well as from a range of beneficial microorganisms. Enzymes are deemed viable and desirable alternatives to traditional chemical-based technology, replacing synthetic chemicals in many processes. Enzymes can help improve the environmental performance of food production processes, reducing energy consumption and improving biodegradability of waste or side products. Enzymes tend to be more specific in their actions than synthetic chemicals, and as such, enzymatic processes tend to give fewer side reactions and waste or byproducts, and consequently producing higher quality products and reducing the likelihood of pollution. Enzymatic processes are often also the only processes possible. An example of this is in the production of clear apple juice concentrate, which relies on the use of the enzyme pectinase. Most of the food enzymes are produced from microorganisms such *Bacillus, Aspergillus, Streptomyces* or *Kluyveromyces.* The disclosure provides an engineered, transformed, or derived eubacterial cell, which has demonstrated improved protein production, suitable and advantageous as a producer of industrial enzymes, variants, and mixtures of interest to such use in food applications.

In another example, the proteinaceous composition thus made can be used in animal feed additive. Cellulases, xylanases, β-glucanases, proteases, lipases, phytases and other carbohydrase of interest have been widely used in animal feed industry. Since many plant based feeds contain substances with anti-nutritional factors that reduce animal growth, the enzymes added to such feeds improve digestibility of these anti-nutritional factors by degrading fibres, proteins, starches and phytates, rendering them more digestible by the animals, and enabling the use of cheaper and often locally produced feeds, while maximizing meat, egg or milk productivity. At the same time, the enzymes added to such feeds also may provide benefits supporting gut health and enhanced animal performance. The disclosure provides an engineered, transformed, or derived eubacterial cell, which has demonstrated improved protein production, suitable and advantageous as a producer of industrial enzymes, variants, and mixtures of interest to such use in animal feed applications.

In yet a further example, the proteinaceous composition thus made can be used in textile applications. Enzymes have become an integral part of the textile processing. There are two well-established enzyme applications in the textile industry. First, enzymes such as amylases are commonly used in the preparatory finishing area for desizing. Second, enzymes such as cellulases are commonly used in the finishing area for softening, bio-stoning and reducing of pilling propensity of cotton goods.

Other enzymes such as, for example, pectinases, lipases, proteases, catalases, xylanases *etc.,* are also used in textile processing. Moreover, there are various applications which entail enzymes included fading of denim and non-denim, bio-scouring, bio-polishing, wool finishing, peroxide removal, decolourization of dyestuff, *etc.* Thus, in certain embodiments the disclosure provides modified Gram-positive bacterial cells (which are demonstrated herein as having improved protein production) as a producer of industrial enzymes, variants, and mixtures of interest to such use in textiles applications.

Non-limiting examples of compositions and methods disclosed herein are as follows:

## EXAMPLES

Certain aspects of the present disclosure may be further understood in light of the following examples, which should not be construed as limiting. Modifications to materials and methods will be apparent to those skilled in the art.

## EXAMPLE 1

## CONSTRUCTION OF MODIFIED *BACILLUS SUBTILIS* HOST CELLS

Taq polymerase, dNTPs and buffers were purchased from Takara Bio Inc. (Clontech Laboratories, Inc.; Mountain View, CA) and used for the construction of the mutant *B. subtilis* cells described below. Phusion High Fidelity DNA polymerase (New England Biolabs; Ipswich, MA) was used for the construction of the vectors. Primers were obtained from Eurogentec (Liege, Belgium).

### A. Construction of a Modified *B. subtilis* Host Comprising a Deleted *rasP* Gene.

The construction of a *rasP* deletion mutant ("*ΔrasP*") in a *B. subtilis* cells was performed using the modified mutation delivery method described by Fabret *et al.* (2002), wherein the parental *B. subtilis* cells comprise a deleted *upp* gene ("*Δupp*"), as set forth in Fabret *et al.* (2002). To completely replace (delete) the *rasP gene* in the *B. subtilis* (Δ*upp*) cells, the 5' and 3' flanking regions of the *rasP* gene were amplified using PCR primer pairs of SEQ ID NO: 44 and SEQ ID NO: 45.

The amplified *rasP* fragments were fused to a phleomycin resistance cassette comprising the *upp* gene and the *cI* gene (*see,* Fabret *et al.,* 2002). The resulting fusion product was used to transform competent *B. subtilis* Δ*upp::neoR* (parental) cells, where competence was induced with 0.3% xylose. This resulted in phleomycin resistant and neomycin sensitive (daughter) cells lacking the target *rasP* gene. The PCR reactions were performed using the oligonucleotide pairs of SEQ ID NO: 10/SEQ ID NO: 9 and oligonucleotide primers of SEQ ID NO: 10/ SEQ ID NO: 11 to verify the correct deletion of the target *rasP* gene.

### B. Construction of a Modified *B. subtilis* Host Comprising a Deleted *tepA* Gene

The modified mutation delivery method of Fabret *et al.* (2002) was further utilized to construct a deletion mutant of the *tepA* gene ("Δ*tepA*") in the *B. subtilis* (Δ*upp*) cells. Thus, to completely replace the *tepA* gene, the 5' and 3' flanking regions of these genes were amplified using the PCR primer pairs SEQ ID NO: 12/SEQ ID NO: 13 and SEQ ID NO: 14/SEQ ID NO: 15. The amplified fragments were then fused to a phleomycin resistance cassette containing the *upp* and *cI* genes (*see*, Fabret *et al.,* 2002). The resulting fusion product was then used to transform competent *B. subtilis* Δ*upp::neoR* (parental) cells, where competence was induced with 0.3% xylose. This resulted in phleomycin resistant and neomycin sensitive strains lacking the target *tepA* gene. PCR reactions were performed to verify the correct deletion of the *tepA* gene using primer combinations of SEQ ID NO: 16/SEQ ID NO: 15 and SEQ ID NO: 16/SEQ ID NO: 11.

### C. Construction of Modified *B. Subtilis* Cells Over-expressing the *rasP* Gene

#### 1. Construction of the integration vector pRS-*spoIIIAA~AG*.

A 1,040 base pair DNA fragment (SEQ ID NO: 17) of the *spoIIIAA~AB* genes was amplified using the primer pair of SEQ ID NO: 18/SEQ ID NO: 19. A spectinomycin resistant marker, flanked by two lox sequences *(i.e.,* SEQ ID NO: 20), was synthetically ordered as g-Block from IDT (Integrated DNA Technologies). A 981 base pair DNA sequence in the *spoIIIAF~AG* genes (*i.e.,* SEQ ID NO: 21) was amplified using the oligonucleotides of SEQ ID NO: 22 and SEQ ID NO: 23. The commercially available plasmid "pRS426" was amplified using the oligonucleotides of SEQ ID NO: 24 and SEQ ID NO: 25. Subsequently, the above DNA sequences *(i.e.,* SEQ ID NO: 17, SEQ ID NO: 20, SEQ ID NO: 21) and the pRS426 amplified vector were assembled *in vitro* using a Gibson Assembly@ HiFi 1 Step Kit (SGI) to generate the integration vector "*pRS-spoIIIAA~AG*".

#### 2. Construction of the plasmid pRS-*spoIIIAA~AG-PspoVG-rasP.*

For certain *rasP* overexpression experiments described herein, the promoter of the B. *subtilis spoVG* gene was used to drive (over-express) the *rasP* gene. For example, a synthetic g-Block containing the sequence of the *spoVG* promoter (SEQ ID NO: 3) was ordered from IDT (Integrated DNA Technologies). The *rasP* coding sequence was amplified by PCR using the oligonucleotides of SEQ ID NO: 26 and SEQ ID NO: 27. The plasmid designated as "pRS-*spoIIIAA~AG*" was amplified using the oligonucleotides of SEQ ID NO: 28 and SEQ ID NO: 29. The plasmid "pRS-*spoIIIAA~AG-PspoVG-rasP*" was made by assembly of the *spoVG* promoter sequence, the *rasP* coding sequence and the plasmid pRS-*spoIIIAA~AG,* using a Gibson Assembly@ HiFi 1 Step Kit (SGI). To further increase the expression and production of the RasP ploypeptide, the *aprE* (gene) leader sequence *(i.e.,* 5'-UTR) of SEQ ID NO: 5 was cloned in front (5') of the *rasP* gene, to generate the plasmid "pRS-*spoIIIAA~AG-PspoVG-UTR-rasP".*

**3. Construction of the plasmid pRS-*spoIIIAA~AG-PaprE-rasP.***

For constructing the *rasP* (gene) over-expression plasmid termed herein as "pRS-*spoIIAA~AG-PaprE-rasP*", the *B. subtilis aprE* (gene) promoter ("P*aprE*"; SEQ ID NO: 4) was incorporated into the vector, 5' and operably linked to the *rasP* coding sequence. The nucleotide sequence of the *B. subtilis aprE* promoter was amplified by PCR from the genome of B. *subtilis* using the oligonucleotides of SEQ ID NO: 30 and SEQ ID NO: 31. The plasmid "*pRS-spoIIIAA~AG-PspoVG-rasP*" was amplified by PCR using the oligonucleotides of SEQ ID NO: 32 and SEQ ID NO: 33. The *aprE* promoter *(PaprE)* and plasmid were ligated with a Gibson Assembly@ HiFi 1 Step Kit (SGI).

**4. Construction of the plasmid pRS-*spoIIIAA~AG-PspoVG-tepA.***

The nucleotide sequence of the signal peptide peptidase *tepA* was amplified from B. *subtilis* genomic DNA using the oligonucleotides of SEQ ID NO: 34 and SEQ ID NO: 35. The plasmid backbone of pRS-*spoIIIAA~AG-PspoVG-rasP* was amplified by PCR using the oligonucleotide pair of SEQ ID NO: 36/SEQ ID NO: 37The *tepA* gene sequence and the amplified plasmid were ligated with a Gibson Assembly@ HiFi 1 Step Kit (SGI). The resulting plasmid was named pRS-*spoIIIAG*-P*spoVG*-tepA.

**EXAMPLE 2**

**TRANSFORMING *B. SUBTILIS* HOST CELLS WITH HETEROLOGOUS *rasP* or *tepA* GENE CONSTRUCTS**

The plasmids pRS-*spoIIIAA~AG-PspoVG-rasP* (*see,* Example 1.C.2), pRS-*spoIIIAA~AG-PspoVG-UTR-rasP* (*see,* Example 1.C.2), *pRS-spoIIIAA~AG-PaprE-rasP* (*see,* Example 1.C.3) and *pRS-spoIIIAA~AG-PspoVG-tepA* (*see,* Example 1.C.4), were each linearized with the restriction endonuclease Seal and each plasmid separately transformed into competent *Bacillus subtilis* cells. Positive colonies were selected on Luria agar plates containing 100μg/ml of spectinimycin. The resulting (transformed) *B. subtilis* (daughter) cells are referred to herein as "PspoVG-rasP", "PspoVG-UTR-rasP", "PaprE-rasP" and PspoVG-tepA.

**EXAMPLE 3**

**TRASNSFORMING MODIFIED *B. SUBTILIS* (HOST) CELLS WITH HETEROLOGOUS GENES (EXPRESSION CONSTRUCTS) ENCODING PROTEINS OF INTEREST**

**A. AmyL Expression Construct**

The *B. subtilis aprE* promoter ("P*aprE*") and *aprE* signal sequence ("*aprE* UTR", hereinafter, "UTR"; *see* Example 1.C.2) were used to drive the expression of the *Bacillus licheniformis* "AmyL" amylase (mature sequence SEQ ID NO: 38). The expression construct, designated herein as "PaprE-AmyL-catR" (which includes a chloramphenicol acetyltransferase resistance ("catR") marker gene), was transformed (1) into *B. subtilis* wild-type ("wt") cells *(i.e.,* parental cells), (2) into modified (daughter) *B. subtilis* cells comprising the *rasP* deletion (*ΔrasP*) and (3) into modified (daughter) *B. subtilis* cells comprising the *tepA* deletion (Δ*tepA*). Transformants were selected on Luria agar plates containing 5 μg/ml of chloramphenicol.

**B. Expression Construct for Amylase PcuAmy1-v6**

The *B. subtilis aprE* promoter ("P*aprE*") and *aprE* signal sequence ("UTR") were used to drive the expression of a *Paenibacillus curdlanolyticus* amylase (mature sequence SEQ ID NO: 39) variant designated PcuAmy1-v6. The expression cassette, designated "PaprE-PcuAmy1-v6-catR" (which includes the chloramphenicol acetyltransferase resistance (catR) marker gene), was transformed (1) into modified *B. subtilis* (daughter) cells comprising and expressing/over-expressing the *rasP* gene under the control of the "PspoVG" promoter *(i.e.,* the "PspoVG-rasP" cells described above in Example 2), (2) into modified *B. subtilis* (daughter) cells comprising and expressing/over-expressing the *rasP gene* under the control of the *PaprE* promoter *(i.e.,* the "PaprE-rasP" cells described above in Example 2), (3) into modified *B. subtilis* (daughter) cells comprising and expressing/over-expressing the *tepA* gene under the control of the *"PspoVG"* promoter *(i.e.,* the "PspoVG-tepA" cells described above in Example 2) and (4) into *B. subtilis* (parental) cells. Positive colonies were selected on Luria agar plates containing 5 μg/ml of chloramphenicol.

### C. AmyE Expression Construct

The *B. subtilis aprE* promoter ("P*aprE*") was used to drive the expression of the B. *subtilis* "AmyE" amylase (mature sequence SEQ ID NO: 40). The expression cassette, designated "PaprE-amyE-catR" (which includes a chloramphenicol acetyltransferase resistance (catR) marker gene), was introduced into the genomic *aprE* locus of (1) modified *B. subtilis* cells comprising and expressing/over-expressing the *rasP* gene under the control of the *"PspoVG"* promoter *(i.e.,* the "PspoVG-rasP" cells described above in Example 2), (2) modified *B. subtilis* cells comprising and expressing/over-expressing the *rasP gene* under the control of the "PspoVG" promoter and *aprE* UTR *(i.e.,* the "PspoVG-UTR-rasP" cells described above in Example 2), (3) modified *B. subtilis* cells comprising and expressing/over-expressing the *rasP* gene under the control of the "P*aprE*" promoter (*i.e,* the "PaprE-rasP" cells described above in Example 2), (4) modified *B. subtilis*) cells comprising and expressing/over-expressing the *tepA* gene under the control of the "P*spoVG*" promoter (the "PspoVG-tepA" cells described above in Example 2), (5) modified *B. subtilis* cells comprising the *rasP* (*ΔrasP*) gene deletion (*see,* Example 1.A), (6) modified *B. subtilis* cells comprising the *tepA* (Δ*tepA*) gene deletion (*see,* Example 1.B) and (7) unmodified (parental) *B. subtilis* control cells. Positive colonies were selected on Luria agar plates containing 5 μg/ml of chloramphenicol.

### D. BglC Expression Construct

An expression construct encoding *B. subtilis* β-D-glucosidase (hereinafter "BglC"; mature sequence SEQ ID NO: 41), the expression of which is under the control of the *aprE* promoter *(PaprE)* was constructed and designated "*PaprE-BglC-catR*". The *PaprE-BglC-catR* construct was introduced into the *aprE* locus of (1) wild-type (unmodified; parental) *B. subtilis* cells, (2) the modified *B. subtilis* cells comprising and expressing/over-expressing "PspoVG-rasP", (3) the modified *B. subtilis* cells comprising and expressing/over-expressing "PaprE-rasP" and (4) the modified *B. subtilis* cells comprising and expressing/over-expressing "PspoVG-tepA". Positive colonies are selected on Luria agar plates containing 5μg/ml of chloramphenicol

### E. Properase Expression Construct

An expression construct encoding the protease Properase (mature sequence SEQ ID NO: 42), the expression of which is under the control of the *aprE* promoter *(PaprE),* was constructed and designated *"PaprE-Properase-catR".* The *PaprE-Properase-catR* expression construct was introduced into the *aprE* locus of (1) the modified *B. subtilis* cells comprising and expressing/over-expressing "PspoVG-rasP", (2) the modified *B. subtilis* cells comprising and expressing/over-expressing "PaprE-rasP", (3) the modified *B. subtilis* cells comprising and expressing/over-expressing "PspoVG-tepA" and (4) the wild-type (unmodified; parental) B. *subtilis* cells. Positive colonies were selected on Luria agar plates containing 5μg/ml of chloramphenicol. Chloramphenicol resistant colonies were amplified on Luria agar plates containing 25μg/ml of chloramphenicol.

### F. BPN'-Y217L Expression Construct

An expression construct encoding the *Bacillus amyloliquefaciens* protease BPN'-Y217L (SEQ ID NO: 43), the expression of which is under the control of the *aprE* promoter *(PaprE),* was constructed and designated "PaprE-BPN'-Y217L-catR". The PaprE-BPN'-Y217L-catR expression cassette was transformed into (1) the modified *Bacillus subtilis* cells comprising the rasP (*ΔrasP*) gene deletion, (2) the modified *Bacillus subtilis* cells comprising the tepA (Δ*tepA*) gene deletion and (3) the wild-type (unmodified; parental) *B. subtilis* cells. Colonies carrying the PaprE-BPN'-Y217L-catR construct were selected on Luria agar plates containing 5 μg/ml of chloramphenicol.

## EXAMPLE 4

## EXPRESSION AND PRODUCTION OF PROTEINS OF INTEREST

### A. Materials and Methods

**Bacterial growth conditions.** The *B. subtilis* cells were grown at 37°C, 280 rpm in Lysogeny Broth (LB; Oxoid Limited) or MBU medium. The MBU medium is similar to the MBD medium as described in Vogtentanz *et al.,* (2007), but lacks soytone, and instead of 7.5% glucose, it contains 2.1% glucose and 3.5 % maltodextrin DE13-17. When necessary, the medium was supplemented with neomycin 15 μg/ml or phleomycin 4 μg/ml for selection of mutations, or chloramphenicol 5 μg/ml or 25 μg/ml for selection or amplification (respectively) of the amylase or protease genes. In the pulse-chase labeling experiments on MBU medium 2.5 μg/ml chloramphenicol was used.

**Analysis of growth.** *B. subtilis* cells were grown overnight in LB with 2.5 μg/ml chloramphenicol at 37°C, 250 rpm. Cultures were diluted 50-fold in LB in 96-well micro-titer plates and grown for approximately 3 hours at 37°C, 800 rpm in a microtiter plate incubator (Grant-bio PHMP-4, Grant Instruments Ltd). Cultures were then diluted 50-fold in MBU and grown for 3 hours at 37°C, 800 rpm in a microtiter plate incubator. A final 50-fold dilution was made in fresh MBU and growth was monitored by $OD_{600}$ measurements in a PowerWave HT Microplate Spectrophotometer (Biotek).

**Analysis of protein expression and secretion.** *B. subtilis* cultures were inoculated from LB plates containing 25 μg/ml chloramphenicol and were grown for approximately 8 hours in LB broth containing 25 μg/ml chloramphenicol. These cultures were diluted 1000-fold in shake flasks with MBU medium containing 2.5 μg/ml chloramphenicol and incubated for approximately 16 hours at 37°C, 280 rpm in a Multitron orbital shaker (Infors) in high humidity. After measuring and correcting for the $OD_{600}$, equal amounts of cells were separated from the culture medium by centrifugation. For the analysis of extracellular proteins, proteins in the culture medium were precipitated with trichloroacetic acid (TCA; 10% w/v final concentration), dissolved in LDS buffer (Life Technologies) and heated for 10 minutes at 95°C. Next, proteins were separated by LDS-PAGE on 10% NuPage gels (Life Technologies) and stained with SimplyBlue™ SafeStain (Life Technologies).

**Pulse-chase protein labeling experiments.** Pulse-chase labeling of *B. subtilis* proteins were performed using Easy tag $^{35}$S Methionine (PerkinElmer Inc.). Immunoprecipitation and LDS-PAGE were performed as described previously (Van Dijl *et al.,* 1991) using the following adaptations. Cells were grown for 16 hours in MBU with 2.5 μg/ml chloramphenicol as described previously and diluted one hour prior to the actual labeling to $OD_{600}$ of approximately 0.7 in fresh MBU with 2.5 μg/ml chloramphenicol. Labeling was performed with 25 μCi $^{35}$S Met for 30 seconds before adding an excess amount of unlabeled methionine (chase; 0.625 mg/ml final concentration). Samples were collected at several time points, followed by direct precipitation of the proteins with 10% TCA on ice. Precipitates were re-suspended in lysis buffer (10 mM Tris pH 8.0, 25 mM $MgCl_2$, 200 mM NaCl and 5 mg/ml lysozyme). After 10-15 minutes of incubation at 37°C, lysis was achieved by adding 1% (w/v) SDS and heating for 10 minutes at 100°C.

Specific polyclonal antibodies against AmyE or AmyL were used for immunoprecipitation of the respective labeled proteins in STD-Tris buffer (10 mM Tris pH 8.2, 0.9% (w/v) NaCl, 1.0% (v/v) Triton X-100, 0.5% (w/v) sodium deoxycholate) with the help of Protein-A affinity medium (Mabselect Sule, GE Healthcare Life Sciences). Because of the high proteolytic activity of BPN'-Y217L, which also degrades antibodies, the immunoprecipitation of BPN'-Y217L was performed in the presence of a specific serine protease inhibitor (4 mM, Pefablock SC, Roche). Due to a specific binding of the BPN'-Y217L antibodies to unidentified cellular proteins of *B. subtilis,* the immunoprecipitation of BPN'-Y217L was only performed to assay secreted BPN'-Y217L in TCA-precipitated culture medium samples. Labelled proteins were separated by LDS-PAGE using 10% NuPage gels (Life Technologies) and visualized using a Cyclon Plus Phosphor Imager (Perkin Elmer).

**Expression and Production of Properase in *B. subtilis.*** To test *B. subtilis* Properase expression, *B. subtilis* cells over-expressing RasP *(e.g., see* Example 1.C) and wild-type (unmodified; parental) *B. subtilis* cells were grown for 5 hours in 5 mL LB. A 1.5 OD of the pre-cultures were used to inoculate 25 ml of MBU medium in shake flasks and the cells were grow at 37°C, 250 rpm, 70% humidity. Samples were taken at 18, 25, 41, 48 and 65 hours of growth. Cell densities were measured at $OD_{600nm}$ using a SpectraMax spectrophotometer (Molecular Devices, Downington, PA, USA) and the absorbance at 600nm was plotted as a function of time. The Properase enzyme expression was monitored using N-suc-AAPF-pNA substrate (herein, "AAPF"; from Sigma Chemical Co.) as described in WO 2010/144283. Briefly, whole broth was diluted 400X in the assay buffer (100 mM Tris, 0.005% Tween 80, pH 8.6) and 10 μl of the diluted samples were arrayed in micro-titer plates. The AAPF stock was diluted and the assay buffer (100 X dilution of 100 mg/ml AAPF stock in DMSO) and 190 μl of this solution were added to the microtiter plates and the absorbance of the solution was measured at 405 nm using a SpectraMax spectrophotometer. The absorbance at 405 nm was plotted as a function of time.

### B. AmyE, AmyL and BPN'-Y217L Protein Production Analysis in wild-type and modified *B. subtilis* cells.

The production of AmyE, AmyL or BPN'-Y217L in each of the modified B. *subtilis* cells lacking a *rasP* or *tepA* secretion machinery component (*i.e.,* the Δ*rasP* cells or the Δ*tepA* cells) was analyzed by LDS-PAGE after 16 hours of growth in MBU with 2.5 μg/ml chloramphenicol. To this end cells were separated from the growth medium by centrifugation and equal amounts of growth medium, corrected for the cell density, are loaded onto the gel. The amount of extracellular AmyE, AmyL or BPN'-Y217L secreted by each of the mutant *B. subtilis* cells *(i.e.,* the Δ*rasP* cells or the Δ*tepA* cells) was compared to the amount of each of these proteins secreted by the respective *B. subtilis* (wild-type) control cells with the integral secretion machinery (*i.e., B. subtilis* cells comprising the native *rasP* or *tepA* gene). Quantification of the secreted enzymes was performed using the ImageJ analysis software.

As presented in FIG. 1A and FIG. 1B, the LDS-PAGE gel and the histogram plot, respectively, show that production of AmyE, AmyL and BPN'-Y217L are decreased in the Δ *rasP* mutant cells relative to the wild-type (parental) cells. The Δ*tepA* mutant cells presented in FIG. 1A and FIG. 1B indicate that the *tepA* deletion does not affect the production of AmyE or BPN'-Y217L when compared to the wild-type (parental) cells.

### C. Expression of AmyE in wild-type *B. subtilis* cells and *B. subtilis* cells modified with an expression cassette expressing/over-expressing *rasP.*

*B. subtilis* wild-type cells, modified *B. subtilis* cells comprising "PspoVG-rasP" and modified *B. subtilis* cells comprising "PspoVG-UTR-rasP", each comprising the AmyE amylase construct ("PaprE-amyE-catR"), were inoculated over-night in 5 ml of Luria Broth containing 5 ppm of chloramphenicol. One (1) ml of pre-culture was used to inoculate 25 ml of BHI (Brain-Heart Infusion) medium in shake flasks. The experiment was performed at 37°C, 250 rpm using an Infors shaker. Time points were taken during the growth, and cell growth was measured at 600 nm.

AmyE amylase activity of whole broth was measured using the Ceralpha reagent from a Ceralpha HR kit (Megazyme, Wicklow, Ireland). The Ceralpha reagent mix was initially dissolved in 10 ml of MilliQ water, followed by the addition of 30 ml of 50 mM malate buffer, pH 5.6. The supernatant of the cultures was diluted 40 fold in MilliQ water and 10 μl of sample was added to 55 μL of diluted working substrate solution. The MTP plate was incubated for 4 minutes at room temperature after shaking. The reaction was quenched by adding 70 μl of 200 mM borate buffer, pH 10.2 (stop solution). The absorbance of the solution was measured at 400 nm using a SpectraMax spectrophotometer,and the absorbance at 400 nm was plotted as a function of time.

As set forth in FIG. 2A, the modified *B. subtilis* cells comprising and expressing/over-expressing the *rasP* gene under the control of the *PspoVG* promoter and *aprE* 5' UTR *(i.e.,* PspoVG-UTR-rasP) demonstrated improved cell growth, suggesting that the highest level of *rasP* expression positively affects the cell growth in the conditions tested. The data presented in FIG. 2B further demonstrate increased production of AmyE amylase in modified *B. subtilis* cells *(i.e.,* the "PspoVG-rasP" cells and the "PspoVG-UTR-rasP" cells) when compared to the wild-type *B. subtilis* control cells.

### D. Expression of variant PcuAmy1-v6 amylase in wild-type *B. subtilis* cells and *B. subtilis* cells modified with an expression cassette expressing/over-expressing *rasP.*

*B. subtilis* wild-type cells and modified *B. subtilis* cells comprising "PspoVG-rasP", each comprising the variant "PaprE-PcuAmy1-v6-catR" construct, were inoculated over-days in 5 ml of Luria broth containing 25 ppm of chloramphenicol. One (1) ml of pre-culture was used to inoculate 25 ml of suitable medium in shake flasks. The experiment was performed at 37°C, 250 RPM using an Infors shaker. Time points were taken during the growth to determine the activity of the amylase during growth.

The PcuAmy1-v6 amylase activity in the whole broth was measured using the Ceralpha reagent from a Ceralpha HR kit (Megazyme, Wicklow, Ireland). The Ceralpha reagent mix was initially dissolved in 10 ml of MilliQ water followed by the addition of 30 ml of 50 mM malate buffer, pH 5.6. The culture supernatants were diluted 40 fold in MilliQ water and 10 μl of sample was added to 55 μL of diluted working substrate solution. The MTP plate was incubated for 4 minutes at room temperature after shaking. The reaction was quenched by adding 70 μl of 200 mM borate buffer pH 10.2 (stop solution). The absorbance of the solution was measured at 400 nm using a SpectraMax spectrophotometer and the absorbance at 400 nm was plotted as a function of time.

As set forth in FIG. 3, the production of PcuAmy1-v6 amylase in shake flasks (corrected for cell density at $OD_{600}$ nm) demonstrates increased secretion of the amylase in the modified *B. subtilis* cells *(i.e.,* over-expressing *rasP*) when compared to the wild-type *B. subtilis* control cells.

### E. Expression of variant PcuAmy1-v6 amylase in wild-type *B. subtilis* cells and *B. subtilis* cells modified with an expression cassette expressing/over-expressing *tepA.*

*B. subtilis* wild-type cells and modified *B. subtilis* cells comprising "PspoVG-tepA", each comprising the variant "PaprE-PcuAmy1-v6-catR" construct, were also tested in shake flasks to determine the effects of over-expressing the TepA signal peptide peptidase. Thus, wild-type *B. subtilis* cells and modified *B. subtilis* cells comprising "PspoVG-tepA", were grown over-night in Luria broth medium at 37°C. One (1) ml of pre-culture was used to inoculate 25 ml of BHI (Brain-Heart Infusion) medium in shake flasks. The experiment was performed at 37°C, 250 RPM using an Infors shaker. Cell growth was measured at 600 nm and time points were taken during growth. The Amylase assay was performed as described above Example 5.D.

As shown in FIG. 4A, the cell densities of the wild-type *B. cells* and modified *B. cells* indicate that the cells have a similar growth profile. As shown in FIG. 4B, production of the amylase in the modified *B. subtilis* cells *(i.e.,* over-expressing *tepA*) was slightly decreased relative to the wild-type *B. subtilis* cells.

**F. Expression of Beta-D-glucanase (BglC) in wild-type *B. subtilis* cells and *B. subtilis* cells modified with an expression cassette expressing/over-expressing *rasP.***

*B. subtilis* wild-type cells and modified *B. subtilis* cells comprising "PspoVG-rasP", each comprising the "*PaprE-BglC-catR*" construct, were grown over-night in 5 mL of Luria broth. One (1) ml of pre-culture was used to inoculate 25 ml of BHI medium in shake flasks, and the cultures were gown at 37°C, 250 rpm to test the expression of the secreted β-D-glucanase. Cell densities of whole broth diluted 20 fold were measured at 600 nm at hourly intervals using a SpectraMax spectrophotometer, and the absorbance (at 600 nm) was plotted as a function of time, indicating that the wild-type and modified *B. subtilis* cells have similar cell densities.

The β-D-glucanase expression (*i.e.,* activity) was monitored using 4-Nitrophenyl-β-D-cellobioside substrate (Sigma Chemicals, St. Louis, MO, USA, Catalogue No. N57590). The substrate was dissolved in 1 ml of DMSO to create the stock solution at 100 mg/ml. The working substrate solution was made by diluting 35 μl of the stock solution in 10 ml of assay buffer (100 mM Tris, 0.005% Tween 80, pH 8.6). Forty (40) microliters of each culture was transferred to a 96 well microtiter plate and 180 μl of the working substrate solution was added to each well. The microtiter plate was incubated at room temperature for 2 hours and at the end of the incubation period, the absorbance of the solution was measured at 405 nm using a SpectraMax spectrophotometer. The absorbance at 405 nm was plotted as a function of the time (FIG. 5). As set forth in FIG. 5, the modified *B. subtilis* cells (*i.e.,* comprising the "PspoVG-rasP" expression construct) demonstrate an increase in the production of β-D-glucanase relative to the wild-type *B. subtilis* cells.

**G. Expression of Properase in wild-type *B. subtilis* cells and *B. subtilis* cells modified with an expression cassette expressing/over-expressing *rasP.***

*B. subtilis* wild-type cells and modified *B. subtilis* cells comprising "PspoVG-rasP", each comprising the *"PaprE-Properase-catR"* construct, were grown for 5 hours in 5 mL of Luria broth. A 1.5 OD of pre-culture was used to inoculate 25 ml of suitable medium in shake flasks and the cells were cultured at 37°C, 250 rpm, 70% humidity. Samples were taken at 18, 25, 41, 48 and 65 hours of growth. Cell densities of whole broth diluted 40 fold were measured at 600 nm using a SpectraMax spectrophotometer and the absorbance at 600 nm was plotted as a function of time (FIG. 6A). As presented in FIG. 6A, the modified *B. subtilis* cells demonstrate increased cell densities relative to the wild-type *B. subtilis* cells.

Properase expression *(i.e.,* activity) was monitored using N-suc-AAPF-pNA substrate ("AAPF"; Sigma Chemical Co.) as described in WO 2010/144283. Briefly, whole broth was diluted 40 fold in the assay buffer (100 mM Tris, 0.005% Tween 80, pH 8.6) and 10 μl of the diluted samples were arrayed in microtiter plates. The AAPF stock was diluted and the assay buffer (100 X dilution of 100 mg/ml AAPF stock in DMSO) and 190 μl of this solution were added to the microtiter plates and the absorbance of the solution was measured at 405 nm using a SpectraMax spectrophotometer. The absorbance at 405 nm was plotted as a function of time and is presented in FIG. 6B. As set forth in FIG. 6B, the production of Properase protease in the modified *B. subtilis* cells was approximately 5-fold greater than the Properase production in the wild-type *B. subtilis* control cells.

## EXAMPLE 5

## EXPRESSION OF AN AmyAc FAMILY α-AMYLASE IN *B. subtilis* Host CELLS OVER-EXPRESSING rasP POLYPEPTIDE

A bacterial α-amylase belonging to the AmyAc family (*e.g., see,* (www.ncbi.nlm.nih.gov/Structure/cdd/wrpsb.cgi?RID=CARRMWTZ01N&mode=all) was expressed in *B. subtilis* wild-type host cells *(i.e.,* unmodified; parental cells) and modified B. *subtilis* host cells *(i.e.,* modified daughter cells), wherein the modified cells comprise an expression cassette for over-expression of rasP (spoIIIAH::PspoVG-rasP), as generally described in the Examples above. More particularly, the promoter of aprE was used to drive the expression of the (AmyAc family) α-amylase (*e.g.*, $_{Pro}$aprE-α-amylase), wherein the expression cassette comprising an acetyltransferase gene (catR) was amplified on LB plates containing 25 μg/ml of chloramphenicol. Four (4) colonies from each strain *(i.e.,* wild-type parental host cells and modified daughter host cells over-expressing rasP) were used to inoculate eight (8) tubes of LB containing 25 μg/ml of chloramphenicol and grown at 37°C for four (4) hours. 0.075 OD were used to inoculate two (2) ml of 5SM12 medium in twenty-four (24) well Deep Microtiter plate, wherein the *B. subtilis* host cells were grown for forty-eight (48) hours. Time points were taken at 18, 25, 41 and 48 hours. $OD_{600}$ measurements were taken by diluting the culture 40 fold in the media used in the experiment.

The 5SM12 (5% soytone, 12% maltodextrin) medium is generally prepared as follows: 1mM sodium citrate, 0.03 mM $CaCl_2$, 0.0053% Ferric Ammonium Citrate, 0.2 mM $MnCl_2$, 0.5 mM $MgSO_4$, 75 mM $K_2HPO_4$, 25 mM $NaH_2PO_4$, 12% maltodextrin and 5% Difco Bacto soytone. The pH of the medium was adjusted to pH 7.4 (with KOH) for the BPN’

proteases and adjusted to pH 7.7 (with KOH) for the amylases. The shake flask conditions were as follows: cultures (32-35 mL) were grown in 250 mL Thomson Ultra Yield Flasks (catalogue no. 931144) with a Thomason AirOtop enhanced seals (catalogue no. 899423). For growth, the cultures were shaken at 280 rpm, 37°C with 70 % humidity (to reduce evaporation) using an Infors MultiTron shaker with a 50 mm throw.

The amylase activity of whole broth was measured using the Ceralpha reagent from a Ceralpha HR kit (Megazyme, Wicklow, Ireland). The Ceralpha reagent mix was initially dissolved in 10 ml of MilliQ water, followed by the addition of 30 ml of 50 mM malate buffer, pH 5.6. The supernatant of the cultures was diluted 40 fold in MilliQ water and 10 μl of sample was added to 55 μL of diluted working substrate solution. The MTP plate was incubated for four (4) minutes at room temperature after shaking. The reaction was quenched by adding 70 μl of 200 mM borate buffer, pH 10.2 (stop solution). The absorbance of the solution was measured at 400 nm using a SpectraMax spectrophotometer, and the absorbance at 400 nm was plotted as a function of time (FIG. 7B). As presented in FIG. 7B, the amylase production from the modified *B. subtilis* cells *(i.e.,* expressing the rasP construct) comprised an approximately 2.5-fold increase in amylase productivity relative to the unmodified (parental) *B. subtilis* cells.

**Claims**

**1.** A method for increasing the production of a protein of interest in a Gram-positive bacterial cell comprising:

(a) obtaining a modified Gram-positive bacterial cell producing an increased amount of a protein of interest, wherein the modified bacterial cell comprises a modification which increases *rasP* gene expression, wherein the modification which increases *rasP gene* expression is a modification to an endogenous chromosomal *rasP gene* or wherein the modification which increases *rasP gene* expression is an exogenous polynucleotide comprising a *rasP* gene, and
(b) culturing the modified cell under conditions such that the POI is expressed,

wherein the modified bacterial cell producing an increased amount of a POI is relative to the production of the same protein of interest in an unmodified parental Gram-positive bacterial cell.

**2.** A modified Gram-positive bacterial cell producing an increased amount of a protein of interest (POI) relative to an unmodified parental Gram-positive bacterial cell, wherein
the modified bacterial cell comprises a modification which increases rasP gene expression,
wherein the modification which increases *rasP gene* expression is a modification to an endogenous chromosomal *rasP gene* or wherein the modification which increases *rasP* gene expression is an exogenous polynucleotide comprising a *rasP* gene, and wherein the POI is an enzyme selected from the group consisting of acetyl esterases, aryl esterases, aminopeptidases, amylases, arabinases, arabinofuranosidases, carboxypeptidases, catalases, cellulases, chitinases, chymosin, cutinase, deoxyribonucleases, epimerases, esterases, α-galactosidases, α-glucanases, glucan lysases, endo-β-glucanases, glucoamylases, glucose oxidases, α-glucosidases, β-glucosidases, glucuronidases, hemicellulases, hexose oxidases, hydrolases, invertases, isomerases, laccases, lipases, lyases, mannosidases, oxidases, oxidoreductases, pectate lyases, pectin acetyl esterases, pectin depolymerases, pectin methyl esterases, pectinolytic enzymes, perhydrolases, polyol oxidases, peroxidases, phenoloxidases, phytases, polygalacturonases, proteases, rhamno-galacturonases, ribonucleases, thaumatin, transferases, transport proteins, transglutaminases, xylanases, hexose oxidases, and combinations thereof.

**3.** The method of claim 1 or the modified cell of claim 2, wherein the modification which increases *rasP* gene expression is a modification to an endogenous chromosomal *rasP* gene.

**4.** The method or the modified cell of claim 3, wherein the native promoter of the endogenous chromosomal *rasP gene* is substituted with any promoter having a higher activity than the native *rasP* promoter.

**5.** The method or the modified cell of claim 3, wherein the native promoter of the endogenous chromosomal *rasP gene* is substituted with a *spoVG* promoter or an *aprE* promoter, optionally wherein:

(a) the *spoVG* promoter comprises a nucleotide sequence comprising 95% sequence identity to SEQ ID NO: 3, or
(b) the *aprE* promoter comprises a nucleotide sequence comprising 95% sequence identity to SEQ ID NO: 4.

**6.** The method or the modified cell of claim 3, wherein the modification to an endogenous chromosomal *rasP gene* is a modification of the native 5'-untranslated region (5'-UTR) of the endogenous chromosomal *rasP* gene, optionally wherein the native *rasP* chromosomal 5-UTR is replaced with a 5-UTR comprising 95% sequence identity to the *aprE* 5'-UTR of SEQ ID NO: 5.

**7.** The method or the modified cell of claim 3, wherein the modification to an endogenous chromosomal *rasP gene* is a modification of both the native promoter and the native 5'-UTR of the endogenous chromosomal *rasP gene.*

**8.** The method of claim 1 or the modified cell of claim 2, wherein the modification which increases *rasP gene* expression is an exogenous polynucleotide comprising a *rasP* gene.

**9.** The modified cell of claim 8, wherein exogenous polynucleotide comprising the *rasP* gene is comprised within an extrachromosomal plasmid, optionally wherein:

(a) the extrachromosomal plasmid is an expression cassette, or
(b) the extrachromosomal plasmid is an integration plasmid, optionally wherein the plasmid stably integrates into the chromosome of the modified cell.

**10.** The method of claim 1 or the modified cell of claim 2, wherein the genetic modification increasing *rasP* expression is a polynucleotide comprising an exogenous *rasP open* reading frame (ORF), wherein the ORF is operably linked and under the control of a constitutive promoter, an inducible promoter or a conditional promoter, optionally wherein exogenous polynucleotide comprising the *rasP* ORF is comprised within an extrachromosomal plasmid.

**11.** The method or modified cell of any one of the preceding claims, wherein the *rasP* gene comprises a nucleic acid sequence comprising at least 60% sequence identity to open reading frame (ORF) nucleic acid sequence of SEQ ID NO: 1, optionally wherein the ORF of SEQ ID NO: 1 encodes a RasP polypeptide, wherein the RasP polypeptide is further defined as a $Zn^{2+}$ metalloprotease having site-2 protease (S2P) activity.

**12.** The method or modified cell of any one of the preceding claims, wherein the *rasP* gene encodes a RasP polypeptide comprising 60% amino acid sequence identity to a RasP polypeptide of SEQ ID NO: 2 and comprises an active site consensus sequence of SEQ ID NO: 6, which aligns with amino acid residues 16 to 26 of the RasP polypeptide of SEQ ID NO: 2; and/or
wherein the *rasP gene* encodes a RasP polypeptide comprising 80% amino acid sequence identity to a RasP polypeptide of SEQ ID NO: 2 and comprises an active site consensus sequence of SEQ ID NO: 7 HEXXH, which aligns with amino acid residues 20 to 24 of the RasP polypeptide of SEQ ID NO: 2.

**13.** The method or modified cell of any one of the preceding claims, wherein:

(a) the increased amount of the protein of interest produced, relative to the unmodified parental Gram-positive cell, is at least a 5% increase, and/or
(b) the Gram-positive bacterial cell is a member of the *Bacillus* genus; and/or
(c) the protein of interest is encoded by a gene exogenous to the modified bacterial cell or a gene endogenous to the modified bacterial cell.

**14.** The method of claim 1, wherein the protein of interest is an enzyme, optionally selected from the group consisting of acetyl esterases, aryl esterases, aminopeptidases, amylases, arabinases, arabinofuranosidases, carboxypeptidases, catalases, cellulases, chitinases, chymosin, cutinase, deoxyribonucleases, epimerases, esterases, α-galactosidases, α-glucanases, glucan lysases, endo-β-glucanases, glucoamylases, glucose oxidases, α-glucosidases, β-glucosidases, glucuronidases, hemicellulases, hexose oxidases, hydrolases, invertases, isomerases, laccases, lipases, lyases, mannosidases, oxidases, oxidoreductases, pectate lyases, pectin acetyl esterases, pectin depolymerases, pectin methyl esterases, pectinolytic enzymes, perhydrolases, polyol oxidases, peroxidases, phenoloxidases, phytases, polygalacturonases, proteases, rhamno-galacturonases, ribonucleases, thaumatin, transferases, transport proteins, transglutaminases, xylanases, hexose oxidases, and combinations thereof.

**15.** A method for obtaining a modified Gram-positive bacterial cell producing an increased amount of a protein of interest comprising:

(a) introducing into a parental Gram-positive bacterial cell at least one gene modification which increases *rasP gene* expression, wherein the modification which increases *rasP gene* expression is a modification to an endogenous chromosomal *rasP gene* or wherein the modification which increases *rasP* gene expression is an exogenous polynucleotide comprising a *rasP gene,* and
(b) selecting one or more daughter cells expressing an increased amount of a protein of interest

wherein the one or more daughter cells selected for producing an increased amount of the protein of interest are defined as modified daughter Gram-positive bacterial cells.

## Patentansprüche

**1.** Verfahren zum Erhöhen der Produktion eines Proteins von Interesse in einer grampositiven Bakterienzelle, umfassend:

(a) Erhalten einer modifizierten grampositiven Bakterienzelle, die eine erhöhte Menge eines Proteins von Interesse erzeugt,
wobei die modifizierte Bakterienzelle eine Modifikation umfasst,
die *rasP* Genexpression erhöht,
wobei die Modifikation, die *rasP* Genexpression erhöht, eine Modifikation an einem endogenen chromosomalen *rasP* Gen ist, oder wobei die Modifikation, die *rasP* Genexpression erhöht, ein exogenes Polynucleotid ist, das ein *rasP* Gen umfasst, und
(b) Kultivieren der modifizierten Zelle unter Bedingungen, unter denen das POI exprimiert wird, wobei die modifizierte Bakterienzelle, die eine erhöhte Menge eines POI erzeugt, im Verhältnis steht zu der Produktion des gleichen Proteins von Interesse in einer nicht modifizierten parentalen grampositiven Bakterienzelle.

**2.** Modifizierte grampositive Bakterienzelle, die eine im Verhältnis zu einer nicht modifizierten parentalen grampositiven Bakterienzelle erhöhte Menge eines Proteins von Interesse (POI) erzeugt, wobei die modifizierte Bakterienzelle eine Modifikation umfasst, die *rasP* Genexpression erhöht,

wobei die Modifikation, die *rasP* Genexpression erhöht, eine Modifikation an einem endogenen chromosomalen *rasP* Gen ist, oder wobei die Modifikation, die *rasP* Genexpression erhöht, ein exogenes Polynucleotid ist, das ein *rasP* Gen umfasst, und wobei das POI ein Enzym ist, das ausgewählt ist aus der Gruppe bestehend aus Acetylesterasen, Arylesterasen, Aminopeptidasen, Amylasen, Arabinasen, Arabinofuranosidasen, Carboxypeptidasen, Katalasen, Cellulasen, Chitinasen, Chymosin, Cutinase, Desoxyribonucleasen, Epimerasen, Esterasen, $\alpha$-Galactosidasen, $\alpha$-Glucanasen, Glucanlysasen, Endo-$\beta$-Glucanasen, Glucoamylasen, Glucoseoxidasen, $\alpha$-Glucosidasen, $\beta$-Glucosidasen, Glucuronidasen, Hemicellulasen, Hexoseoxidasen, Hydrolasen, Invertasen, Isomerasen, Laccasen, Lipasen, Lyasen, Mannosidasen, Oxidasen, Oxidoreduktasen, Pektatlyasen, Pektinacetylesterasen, Pektindepolymerasen, Pektinmethylesterasen, pektinolytische Enzyme, Perhydrolasen, Polyoloxidasen, Peroxidasen, Phenoloxidasen, Phytasen, Polygalacturonasen, Proteasen, Rhamno-Galacturonasen, Ribonucleasen, Thaumatin, Transferasen, Transportproteine, Transglutaminasen, Xylanasen, Hexoseoxidasen und Kombinationen davon.

**3.** Verfahren nach Anspruch 1 oder modifizierte Zelle nach Anspruch 2, wobei die Modifikation, die *rasP* Genexpression erhöht, eine Modifikation an einem endogenen chromosomalen *rasP* Gen ist.

**4.** Verfahren oder modifizierte Zelle nach Anspruch 3, wobei der native Promotor des endogenen chromosomalen *rasP* Gens substituiert ist mit einem beliebigen Promotor, der eine höhere Aktivität als der native *rasP* Promotor aufweist.

**5.** Verfahren oder modifizierte Zelle nach Anspruch 3, wobei der native Promotor des endogenen chromosomalen *rasP-Gens* substituiert ist mit einem *spoVG* Promotor oder einem *aprE* Promotor, wahlweise wobei:

(a) der *spoVG* Promotor eine Nucleotidsequenz umfasst, die 95% Sequenzidentität zu SEQ ID NO: 3 umfasst, oder
(b) der *aprE* Promotor eine Nucleotidsequenz umfasst, die 95% Sequenzidentität zu SEQ ID NO: 4 umfasst.

**6.** Verfahren oder modifizierte Zelle nach Anspruch 3, wobei die Modifikation zu einem endogenen chromosomalen *rasP* Gen eine Modifikation der nativen 5'-nicht translatierten Region (5'-UTR) des endogenen chromosomalen *rasP* Gens ist, wahlweise wobei die native *rasP* chromosomale 5'-UTR ausgetauscht ist durch eine 5'-UTR, die 95% Sequenzidentität zu der *aprE* 5'-UTR von SEQ ID NO: 5 umfasst.

**7.** Verfahren oder modifizierte Zelle nach Anspruch 3, wobei die Modifikation zu einem endogenen chromosomalen *rasP* Gen eine Modifikation sowohl des nativen Promotors als auch der nativen 5'-UTR des endogenen chromosomalen *rasP* Gens ist.

**8.** Verfahren nach Anspruch 1 oder modifizierte Zelle nach Anspruch 2, wobei die Modifikation, die *rasP* Genexpression erhöht, ein exogenes Polynucleotid ist, das ein *rasP*-Gen umfasst.

**9.** Modifizierte Zelle nach Anspruch 8, wobei das exogene Polynucleotid, das das *rasP* Gen umfasst, enthalten ist in einem extrachromosomalen Plasmid, wahlweise worin:

(a) das extrachromosomale Plasmid eine Expressionkasette ist, oder
(b) das extrachromosomale Plasmid ein Integrationsplasmid ist, wahlweise worin das Plasmid sich stabil in das Chromosom der modifizierten Zelle integriert.

**10.** Verfahren nach Anspruch 1 oder modifizierte Zelle nach Anspruch 2, wobei die genetische Modifikation, die die *rasP* Expression erhöht, ein Polynucleotid ist, das einen exogenen offenen *rasP* Leserahmen (ORF) umfasst, wobei der ORF funktionsfähig verbunden und unter der Kontrolle eines konstitutiven Promotors, eines induzierbaren Promotors oder eines konditionalen Promotors ist, wahlweise wobei das exogene Polynucleotid, welches den *rasP* ORF umfasst, in einem extrachromosomalen Plasmid enthalten ist.

**11.** Verfahren oder modifizierte Zelle nach einem der vorhergehenden Ansprüche, wobei das *rasP* Gen eine Nucleinsäuresequenz umfasst, die mindestens 60% Sequenzidentität zum offenen Leserahmen (ORF) Nucleinsäuresequenz von SEQ ID NO: 1 umfasst, wahlweise wobei der ORF von SEQ ID NO: 1 ein RasP Polypeptid codiert, wobei das RasP Polypeptid ferner als eine $Zn^{2+}$-Metalloprotease festgelegt ist, die Site-2 Protease (S2P)-Aktivität aufweist.

**12.** Verfahren oder modifizierte Zelle nach einem der vorhergehenden Ansprüche, wobei das *rasP* Gen ein RasP-Polypeptid codiert, das 60% Aminosäuresequenz-Identität zu einem RasP Polypeptid von SEQ ID NO: 2 umfasst und eine Konsensussequenz des aktiven Zentrums von SEQ ID NO: 6 umfasst, die mit Aminosäureresten 16 bis 26 des RasP Polypeptids von SEQ ID NO: 2 übereinstimmt; und/oder
wobei das *rasP-Gen* ein RasP Polypeptid codiert, das 80% Aminosäuresequenz-Identität zu einem RasP Polypeptid von SEQ ID NO: 2 umfasst, und eine Konsensussequenz der aktiven Stelle von SEQ ID NO: 7 HEXXH umfasst, die mit Aminosäureresten 20 bis 24 des RasP Polypeptids von SEQ ID NO: 2 übereinstimmt.

**13.** Verfahren oder modifizierte Zelle nach einem der vorhergehenden Ansprüche, wobei:

(a) die erhöhte Menge des erzeugten Proteins von Interesse im Verhältnis zu der nicht modifizierten parentalen grampositiven Zelle mindestens eine Erhöhung von 5% ist, und/oder
(b) die grampositive bakterielle Zelle ein Vertreter der Gattung *Bacillus* ist; und/oder
(c) das Proteins von Interesse codiert wird durch ein zu der modifizierten bakteriellen Zelle exogenes Gen oder durch ein zu der modifizierten bakteriellen Zelle endogenes Gen.

**14.** Verfahren nach Anspruch 1, wobei das Proteins von Interesse ein Enzym ist, wahlweise ausgewählt aus der Gruppe bestehend aus Acetylesterasen, Arylesterasen, Aminopeptidasen, Amylasen, Arabinasen, Arabinofuranosidasen, Carboxypeptidasen, Katalasen, Cellulasen, Chitinasen, Chymosin, Cutinase, Desoxyribonucleasen, Epimerasen, Esterasen, $\alpha$-Galactosidasen, $\alpha$-Glucanasen, Glucanlysasen, Endo-ß-Glucanasen, Glucoamylasen, Glucoseoxidasen, $\alpha$-Glucosidasen, $\beta$-Glucosidasen, Glucuronidasen, Hemicellulasen, Hexoseoxidasen, Hydrolasen, Invertasen, Isomerasen, Laccasen, Lipasen, Lyasen, Mannosidasen, Oxidasen, Oxidoreduktasen, Pektatlyasen, Pektinacetylesterasen, Pektindepolymerasen, Pektinmethylesterasen, pektinolytische Enzyme, Perhydrolasen, Polyoloxidasen, Peroxidasen, Phenoloxidasen, Phytasen, Polygalacturonasen, Proteasen, Rhamno-Galacturonasen, Ribonucleasen, Thaumatin, Transferasen, Transportproteine, Transglutaminasen, Xylanasen, Hexoseoxidasen und Kombinationen davon.

**15.** Verfahren zum Erhalten einer modifizierten grampositiven Bakterienzelle, die eine erhöhte Menge eines Proteins von Interesse erzeugt, umfassend:

(a) Einführen in eine parentale grampositive Bakterienzelle mindestens eine Genmodifikation, die *rasP*-Genexpression erhöht, wobei die Modifikation, die *rasP* Genexpression erhöht, eine Modifikation an einem endogenen chromosomalen *rasP* Gen ist oder wobei die Modifikation, die rasP-Genexpression erhöht, ein exogenes, ein exogenes *rasP* Gen umfassendes Polynucleotid ist, und
(b) Auswählen einer oder mehrerer Tochterzellen, die eine erhöhte Menge eines Proteins von Interesse exprimieren,

wobei die einen oder mehreren Tochterzellen, die zum Erzeugen einer erhöhten Menge des Proteins von Interesse ausgewählt werden, als modifizierte grampositive bakterielle Tochterzellen festgelegt sind.

## Revendications

**1.** Méthode pour l'augmentation de la production d'une protéine intéressante dans une cellule bactérienne Gram-positif comprenant :

(a) l'obtention d'une cellule bactérienne Gram-positif modifiée produisant une quantité accrue d'une protéine intéressante,
dans laquelle la cellule bactérienne modifiée comprend une modification qui augmente l'expression d'un gène *rasP*,
dans laquelle la modification qui augmente l'expression d'un gène *rasP* est une modification sur un gène *rasP* chromosomique endogène ou dans laquelle la modification qui augmente l'expression d'un gène *rasP* est un polynucléotide exogène comprenant un gène *rasP*, et
(b) la culture de la cellule modifiée dans des conditions telles que la POI est exprimée, dans laquelle la cellule bactérienne modifiée produisant une quantité accrue d'une POI est relativement à la production de la même protéine intéressante dans une cellule bactérienne Gram-positif parentale non modifiée.

**2.** Cellule bactérienne Gram-positif modifiée produisant une quantité accrue d'une protéine intéressante (POI) relativement à une cellule bactérienne Gram-positif parentale non modifiée, où la cellule bactérienne modifiée comprend une modification qui augmente l'expression d'un gène *RasP,* dans laquelle la modification qui augmente l'expression d'un gène *RasP* est une modification sur un gène *rasP* chromosomique endogène ou dans laquelle la modification qui augmente l'expression d'un gène *RasP* est un polynucléotide exogène comprenant un gène *rasP*, et dans laquelle la POI est une enzyme choisie dans le groupe constitué de: acétylestérases, arylestérases, aminopeptidases, amylases, arabinases, arabinofuranosidases, carboxypeptidases, catalases, cellulases, chitinases, chymosine, cutinase, désoxyribonucléases, épimérases, estérases, $\alpha$-galactosidases, $\alpha$-glucanases, glucane lysases, endo-$\beta$-glucanases, glucoamylases, glucose oxydases, $\alpha$-glucosidases, $\beta$-glucosidases, glucuronidases, hémicellulases, hexose oxydases, hydrolases, invertases, isomérases, laccases, lipases, lyases, mannosidases, oxydases, oxydoréductases, pectate lyases, pectine acétylestérases, pectine dépolymérases, pectine méthylestérases, enzymes pectinolytiques, perhydrolases, polyol oxydases, peroxydases, phénoloxydases, phytases, polygalacturonases, protéases, rhamno-galacturonases, ribonucléases, thaumatine, transférases, protéines de transport, transglutaminases, xylanases, hexose oxydases et des combinaisons de celles-ci.

**3.** Méthode selon la revendication 1 ou cellule modifiée selon la revendication 2, dans laquelle la modification qui augmente l'expression d'un gène *rasP* est une modification sur un gène *rasP* chromosomique endogène.

**4.** Méthode ou cellule modifiée selon la revendication 3, dans laquelle le promoteur natif du gène *rasP* chromosomique endogène est substitué avec tout promoteur ayant une activité supérieure à celle du promoteur natif de *rasP.*

**5.** Méthode ou cellule modifiée selon la revendication 3, dans laquelle le promoteur natif du gène *rasP* chromosomique endogène est substitué avec un promoteur *spoVG* ou un promoteur *aprE,* optionnellement dans laquelle :

(a) le promoteur *spoVG* comprend une séquence de nucléotides comprenant 95% d'identité de séquence avec la SEQ ID NO : 3, ou
(b) le promoteur *aprE* comprend une séquence de nucléotides comprenant 95% d'identité de séquence avec la SEQ ID NO : 4.

**6.** Méthode ou cellule modifiée selon la revendication 3, dans laquelle la modification sur un gène *rasP* chromosomique endogène est une modification de la région 5'-non traduite (5'-UTR) native du gène *rasP* chromosomique endogène, optionnellement dans laquelle la 5'-UTR chromosomique de *rasP* native est remplacée avec une 5'-UTR comprenant 95% d'identité de séquence avec la 5'-UTR de *aprE* de la SEQ ID NO : 5.

**7.** Méthode ou cellule modifiée selon la revendication 3, dans laquelle la modification sur un gène *rasP* chromosomique endogène est une modification à la fois du promoteur natif et de la 5'-UTR native du gène *rasP* chromosomique endogène.

**8.** Méthode selon la revendication 1 ou cellule modifiée selon la revendication 2, dans laquelle la modification qui augmente l'expression d'un gène *rasP* est un polynucléotide exogène comprenant un gène *rasP.*

**9.** Cellule modifiée selon la revendication 8, dans laquelle le polynucléotide exogène comprenant le gène *rasP* est compris dans un plasmide extrachromosomique, optionnellement dans laquelle :

(a) le plasmide extrachromosomique est une cassette d'expression, ou
(b) le plasmide extrachromosomique est un plasmide d'intégration, optionnellement où le plasmide s'intègre de façon stable dans le chromosome de la cellule modifiée.

**10.** Méthode selon la revendication 1 ou cellule modifiée selon la revendication 2, dans laquelle la modification génétique augmentant l'expression de *rasP* est un polynucléotide comprenant un cadre de lecture ouvert (ORF) de *rasP* exogène, où le ORF est lié de manière fonctionnelle à et sous le contrôle d'un promoteur constitutif, d'un promoteur inductible ou d'un promoteur conditionnel, optionnellement dans laquelle le polynucléotide exogène comprenant le ORF de *rasP* est compris dans un plasmide extrachromosomique.

**11.** Méthode ou cellule modifiée selon l'une quelconque des revendications précédentes, dans laquelle le gène *rasP* comprend une séquence d'acide nucléique comprenant au moins 60% d'identité de séquence avec la séquence d'acide nucléique du cadre de lecture ouvert (ORF) de la SEQ ID NO: 1, optionnellement dans laquelle le ORF de la SEQ ID NO: 1 code pour un polypeptide RasP, dans laquelle le polypeptide RasP est en outre défini comme une $Zn^{2+}$ métalloprotéase ayant une activité de site-2 protéase (S2P).

**12.** Méthode ou cellule modifiée selon l'une quelconque des revendications précédentes, dans laquelle le gène *rasP* code pour un polypeptide RasP comprenant 60% d'identité de séquence d'acides aminés avec un polypeptide RasP de la SEQ ID NO: 2 et comprend une séquence consensus de site actif de la SEQ ID NO: 6, qui s'aligne avec les résidus d'acides aminés 16 à 26 du polypeptide RasP de la SEQ ID NO: 2; et/ou

dans laquelle le gène *rasP* code pour un polypeptide RasP comprenant 80% d'identité de séquence d'acides aminés avec un polypeptide RasP de la SEQ ID NO: 2 et comprend une séquence consensus de site actif de la SEQ ID NO: 7 HEXXH, qui s'aligne avec les résidus d'acides aminés 20 à 24 du polypeptide RasP de la SEQ ID NO: 2.

**13.** Méthode ou cellule modifiée selon l'une quelconque des revendications précédentes, dans laquelle :

(a) la quantité accrue de la protéine intéressante produite, relativement à la cellule Gram-positif parentale non modifiée, est au moins une augmentation de 5%, et/ou
(b) la cellule bactérienne Gram-positif est un membre du genre Bacillus ; et/ou
(c) la protéine intéressante est codée par un gène exogène à la cellule bactérienne modifiée ou un gène endogène à la cellule bactérienne modifiée.

**14.** Méthode selon la revendication 1, dans laquelle la protéine intéressante est une enzyme, optionnellement choisie dans le groupe constitué de: acétylestérases, arylestérases, aminopeptidases, amylases, arabinases, arabinofuranosidases, carboxypeptidases, catalases, cellulases, chitinases, chymosine, cutinase, désoxyribonucléases, épimérases, estérases, α-galactosidases, α-glucanases, glucane lysases, endo-β-glucanases, glucoamylases, glucose oxydases, α-glucosidases, β-glucosidases, glucuronidases, hémicellulases, hexose oxydases, hydrolases, invertases, isomérases, laccases, lipases, lyases, mannosidases, oxydases, oxydoréductases, pectate lyases, pectine acétylestérases, pectine dépolymérases, pectine méthylestérases, enzymes pectinolytiques, perhydrolases, polyol oxydases, peroxydases, phénoloxydases, phytases, polygalacturonases, protéases, rhamno-galacturonases, ribonucléases, thaumatine, transférases, protéines de transport, transglutaminases, xylanases, hexose oxydases et des combinaisons de celles-ci.

**15.** Méthode pour l'obtention d'une cellule bactérienne Gram-positif modifiée produisant une quantité accrue d'une protéine intéressante comprenant :

(a) l'introduction dans une cellule bactérienne Gram-positif parentale d'au moins une modification de gène qui augmente l'expression d'un gène *rasP*, dans laquelle la modification qui augmente l'expression d'un gène *rasP* est une modification sur un gène *rasP* chromosomique endogène ou dans laquelle la modification qui augmente l'expression d'un gène *rasP* est un polynucléotide exogène comprenant un gène *rasP*, et
(b) la sélection d'une ou de plusieurs cellules filles exprimant une quantité accrue d'une protéine intéressante

dans laquelle les une ou plusieurs cellules filles choisies pour la production d'une quantité accrue de la protéine intéressante sont définies comme des cellules bactériennes Gram-positif filles modifiées.

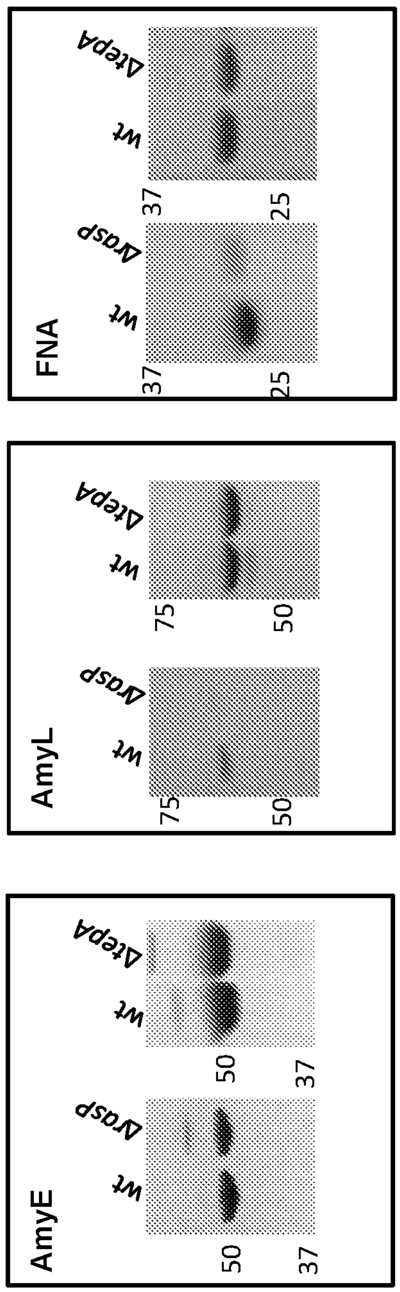
AmyE
wt
Δrasp
wt
ΔtepA
50
37
50
37
AmyL
wt
Δrasp
wt
ΔtepA
75
50
75
50
FNA
wt
Δrasp
wt
ΔtepA
37
25
37
25

FIG. 1A

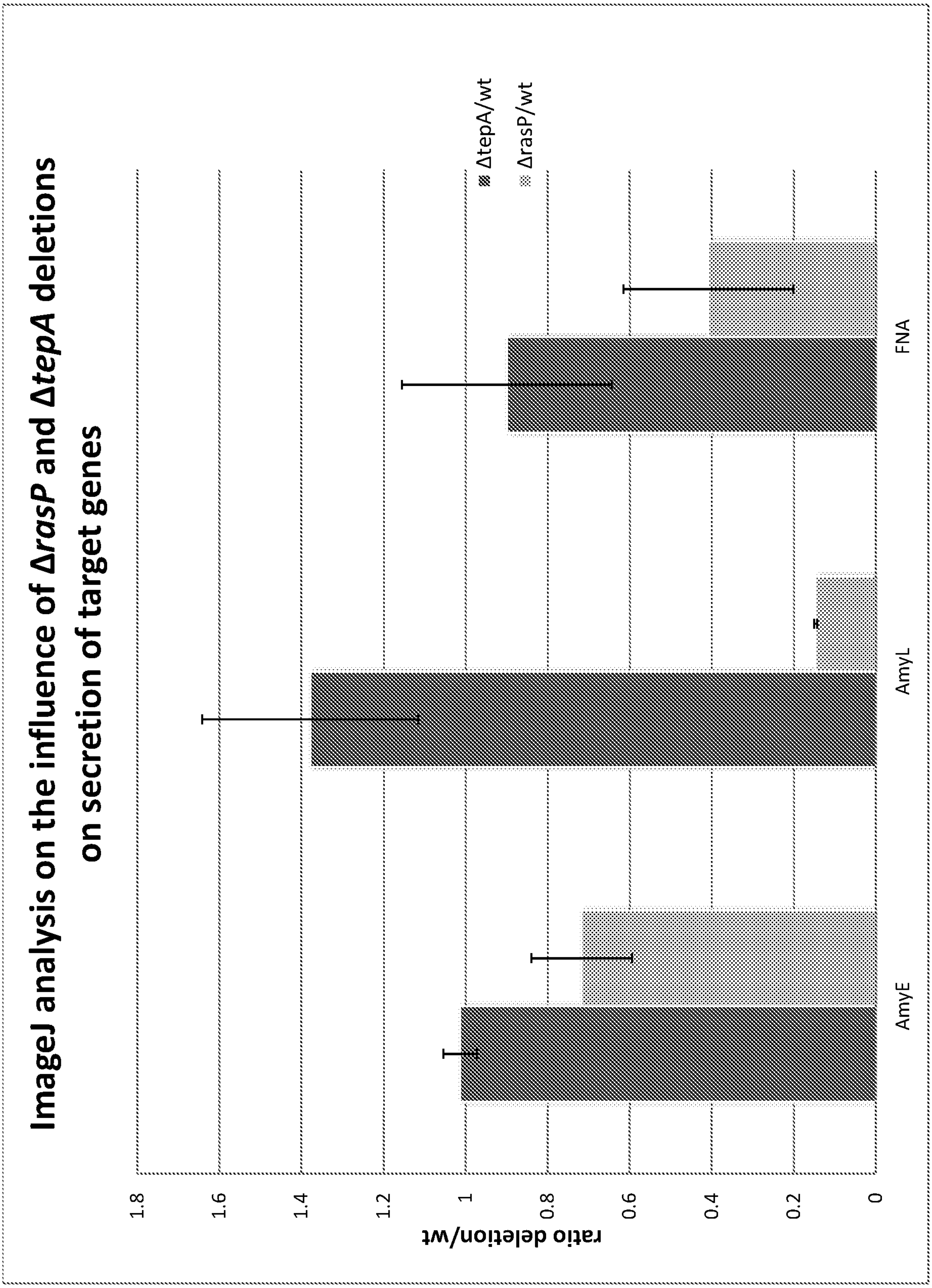
ImageJ analysis on the influence of ΔrasP and ΔtepA deletions on secretion of target genes
ratio deletion/wt
0
0.2
0.4
0.6
0.8
1
1.2
1.4
1.6
1.8
AmyE
AmyL
FNA
ΔtepA/wt
ΔrasP/wt

FIG. 1B

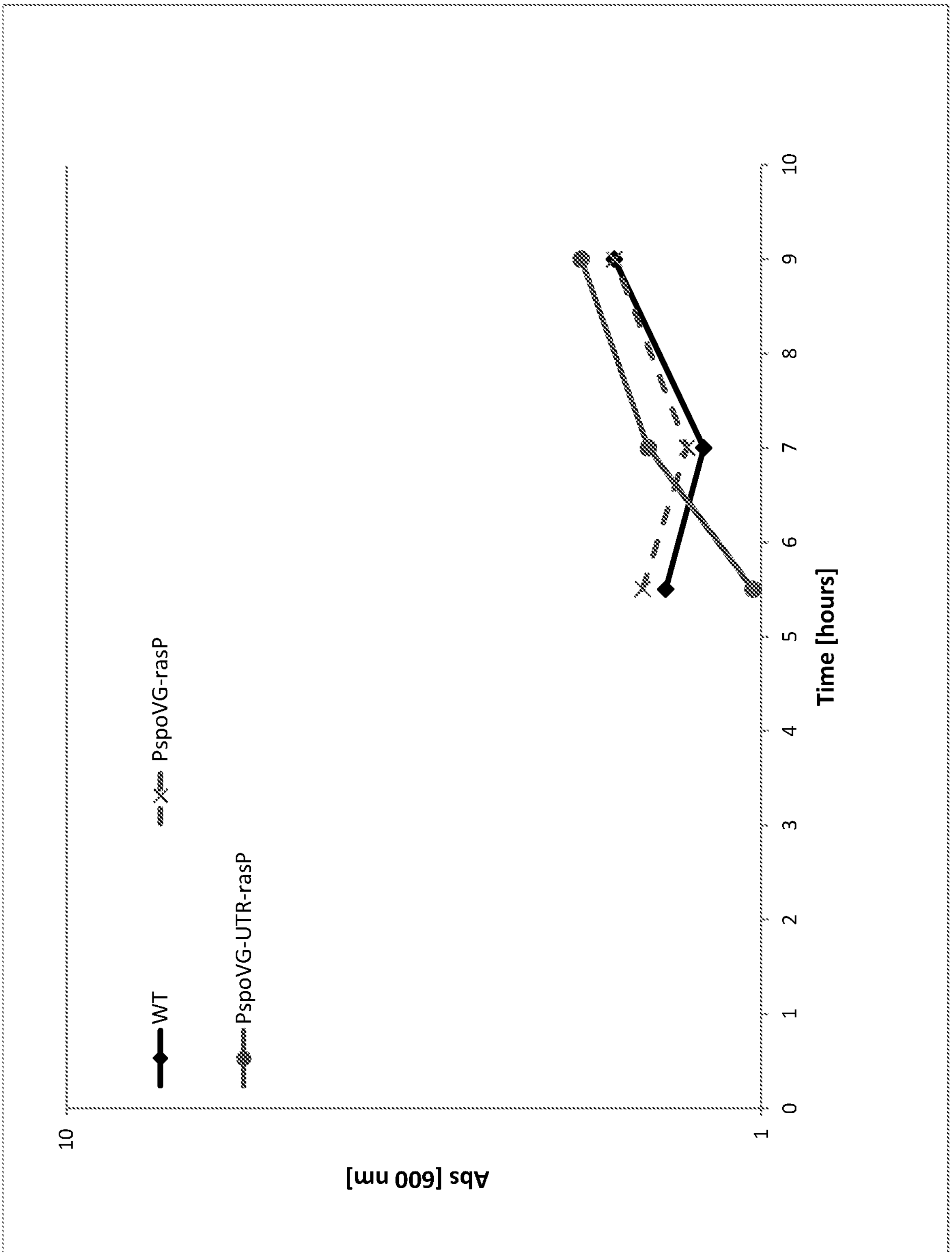

WT
PspoVG-rasP
PspoVG-UTR-rasP
Abs [600 nm]
10
1
0
1
2
3
4
5
6
7
8
9
10
Time [hours]


FIG. 2A

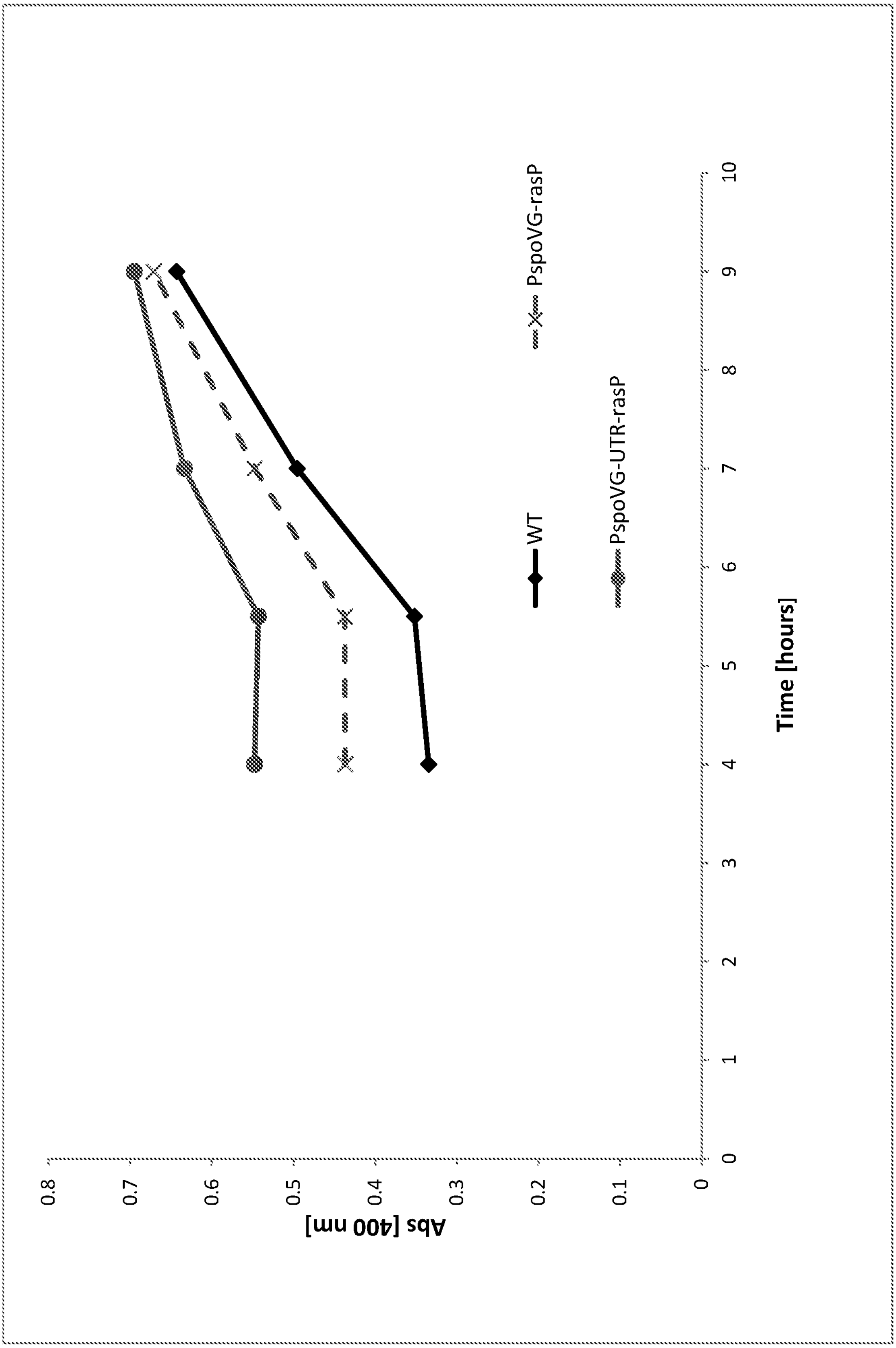
Abs [400 nm]
0
0.1
0.2
0.3
0.4
0.5
0.6
0.7
0.8
0
1
2
3
4
5
6
7
8
9
10
Time [hours]
WT
PspoVG-rasP
PspoVG-UTR-rasP

FIG. 2B

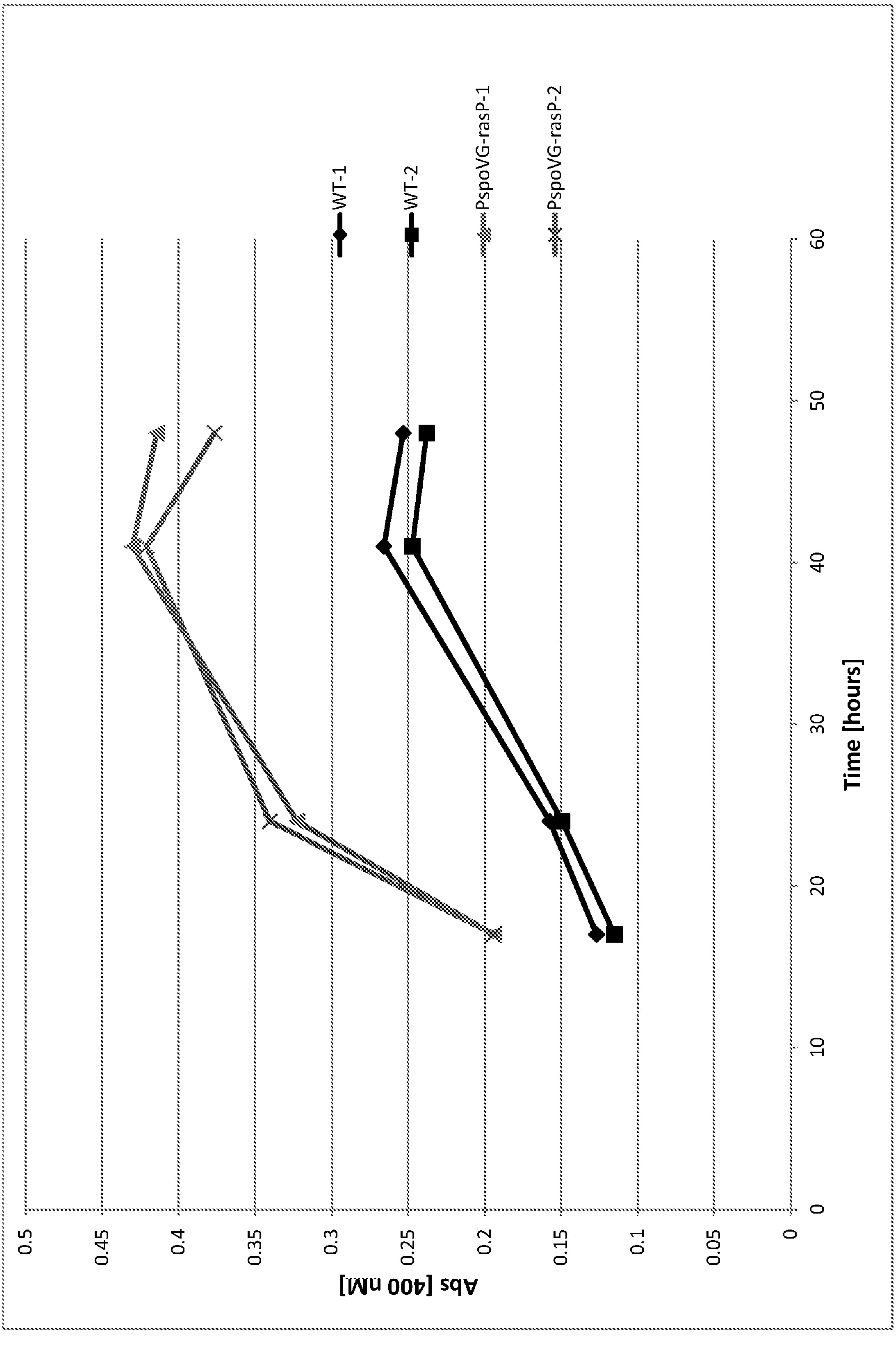
Abs [400 nM]
0.5
0.45
0.4
0.35
0.3
0.25
0.2
0.15
0.1
0.05
0
0
10
20
30
40
50
60
Time [hours]
WT-1
WT-2
PspoVG-rasP-1
PspoVG-rasP-2

FIG. 3

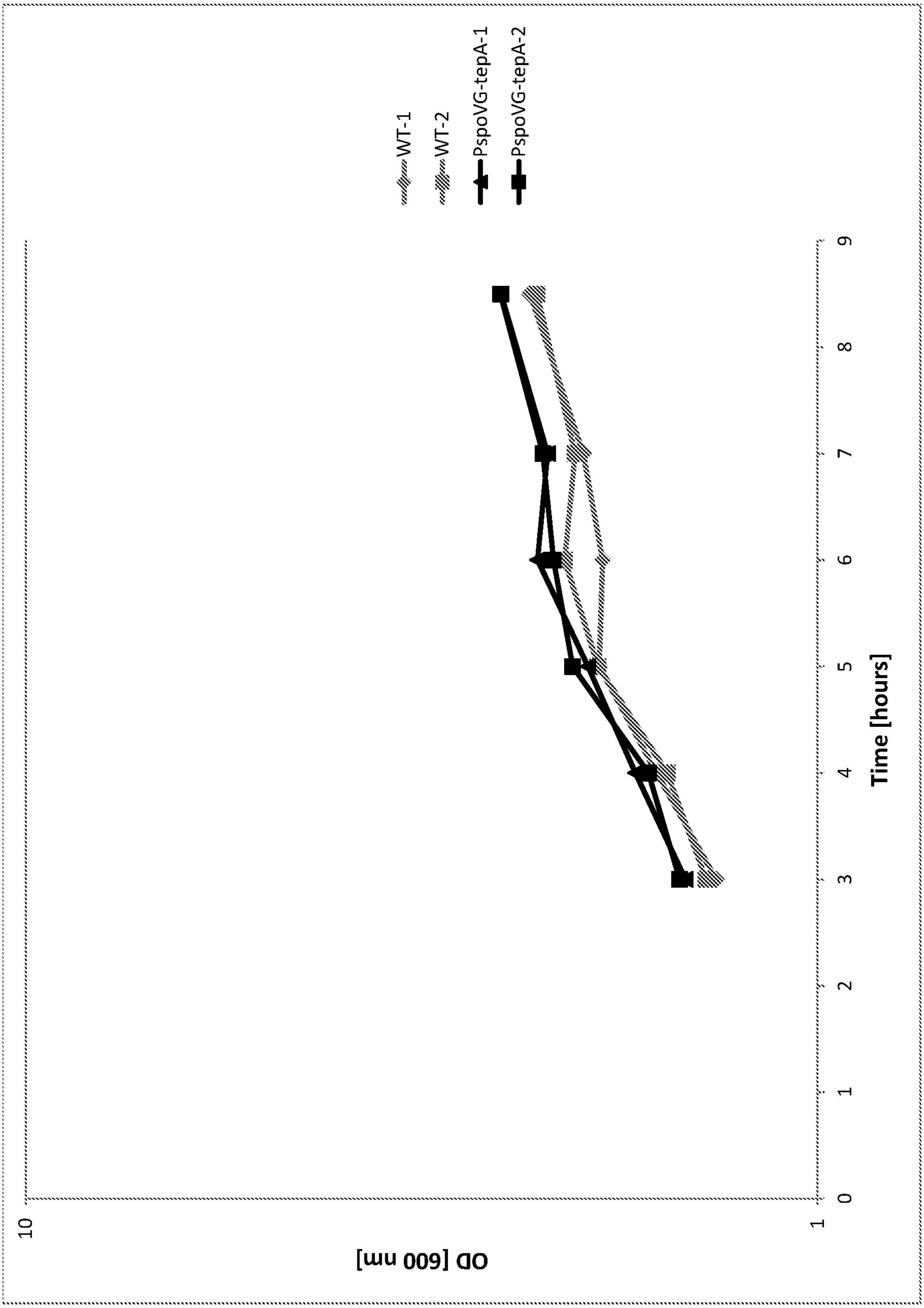
OD [600 nm]
10
1
0
1
2
3
4
5
6
7
8
9
Time [hours]
WT-1
WT-2
PspoVG-tepA-1
PspoVG-tepA-2

FIG. 4A

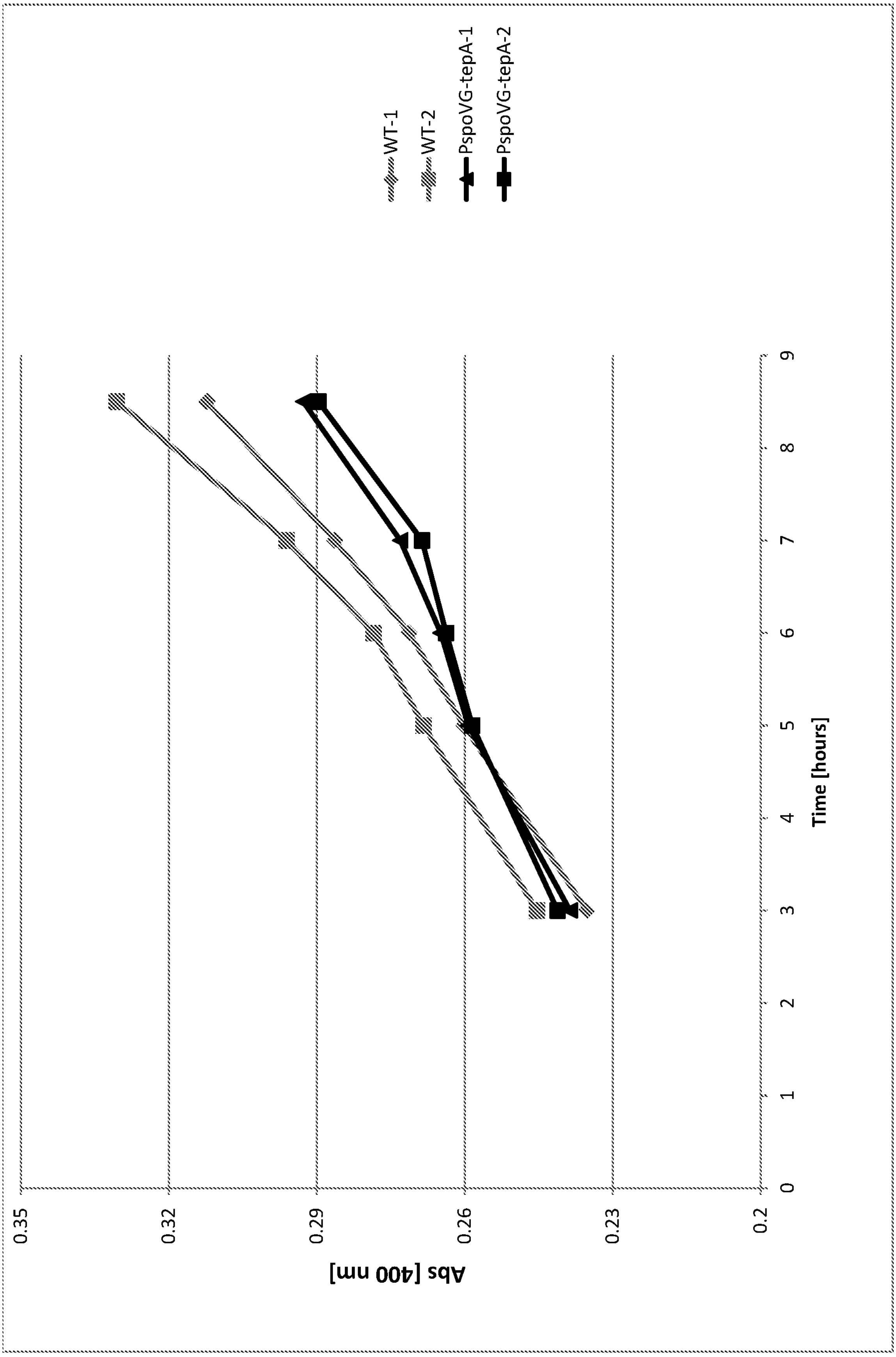
WT-1
WT-2
PspoVG-tepA-1
PspoVG-tepA-2
Abs [400 nm]
0.35
0.32
0.29
0.26
0.23
0.2
Time [hours]
0
1
2
3
4
5
6
7
8
9

FIG. 4B

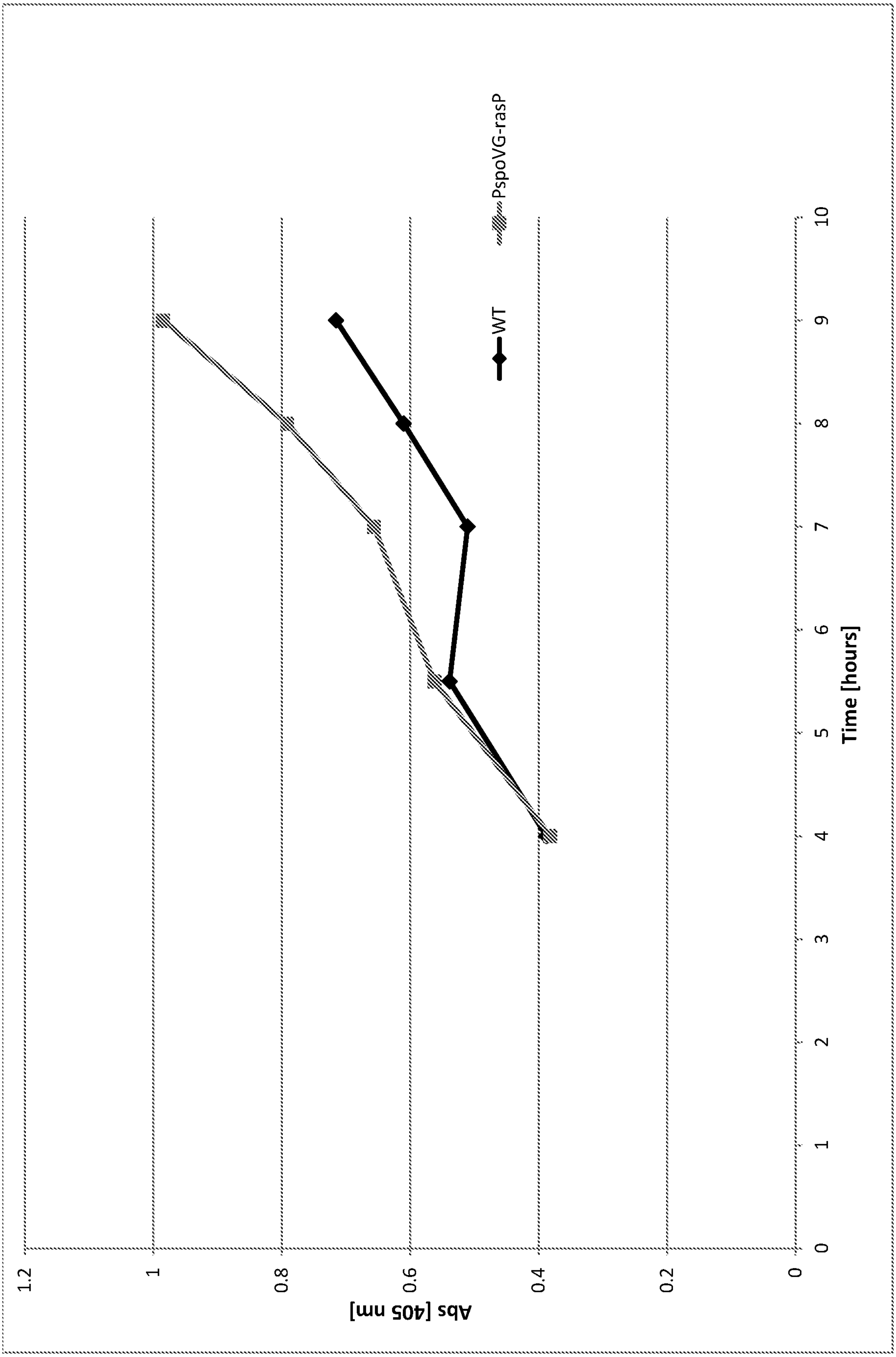
Abs [405 nm]
1.2
1
0.8
0.6
0.4
0.2
0
0
1
2
3
4
5
6
7
8
9
10
Time [hours]
WT
PspoVG-rasP

FIG. 5

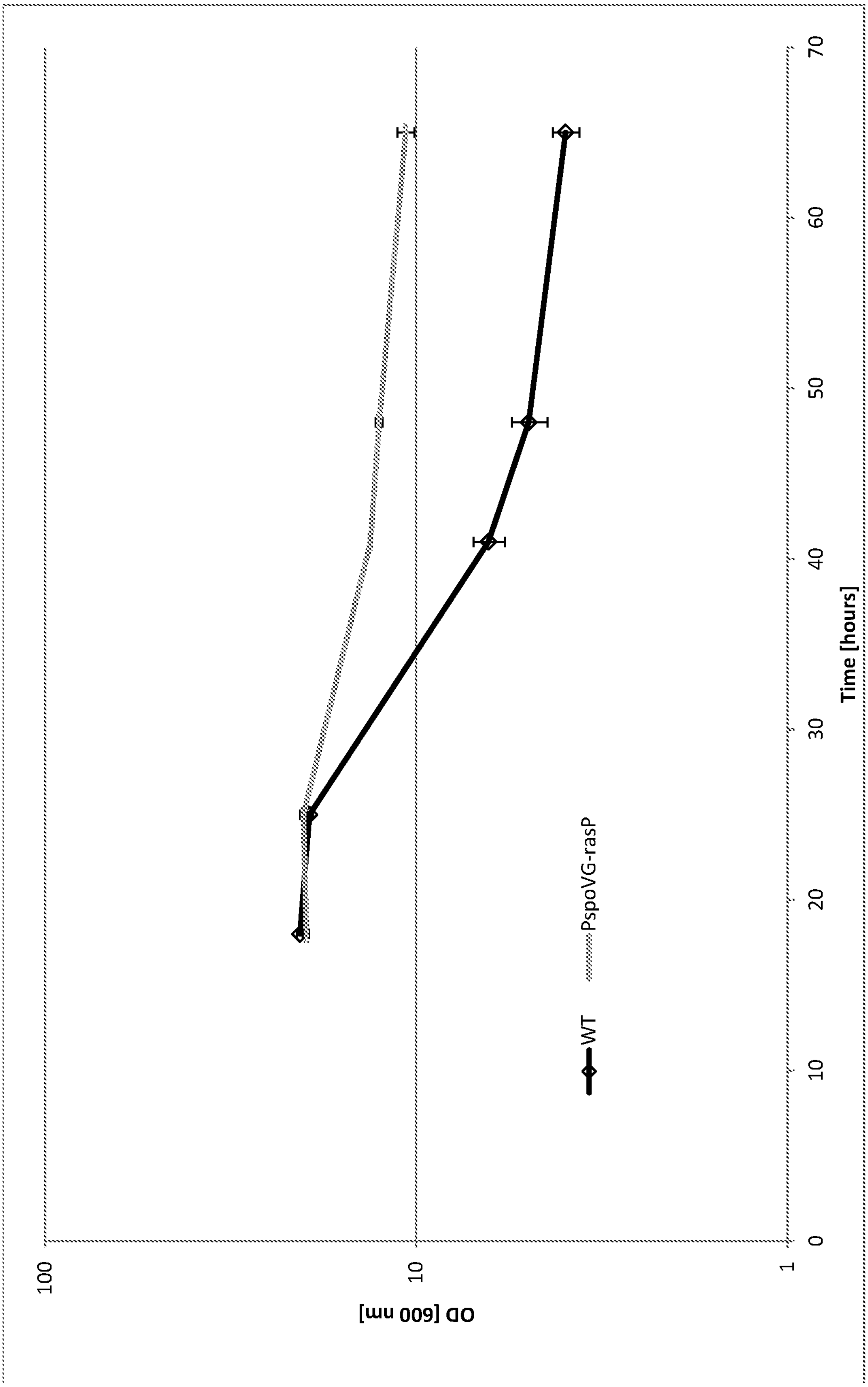


FIG. 6A

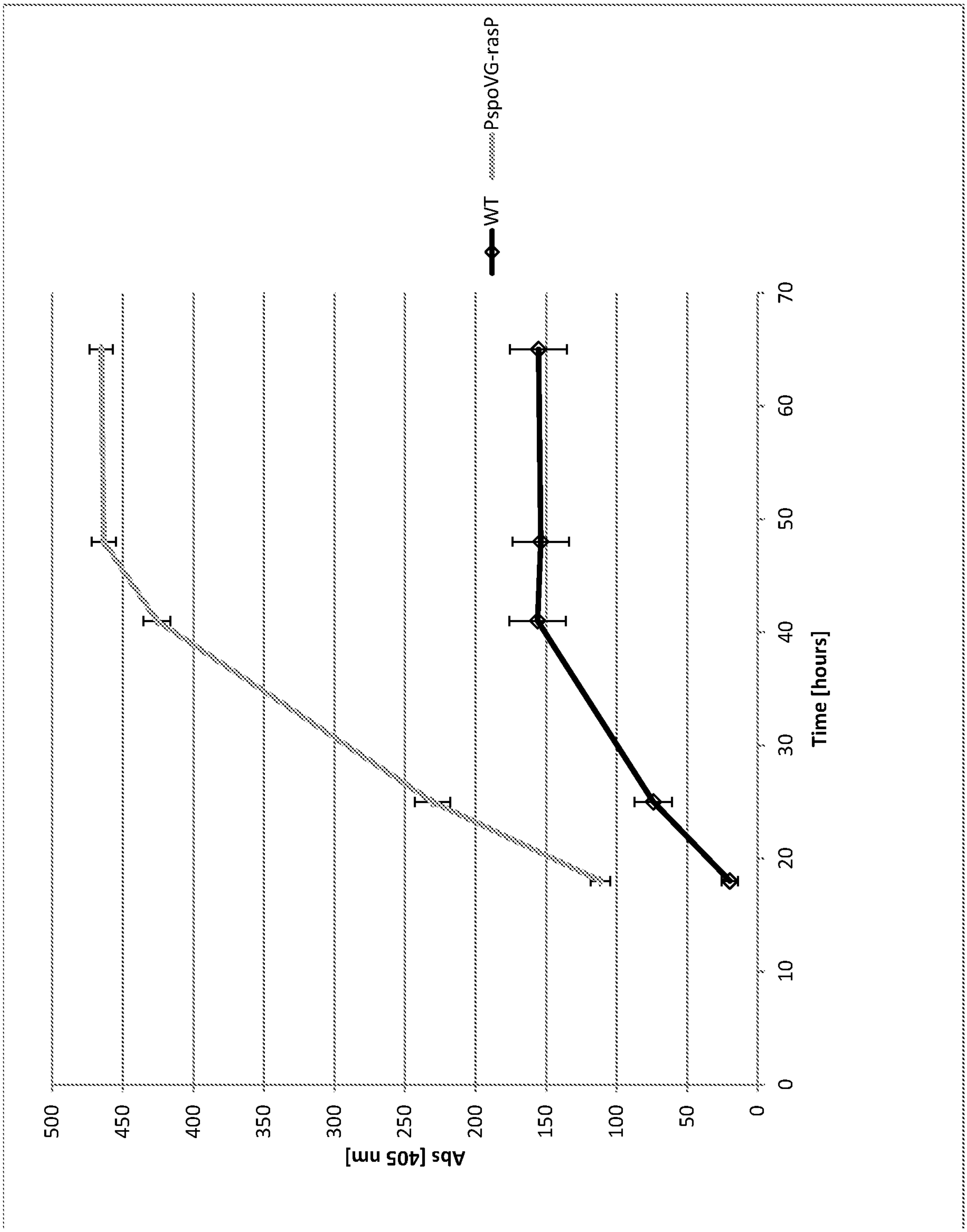
Abs [405 nm]
500
450
400
350
300
250
200
150
100
50
0
0
10
20
30
40
50
60
70
Time [hours]
WT
PspoVG-rasP

FIG. 6B

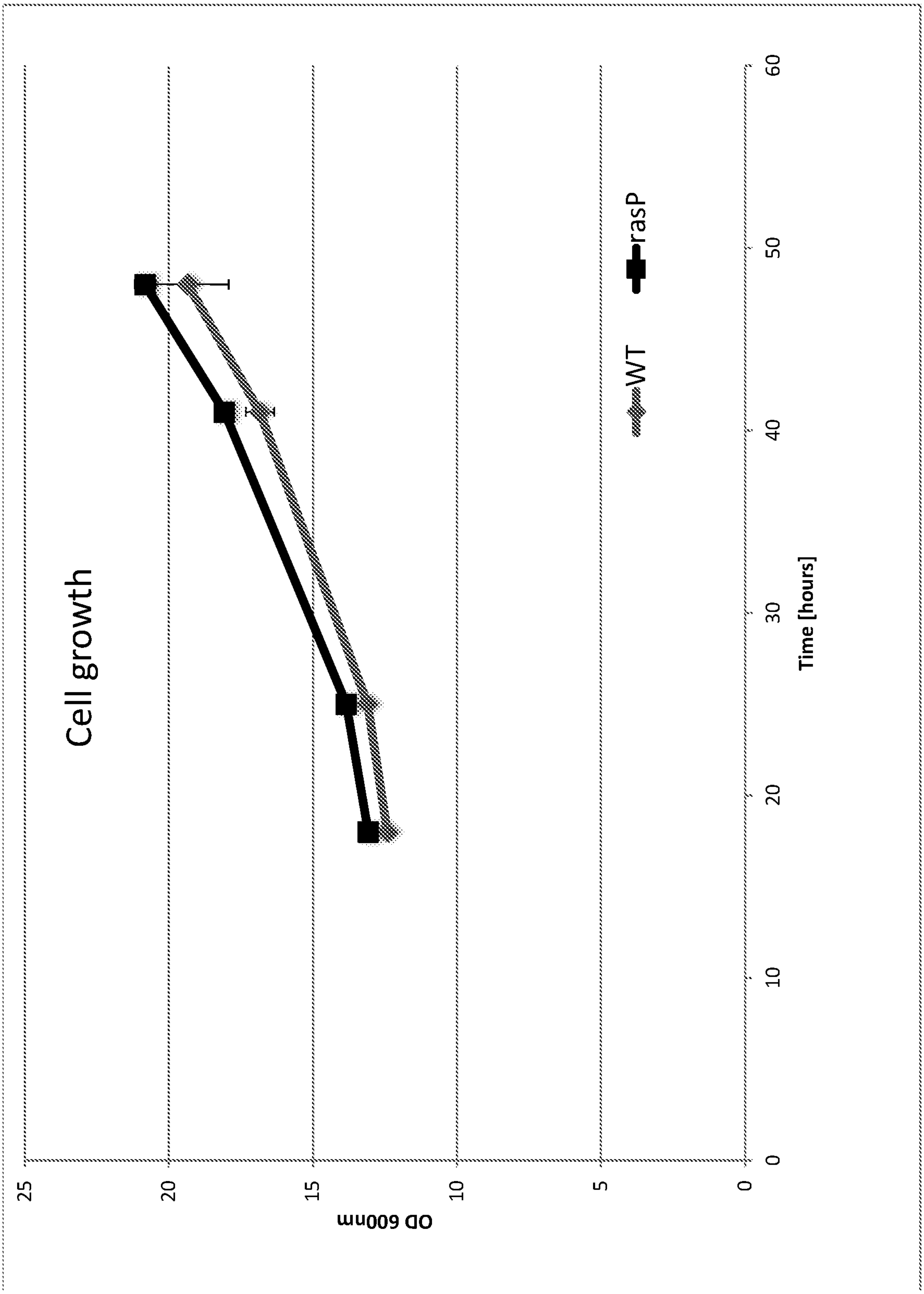
Cell growth
OD 600nm
0
5
10
15
20
25
Time [hours]
0
10
20
30
40
50
60
WT
rasP

FIG. 7A

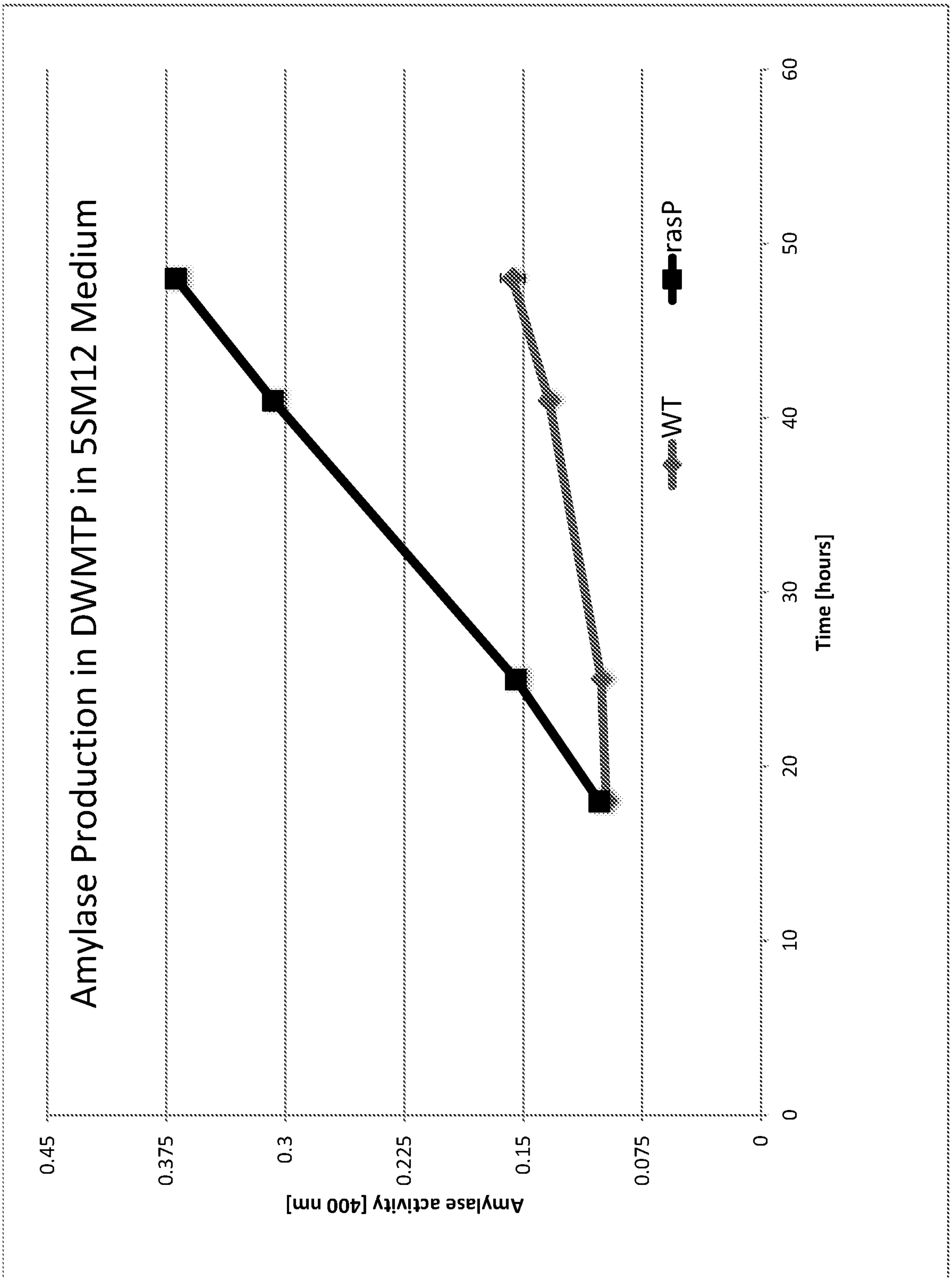
Amylase Production in DWMTP in 5SM12 Medium
Amylase activity [400 nm]
0.45
0.375
0.3
0.225
0.15
0.075
0
Time [hours]
0
10
20
30
40
50
60
WT
rasP

FIG. 7B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 62387578
- US 62308440
- WO 200151643 PCT
- US 2000035312 W
- WO 2013086219 PCT
- US 5264366 A
- WO 2010144283 A
- US 62387578 B
- US 62308440 B

SEQUENCE LISTING

<110> DANISCO

<120> ENHANCED PROTEIN PRODUCTION AND METHODS THEREOF

<130> NB40883WOPCT

<150> US62/387,578
<151> 2015-12-23

<150> US62/308,440
<151> 2016-03-16

<160> 46

<170> PatentIn version 3.5

<210> 1
<211> 1269
<212> DNA
<213> Bacillus subtilis

<400> 1

```
atgttcgtga atacagttat agcgtttatc attattttcg gaacgctcgt tttcttccat      60
gaactgggcc atttattgct agcccaaaga gcgggaattc tctgccgtga atttgcgatc     120
ggcttcggtc caaagatttt ttctttcaaa aaaaatgaaa cagtttatac gatcaggctg     180
cttccggtcg gcggatttgt tcgtatggcc ggcgaagatc cggaaatgat tgaggtgaaa     240
cccggataca cggtcgggct tctgtttaat aaggaagatc aagttgagaa agtcatcatc     300
aatcaaaagg aaaaatatcc ggatgcttta gtcattgaag tggaaacagc ggatctagag     360
catgacatga agatcaccgg ttatgaacag gggaaagagg acgaactttc cagctttact     420
gtcagcgaaa catccttttt tattgtagac ggagaagaag tgcagattgc gccgtataat     480
cgccaatttg gttccaaacc tgtatggcag cggattaaag caattgctgc agggccgatt     540
atgaacttta ttttagctta cgtcatttta gtgatgcttg ggctgattca aggcgtaccg     600
tcaaatgaac ctatgctcgg gcagctgaca gacaatggac gggcggctga agcagggcta     660
aaagaagggg attatatcca aagcattaac ggagagaaaa tgaggtcttg gactgacatt     720
gtctccgctg taaaagaaaa cccggagaaa gaaatggacg ttgcagtaaa aagagataac     780
aaaacgcttc atatttcggt gactccggaa gctgttaaag atgagaacaa aaaaacaatc     840
ggacgtttcg gttcctatgc gccgactgaa aaaggcgtac tctcagcggt tgcttacggc     900
gcgacatcaa cagttgatgt caccaaagcc attttaacca atctgagcaa attagtaaca     960
ggccaattta aactcgatat gctgtcaggt cctgtcggca tatatgacat gacagaccaa    1020
gtggcgaaaa cagggatcgt gaacttattt cagtttgcgg cgtttttaag cattaacctt    1080
gggattgtca acctgcttcc gattccggca cttgacggag gaagactgtt gtttctattt    1140
attgaagcga ttcggggcaa accgattaac agggaaaaag aagcatttgt tgtgtttatc    1200

ggagtagctt tcttaatgct tcttatgctg gttgtcacat ggaacgatat ccagcggctg    1260
tttttgtaa                                                            1269
```

<210> 2
<211> 422
<212> PRT
<213> Bacillus subtilis

<400> 2

```
Met Phe Val Asn Thr Val Ile Ala Phe Ile Ile Ile Phe Gly Thr Leu
1               5                   10                  15

Val Phe Phe His Glu Leu Gly His Leu Leu Leu Ala Gln Arg Ala Gly
            20                  25                  30

Ile Leu Cys Arg Glu Phe Ala Ile Gly Phe Gly Pro Lys Ile Phe Ser
        35                  40                  45

Phe Lys Lys Asn Glu Thr Val Tyr Thr Ile Arg Leu Leu Pro Val Gly
    50                  55                  60

Gly Phe Val Arg Met Ala Gly Glu Asp Pro Glu Met Ile Glu Val Lys
65                  70                  75                  80

Pro Gly Tyr Thr Val Gly Leu Leu Phe Asn Lys Glu Asp Gln Val Glu
                85                  90                  95

Lys Val Ile Ile Asn Gln Lys Glu Lys Tyr Pro Asp Ala Leu Val Ile
            100                 105                 110

Glu Val Glu Thr Ala Asp Leu Glu His Asp Met Lys Ile Thr Gly Tyr
        115                 120                 125

Glu Gln Gly Lys Glu Asp Glu Leu Ser Ser Phe Thr Val Ser Glu Thr
    130                 135                 140

Ser Phe Phe Ile Val Asp Gly Glu Glu Val Gln Ile Ala Pro Tyr Asn
145                 150                 155                 160

Arg Gln Phe Gly Ser Lys Pro Val Trp Gln Arg Ile Lys Ala Ile Ala
                165                 170                 175

Ala Gly Pro Ile Met Asn Phe Ile Leu Ala Tyr Val Ile Leu Val Met
            180                 185                 190

Leu Gly Leu Ile Gln Gly Val Pro Ser Asn Glu Pro Met Leu Gly Gln
        195                 200                 205
```

```
Leu Thr Asp Asn Gly Arg Ala Ala Glu Ala Gly Leu Lys Glu Gly Asp
    210                 215                 220

Tyr Ile Gln Ser Ile Asn Gly Glu Lys Met Arg Ser Trp Thr Asp Ile
225                 230                 235                 240

Val Ser Ala Val Lys Glu Asn Pro Glu Lys Glu Met Asp Val Ala Val
                245                 250                 255

Lys Arg Asp Asn Lys Thr Leu His Ile Ser Val Thr Pro Glu Ala Val
            260                 265                 270

Lys Asp Glu Asn Lys Lys Thr Ile Gly Arg Phe Gly Ser Tyr Ala Pro
        275                 280                 285

Thr Glu Lys Gly Val Leu Ser Ala Val Ala Tyr Gly Ala Thr Ser Thr
    290                 295                 300

Val Asp Val Thr Lys Ala Ile Leu Thr Asn Leu Ser Lys Leu Val Thr
305                 310                 315                 320

Gly Gln Phe Lys Leu Asp Met Leu Ser Gly Pro Val Gly Ile Tyr Asp
                325                 330                 335

Met Thr Asp Gln Val Ala Lys Thr Gly Ile Val Asn Leu Phe Gln Phe
            340                 345                 350

Ala Ala Phe Leu Ser Ile Asn Leu Gly Ile Val Asn Leu Leu Pro Ile
        355                 360                 365

Pro Ala Leu Asp Gly Gly Arg Leu Leu Phe Leu Phe Ile Glu Ala Ile
    370                 375                 380

Arg Gly Lys Pro Ile Asn Arg Glu Lys Glu Ala Phe Val Val Phe Ile
385                 390                 395                 400

Gly Val Ala Phe Leu Met Leu Leu Met Leu Val Val Thr Trp Asn Asp
                405                 410                 415

Ile Gln Arg Leu Phe Leu
            420
```

<210> 3
<211> 159
<212> DNA
<213> Artificial Sequence

<220>

<223> OLIGO

<400> 3

```
taagaaaagt gattctggga gagccgggat cactttttta tttaccttat gcccgaaatg        60
aaagctttat gacctaattg tgtaactata tcctattttt tcaaaaaata ttttaaaaac       120
gagcaggatt tcagaaaaaa tcgtggaatt gatacacta                              159
```

<210> 4
<211> 568
<212> DNA
<213> Bacillus subtilis

<400> 4

```
aattcctcca ttttcttctg ctatcaaaat aacagactcg tgattttcca aacgagcttt        60
caaaaaagcc tctgcccctt gcaaatcgga tgcctgtcta taaaattccc gatattggct       120
taaacagcgg cgcaatggcg gccgcatctg atgtctttgc ttggcgaatg ttcatcttat       180
ttcttcctcc ctctcaataa ttttttcatt ctatcccttt tctgtaaagt ttatttttca       240
gaatactttt atcatcatgc tttgaaaaaa tatcacgata atatccattg ttctcacgga       300
agcacacgca ggtcatttga acgaattttt tcgacaggaa tttgccggga ctcaggagca       360
tttaacctaa aaaagcatga catttcagca taatgaacat ttactcatgt ctattttcgt       420
tcttttctgt atgaaaatag ttatttcgag tctctacgga aatagcgaga gatgatatac       480
ctaaatagag ataaaatcat ctcaaaaaaa tgggtctact aaaatattat tccatctatt       540
acaataaata atactcacat aaggtggt                                          568
```

<210> 5
<211> 58
<212> DNA
<213> Bacillus subtilis

<400> 5
acagaatagt cttttaagta agtctactct gaattttttt aaaaggagag ggtaaaga 58

<210> 6
<211> 11
<212> PRT
<213> Artificial Sequence

<220>
<223> 12-amino acid residue active site consensus sequence, which aligns with amino acid residues 16-26 of SEQ ID NO: 2.

<220>
<221> ACT_SITE
<222> (1)..(11)
<223> 11-amin acod residue consensus sequence qhich aligns to amino acid residues 16-26 of SEQ ID NO: 2.

<400> 6

Leu Val Phe Phe His Glu Leu Gly His Leu Leu
1 5 10

<210> 7
<211> 5
<212> PRT
<213> Artificial Sequence

<220>
<223> Active site consnesus sequence, aligns to amino acid residues 20-24 of SEQ ID NO:2

<220>
<221> ACT_SITE
<222> (1)..(5)
<223> 5-amino acid consensus sequence which aligns with amino acid residues 20-24 of SEQ ID NO: 2.

<220>
<221> MISC_FEATURE
<222> (3)..(3)
<223> Xaa is any amino acid

<220>
<221> MISC_FEATURE
<222> (4)..(4)
<223> Xaa is any amino acid

<400> 7

His Glu Xaa Xaa His
1 5

<210> 8
<211> 83
<212> DNA
<213> Artificial Sequence

<220>
<223> oligo

<400> 8

cgagctcgaa ttcactggcc gtcgggatac gtcaattcaa tactcacata aggtggtacg 60

aaaagtaaat caatcagagg tgc 83

<210> 9
<211> 17
<212> DNA
<213> Artificial Sequence

<220>
<223> oligo

<400> 9
gatcgtacgg cgcaacg 17

<210> 10
<211> 19
<212> DNA
<213> Artificial Sequence

<220>
<223> oligo

<400> 10
gctcttcaag gcgaacagg 19

<210> 11
<211> 19
<212> DNA
<213> Artificial Sequence

<220>
<223> oligo

<400> 11
cttcaacgct aactttgag 19

<210> 12
<211> 20
<212> DNA
<213> Artificial Sequence

<220>
<223> oligo

<400> 12
ctgtccgttc cagtgtacgg 20

<210> 13
<211> 40
<212> DNA
<213> Artificial Sequence

<220>
<223> oligo

<400> 13
cgacctgcag gcatgcaagc tctcgctttc atcctttccg 40

<210> 14
<211> 78
<212> DNA
<213> Artificial Sequence

<220>
<223> oligo

<400> 14

**cgagctcgaa ttcactggcc gtcgcaaaga gaaactcgga aaggatgaaa gcgagttctt 60**

**tataccgtga tgcctcag 78**

<210> 15
<211> 26
<212> DNA
<213> Artificial Sequence

<220>
<223> oligo

<400> 15
ggtctgtcat tcaatttaga ctccag 26

<210> 16
<211> 15
<212> DNA
<213> Artificial Sequence

<220>
<223> oligo

<400> 16
cgcacgggca cgatg 15

<210> 17
<211> 1040
<212> DNA
<213> Bacillus subtilis

<400> 17

tcgctgaggt tctccctgaa tccatgaaaa acgccctttc tgaaataccg gaacaacaat 60
ggctggaaat cgaagaagtc agaattcgga tcaaccgccc tgttgaattg attcgaagag 120
gacaacctgt ttatttgtcc tatgcgggca cggccgagga tgcccatctg atcctgagcc 180
ggctcagcaa ctatagtatg tatacacttg aagaagagct gaagaaaggg tatgtcacga 240
tcagaggcgg ccacagagtc ggactggcgg gcagagtcat tacggaaaac ggaggtgtga 300
aaggcctccg tgacatcgca tcttttaata tacgaattgc ccggcaaaag ctggggattg 360
cggagccgct gcttccatac ttatatcaaa acagctggct gaatacgctg attatcggac 420
cgccccaaac cggcaaaaca acacttctcc gggaccttgc gaggctctca agcaccggca 480
aaaaaaacat gctgcccgta aagacaggga tcgtcgatga acggtcagag attgccggat 540
gtcttcgcgg tattccgcag catcaatttg gtcagagaat tgacgtgtta gacgcctgtc 600
caaaagctga agggctgatg atgatgattc gatccatgag ccctgaggtg atgattgtcg 660
atgaaatcgg gcgtatggaa gatacagatg cacttctgga ggcgctgcat gccggtgttt 720
cagtaatcgt atcggcgcat ggctggagta tatccgacct gatgaagcgg ccttcattga 780
agcgtttgtg ggaagaaaga gcctttgacc gctatttgga attatcaaga gcaaaagggc 840
cgggcacagt gagccaaatt tatgacaaag acggaaatgt gctgtctagg acgacgggcg 900
tgaaaacatg ctgaagctgc tgggcgctgt attcattgtg gtagcgacaa catggacagg 960
ttttgagatg gcgaagattt atacagaacg gccgcggcaa atccgccagc tgcgtgcggc 1020

gcttcaatcc ctcgaggctg 1040

<210> 18
<211> 52
<212> DNA
<213> Artificial Sequence

<220>
<223> oligo

<400> 18
cgagaaagga agggaagaaa gcgaaatcgc tgaggttctc cctgaatcca tg 52

<210> 19
<211> 22
<212> DNA
<213> Artificial Sequence

<220>
<223> oligo

<400> 19
cagcctcgag ggattgaagc gc 22

<210> 20
<211> 1131
<212> DNA
<213> Artificial Sequence

<220>
<223> SpcR

<400> 20

```
gcgcttcaat ccctcgaggc tgtcgacggt atcttattgc ggatccataa cttcgtataa      60
tgtatgctat acgaagttat ctagataaaa aatttagaag ccaatgaaat ctataaataa     120
actaaattaa gtttatttaa ttaacaacta tggatataaa ataggtacta atcaaaatag     180
tgaggaggat atatttgaat acatacgaac aaattaataa agtgaaaaaa atacttcgga     240
aacatttaaa aaataacctt attggtactt acatgtttgg atcaggagtt gagagtggac     300
taaaaccaaa tagtgatctt gactttttag tcgtcgtatc tgaaccattg acagatcaaa     360
gtaaagaaat acttatacaa aaaattagac ctatttcaaa aaaaatagga gataaaagca     420
acttacgata tattgaatta acaattatta ttcagcaaga aatggtaccg tggaatcatc     480
ctcccaaaca agaatttatt tatggagaat ggttacaaga gctttatgaa caaggataca     540
ttcctcagaa ggaattaaat tcagatttaa ccataatgct ttaccaagca aaacgaaaaa     600
ataaaagaat atacggaaat tatgacttag aggaattact acctgatatt ccattttctg     660
atgtgagaag agccattatg gattcgtcag aggaattaat agataattat caggatgatg     720
aaaccaactc tatattaact ttatgccgta tgattttaac tatggacacg ggtaaaatca     780
taccaaaaga tattgcggga aatgcagtgg ctgaatcttc tccattagaa catagggaga     840
gaattttgtt agcagttcgt agttatcttg gagagaatat tgaatggact aatgaaaatg     900
taaatttaac tataaactat ttaaataaca gattaaaaaa attataaaaa aattgaaaaa     960
atggtggaaa cactttttc aatttttttg ttttattatt taatatttgg gaaatattca     1020
ttctaattgg taatcagatt ttagaaaaca ataaaccctt gcatatgtct agataacttc    1080
gtataatgta tgctatacga agttatgcgg ccgccatatg catcctaggc c             1131
```

<210> 21
<211> 981
<212> DNA
<213> Bacillus subtilis

<400> 21

```
aagcttccca gcgcgcatat attctagaag aaatggctgt ccaactaaaa aagaaagcgg    60
aggagagatt cagtcatgat gaatataaag taggccgcat caaactcacg gcaggagaaa   120
aggtagattc agaagaggat attaaaacaa tcagcgtgta tatggccccg tcttctgaaa   180
aaacggtcca aacggtcgcg ccggtccaca ttgatacaga tcatgcatac gtaacaaaag   240
aagcagccga acaaaaagaa gccaaacaga tacaaacgca gctcgcggac atttgggaaa   300
tcggaagtga gaaaattacg gttcatatgg aaggcgggga gagtgtcggc aatgaataaa   360
aacggattat ggaatgtatt gaaaaagcag tttcttccgg gacaaacgaa ggacggcgaa   420
aagccgaagc tgaccaaata tcattacttt ctattcgttt ttgttctcgg agtttccttt   480
atgcttgtca gccagctgtt ttcttcacct gagaaaactg aaaacgccaa aacgataacg   540
gccgtatcat cacaacattc cgcagacagc aaagagaaaa cagccgaagt gtttaaagcc   600
tcaaaatctg ataaacctaa agactcgatc gatgactatg aaaaagaata cgaaaatcaa   660
ctgaaagaaa ttcttgaaac aatcattggt gtggacgatg tctcggttgt cgtgaatgta   720
gatgcaacgt cgctgaaagt atacgaaaaa aacaaatcaa acaaaaatac aaccactgaa   780
gaaactgaca aagaaggcgg aaaaagaagc gttacagacc aatcatcgga ggaagaaatc   840
gtcatgatca aaaacggcga taaagaaaca cctgtcgtcg tccaaacgaa aaaacctgat   900
atacgcggtg tactcgttgt tgctcaagga gtggacaacg ttcaaataaa acaaaccatt   960
atcgaagcgg tgacacgggt c                                              981
```

<210> 22
<211> 49
<212> DNA
<213> Artificial Sequence

<220>
<223> oligo

<400> 22
gcggccgcca tatgcatcct aggccaagct tcccagcgcg catatattc 49

<210> 23
<211> 50
<212> DNA
<213> Artificial Sequence

<220>
<223> oligo

<400> 23
tcattaggca ccccaggctt tacacgaccc gtgtcaccgc ttcgataatg 50

<210> 24
<211> 52
<212> DNA

<213> Artificial Sequence

<220>
<223> oligo

<400> 24
catggattca gggagaacct cagcgatttc gctttcttcc cttcctttct cg 52

<210> 25
<211> 50
<212> DNA
<213> Artificial Sequence

<220>
<223> oligo

<400> 25
cattatcgaa gcggtgacac gggtcgtgta aagcctgggg tgcctaatga 50

<210> 26
<211> 55
<212> DNA
<213> Artificial Sequence

<220>
<223> oligo

<400> 26
cagaaaaaat cgtggaattg atacactaca atactcacat aaggtggtat gttcg 55

<210> 27
<211> 51
<212> DNA
<213> Artificial Sequence

<220>
<223> oligo

<400> 27
ggacggaagt ccgggttatc ggtcgtttac aaaaacagcc gctggatatc g 51

<210> 28
<211> 22
<212> DNA
<213> Artificial Sequence

<220>
<223> oligo

<400> 28
cagcctcgag ggattgaagc gc 22

<210> 29
<211> 51
<212> DNA
<213> Artificial Sequence

<220>

<223> oligo

<400> 29
cgatatccag cggctgtttt tgtaaacgac cgataacccg gacttccgtc c 51

<210> 30
<211> 51
<212> DNA
<213> Artificial Sequence

<220>
<223> oligo

<400> 30
gatttataca gaacggccgc ggcaaatccg gaattcctcc attttcttct g 51

<210> 31
<211> 57
<212> DNA
<213> Artificial Sequence

<220>
<223> oligo

<400> 31
gataaacgct ataactgtat tcacgaacat accaccttat gtgagtatta tttattg 57

<210> 32
<211> 51
<212> DNA
<213> Artificial Sequence

<220>
<223> oligo

<400> 32
cagaagaaaa tggaggaatt ccggatttgc cgcggccgtt ctgtataaat c 51

<210> 33
<211> 57
<212> DNA
<213> Artificial Sequence

<220>
<223> oligo

<400> 33
caataaataa tactcacata aggtggtatg ttcgtgaata cagttatagc gtttatc 57

<210> 34
<211> 57
<212> DNA
<213> Artificial Sequence

<220>
<223> oligo

<400> 34

cagaaaaaat cgtggaattg atacactaac aaagagaaac tcggaaagga tgaaagc 57

<210> 35
<211> 53
<212> DNA
<213> Artificial Sequence

<220>
<223> oligo

<400> 35
ggacggaagt ccgggttatc ggtcgtacac aatttcctga ggcatcacgg tat 53

<210> 36
<211> 57
<212> DNA
<213> Artificial Sequence

<220>
<223> oligo

<400> 36
gctttcatcc tttccgagtt tctctttgtt agtgtatcaa ttccacgatt ttttctg 57

<210> 37
<211> 53
<212> DNA
<213> Artificial Sequence

<220>
<223> oligo

<400> 37
ataccgtgat gcctcaggaa attgtgtacg accgataacc cggacttccg tcc 53

<210> 38
<211> 483
<212> PRT
<213> Bacillus licheniformis

<400> 38

```
Ala Asn Leu Asn Gly Thr Leu Met Gln Tyr Phe Glu Trp Tyr Met Pro
1               5                   10                  15

Asn Asp Gly Gln His Trp Lys Arg Leu Gln Asn Asp Ser Ala Tyr Leu
            20                  25                  30

Ala Glu His Gly Ile Thr Ala Val Trp Ile Pro Pro Ala Tyr Lys Gly
        35                  40                  45

Thr Ser Gln Ala Asp Val Gly Tyr Gly Ala Tyr Asp Leu Tyr Asp Leu
    50                  55                  60

Gly Glu Phe His Gln Lys Gly Thr Val Arg Thr Lys Tyr Gly Thr Lys
65                  70                  75                  80

Gly Glu Leu Gln Ser Ala Ile Lys Ser Leu His Ser Arg Asp Ile Asn
                85                  90                  95

Val Tyr Gly Asp Val Val Ile Asn His Lys Gly Gly Ala Asp Ala Thr
            100                 105                 110

Glu Asp Val Thr Ala Val Glu Val Asp Pro Ala Asp Arg Asn Arg Val
        115                 120                 125

Ile Ser Gly Glu His Leu Ile Lys Ala Trp Thr His Phe His Phe Pro
    130                 135                 140

Gly Arg Gly Ser Thr Tyr Ser Asp Phe Lys Trp His Trp Tyr His Phe
145                 150                 155                 160

Asp Gly Thr Asp Trp Asp Glu Ser Arg Lys Leu Asn Arg Ile Tyr Lys
                165                 170                 175

Phe Gln Gly Lys Ala Trp Asp Trp Glu Val Ser Asn Glu Asn Gly Asn
            180                 185                 190

Tyr Asp Tyr Leu Met Tyr Ala Asp Ile Asp Tyr Asp His Pro Asp Val
        195                 200                 205

Ala Ala Glu Ile Lys Arg Trp Gly Thr Trp Tyr Ala Asn Glu Leu Gln
    210                 215                 220

Leu Asp Gly Phe Arg Leu Asp Ala Val Lys His Ile Lys Phe Ser Phe
225                 230                 235                 240

Leu Arg Asp Trp Val Asn His Val Arg Glu Lys Thr Gly Lys Glu Met
                245                 250                 255
```

Phe Thr Val Ala Glu Tyr Trp Gln Asn Asp Leu Gly Ala Leu Glu Asn
260 265 270

Tyr Leu Asn Lys Thr Asn Phe Asn His Ser Val Phe Asp Val Pro Leu
275 280 285

His Tyr Gln Phe His Ala Ala Ser Thr Gln Gly Gly Gly Tyr Asp Met
290 295 300

Arg Lys Leu Leu Asn Gly Thr Val Val Ser Lys His Pro Leu Lys Ser
305 310 315 320

Val Thr Phe Val Asp Asn His Asp Thr Gln Pro Gly Gln Ser Leu Glu
325 330 335

Ser Thr Val Gln Thr Trp Phe Lys Pro Leu Ala Tyr Ala Phe Ile Leu
340 345 350

Thr Arg Glu Ser Gly Tyr Pro Gln Val Phe Tyr Gly Asp Met Tyr Gly
355 360 365

Thr Lys Gly Asp Ser Gln Arg Glu Ile Pro Ala Leu Lys His Lys Ile
370 375 380

Glu Pro Ile Leu Lys Ala Arg Lys Gln Tyr Ala Tyr Gly Ala Gln His
385 390 395 400

Asp Tyr Phe Asp His His Asp Ile Val Gly Trp Thr Arg Glu Gly Asp
405 410 415

Ser Ser Val Ala Asn Ser Gly Leu Ala Ala Leu Ile Thr Asp Gly Pro
420 425 430

Gly Gly Ala Lys Arg Met Tyr Val Gly Arg Gln Asn Ala Gly Glu Thr
435 440 445

Trp His Asp Ile Thr Gly Asn Arg Ser Glu Pro Val Val Ile Asn Ser
450 455 460

Glu Gly Trp Gly Glu Phe His Val Asn Gly Gly Ser Val Ser Ile Tyr
465 470 475 480

Val Gln Arg

<210> 39
<211> 478
<212> PRT

<213> Paenibacillus curdlanolyticus

<400> 39

```
Ala Asp Asn Gly Thr Ile Met Gln Tyr Phe Glu Trp Tyr Leu Pro Asn
1               5                   10                  15

Asp Gly Ala His Trp Asn Arg Leu Asn Asn Asp Ala Gln Asn Leu Lys
            20                  25                  30

Asn Val Gly Ile Thr Ala Val Trp Ile Pro Pro Ala Tyr Lys Gly Gly
        35                  40                  45

Ser Ser Ala Asp Val Gly Tyr Gly Val Tyr Asp Thr Tyr Asp Leu Gly
    50                  55                  60

Glu Phe Asn Gln Lys Gly Thr Val Arg Thr Lys Tyr Gly Thr Lys Ser
65                  70                  75                  80

Glu Leu Ile Ser Ala Val Asn Asn Leu His Ala Lys Gly Ile Ala Val
                85                  90                  95

Tyr Gly Asp Val Val Leu Asn His Arg Met Asn Ala Asp Ala Thr Glu
            100                 105                 110

Leu Val Asp Ala Val Glu Val Asp Pro Asn Asn Arg Tyr Val Glu Thr
        115                 120                 125

Thr Ser Thr Tyr Gln Ile Gln Ala Trp Thr Gln Tyr Asp Phe Pro Gly
    130                 135                 140

Arg Gly Asn Thr Tyr Ser Ser Trp Lys Trp Arg Trp Tyr His Phe Asp
145                 150                 155                 160

Gly Val Asp Trp Asp Gln Ser Arg Gly Leu Asn Arg Ile Tyr Lys Leu
                165                 170                 175

Asp Gly Lys Asp Trp Asp Trp Pro Val Asp Ser Glu Tyr Gly Asn Tyr
            180                 185                 190

Asp Tyr Leu Met Gly Ala Asp Leu Asp Phe Asn His Pro Asp Val Val
        195                 200                 205

Asn Glu Thr Lys Thr Trp Gly Lys Trp Phe Val Asn Thr Val Asn Leu
    210                 215                 220

Asp Gly Val Arg Leu Asp Ala Val Lys His Ile Lys Phe Asp Phe Met
```

225 230 235 240

Arg Asp Trp Val Asn Asn Val Arg Ser Thr Thr Gly Lys Asn Leu Phe
245 250 255

Ala Val Gly Glu Tyr Trp His Tyr Asp Val Asn Lys Leu Asn Ser Tyr
260 265 270

Ile Thr Lys Thr Asn Gly Thr Met Ser Leu Phe Asp Val Pro Leu His
275 280 285

Phe Arg Phe Tyr Asp Ala Ser Asn Gly Gly Gly Gly Tyr Asp Met Arg
290 295 300

Asn Leu Leu Asn Asn Thr Leu Met Ser Ser Asn Pro Met Lys Ala Val
305 310 315 320

Thr Phe Val Glu Asn His Asp Thr Gln Pro Gly Gln Ser Leu Glu Ser
325 330 335

Thr Val Gln Ser Trp Phe Lys Pro Leu Ala Tyr Ala Thr Ile Leu Thr
340 345 350

Arg Glu Gln Gly Tyr Pro Cys Val Phe Tyr Gly Asp Tyr Tyr Gly Thr
355 360 365

Ser Asp Gly Lys Ile Ser Ser Tyr Lys Pro Ile Met Asp Lys Leu Leu
370 375 380

Asn Ala Arg Lys Val Tyr Ala Tyr Gly Thr Gln Arg Asp Tyr Phe Asp
385 390 395 400

His Pro Asp Ile Val Gly Trp Thr Arg Glu Gly Asp Ala Ala His Ala
405 410 415

Gly Ser Gly Leu Ala Thr Leu Ile Thr Asp Gly Pro Gly Gly Ser Lys
420 425 430

Trp Met Tyr Val Gly Thr Ser Lys Ala Gly Gln Val Trp Thr Asp Lys
435 440 445

Thr Gly Asn Arg Ser Gly Thr Val Thr Ile Asp Ala Asn Gly Trp Gly
450 455 460

Asn Phe Trp Val Asn Lys Gly Ser Val Ser Val Trp Ala Lys
465 470 475

<210> 40
<211> 425
<212> PRT
<213> Bacillus subtilis

<400> 40

```
Leu Thr Ala Pro Ser Ile Lys Ser Gly Thr Ile Leu His Ala Trp Asn
1               5                   10                  15

Trp Ser Phe Asn Thr Leu Lys His Asn Met Lys Asp Ile His Asp Ala
            20                  25                  30

Gly Tyr Thr Ala Ile Gln Thr Ser Pro Ile Asn Gln Val Lys Glu Gly
        35                  40                  45

Asn Gln Gly Asp Lys Ser Met Ser Asn Trp Tyr Trp Leu Tyr Gln Pro
    50                  55                  60

Thr Ser Tyr Gln Ile Gly Asn Arg Tyr Leu Gly Thr Glu Gln Glu Phe
65                  70                  75                  80

Lys Glu Met Cys Ala Ala Ala Glu Glu Tyr Gly Ile Lys Val Ile Val
                85                  90                  95

Asp Ala Val Ile Asn His Thr Thr Ser Asp Tyr Ala Ala Ile Ser Asn
            100                 105                 110

Glu Val Lys Ser Ile Pro Asn Trp Thr His Gly Asn Thr Gln Ile Lys
        115                 120                 125

Asn Trp Ser Asp Arg Trp Asp Val Thr Gln Asn Ser Leu Leu Gly Leu
    130                 135                 140

Tyr Asp Trp Asn Thr Gln Asn Thr Gln Val Gln Ser Tyr Leu Lys Arg
145                 150                 155                 160

Phe Leu Asp Arg Ala Leu Asn Asp Gly Ala Asp Gly Phe Arg Phe Asp
                165                 170                 175

Ala Ala Lys His Ile Glu Leu Pro Asp Asp Gly Ser Tyr Gly Ser Gln
            180                 185                 190

Phe Trp Pro Asn Ile Thr Asn Thr Ser Ala Glu Phe Gln Tyr Gly Glu
        195                 200                 205

Ile Leu Gln Asp Ser Ala Ser Arg Asp Ala Ala Tyr Ala Asn Tyr Met
    210                 215                 220
```

Asp Val Thr Ala Ser Asn Tyr Gly His Ser Ile Arg Ser Ala Leu Lys
225 230 235 240

Asn Arg Asn Leu Gly Val Ser Asn Ile Ser His Tyr Ala Ser Asp Val
245 250 255

Ser Ala Asp Lys Leu Val Thr Trp Val Glu Ser His Asp Thr Tyr Ala
260 265 270

Asn Asp Asp Glu Glu Ser Thr Trp Met Ser Asp Asp Asp Ile Arg Leu
275 280 285

Gly Trp Ala Val Ile Ala Ser Arg Ser Gly Ser Thr Pro Leu Phe Phe
290 295 300

Ser Arg Pro Glu Gly Gly Gly Asn Gly Val Arg Phe Pro Gly Lys Ser
305 310 315 320

Gln Ile Gly Asp Arg Gly Ser Ala Leu Phe Glu Asp Gln Ala Ile Thr
325 330 335

Ala Val Asn Arg Phe His Asn Val Met Ala Gly Gln Pro Glu Glu Leu
340 345 350

Ser Asn Pro Asn Gly Asn Asn Gln Ile Phe Met Asn Gln Arg Gly Ser
355 360 365

His Gly Val Val Leu Ala Asn Ala Gly Ser Ser Ser Val Ser Ile Asn
370 375 380

Thr Ala Thr Lys Leu Pro Asp Gly Arg Tyr Asp Asn Lys Ala Gly Ala
385 390 395 400

Gly Ser Phe Gln Val Asn Asp Gly Lys Leu Thr Gly Thr Ile Asn Ala
405 410 415

Arg Ser Val Ala Val Leu Tyr Pro Asp
420 425

<210> 41
<211> 470
<212> PRT
<213> Bacillus subtilis

<400> 41

Ala Gly Thr Lys Thr Pro Val Ala Lys Asn Gly Gln Leu Ser Ile Lys
1 5 10 15

```
Gly Thr Gln Leu Val Asn Arg Asp Gly Lys Ala Val Gln Leu Lys Gly
            20                  25                  30

Ile Ser Ser His Gly Leu Gln Trp Tyr Gly Glu Tyr Val Asn Lys Asp
        35                  40                  45

Ser Leu Lys Trp Leu Arg Asp Asp Trp Gly Ile Thr Val Phe Arg Ala
    50                  55                  60

Ala Met Tyr Thr Ala Asp Gly Gly Tyr Ile Asp Asn Pro Ser Val Lys
65                  70                  75                  80

Asn Lys Val Lys Glu Ala Val Glu Ala Ala Lys Glu Leu Gly Ile Tyr
                85                  90                  95

Val Ile Ile Asp Trp His Ile Leu Asn Asp Gly Asn Pro Asn Gln Asn
            100                 105                 110

Lys Glu Lys Ala Lys Glu Phe Phe Lys Glu Met Ser Ser Leu Tyr Gly
        115                 120                 125

Asn Thr Pro Asn Val Ile Tyr Glu Ile Ala Asn Glu Pro Asn Gly Asp
    130                 135                 140

Val Asn Trp Lys Arg Asp Ile Lys Pro Tyr Ala Glu Glu Val Ile Ser
145                 150                 155                 160

Val Ile Arg Lys Asn Asp Pro Asp Asn Ile Ile Ile Val Gly Thr Gly
                165                 170                 175

Thr Trp Ser Gln Asp Val Asn Asp Ala Ala Asp Asp Gln Leu Lys Asp
            180                 185                 190

Ala Asn Val Met Tyr Ala Leu His Phe Tyr Ala Gly Thr His Gly Gln
        195                 200                 205

Phe Leu Arg Asp Lys Ala Asn Tyr Ala Leu Ser Lys Gly Ala Pro Ile
    210                 215                 220

Phe Val Thr Glu Trp Gly Thr Ser Asp Ala Ser Gly Asn Gly Gly Val
225                 230                 235                 240

Phe Leu Asp Gln Ser Arg Glu Trp Leu Lys Tyr Leu Asp Ser Lys Thr
                245                 250                 255

Ile Ser Trp Val Asn Trp Asn Leu Ser Asp Lys Gln Glu Ser Ser Ser
            260                 265                 270
```

Ala Leu Lys Pro Gly Ala Ser Lys Thr Gly Gly Trp Arg Leu Ser Asp
275 280 285

Leu Ser Ala Ser Gly Thr Phe Val Arg Glu Asn Ile Leu Gly Thr Lys
290 295 300

Asp Ser Thr Lys Asp Ile Pro Glu Thr Pro Ser Lys Asp Lys Pro Thr
305 310 315 320

Gln Glu Asn Gly Ile Ser Val Gln Tyr Arg Ala Gly Asp Gly Ser Met
325 330 335

Asn Ser Asn Gln Ile Arg Pro Gln Leu Gln Ile Lys Asn Asn Gly Asn
340 345 350

Thr Thr Val Asp Leu Lys Asp Val Thr Ala Arg Tyr Trp Tyr Lys Ala
355 360 365

Lys Asn Lys Gly Gln Asn Phe Asp Cys Asp Tyr Ala Gln Ile Gly Cys
370 375 380

Gly Asn Val Thr His Lys Phe Val Thr Leu His Lys Pro Lys Gln Gly
385 390 395 400

Ala Asp Thr Tyr Leu Glu Leu Gly Phe Lys Asn Gly Thr Leu Ala Pro
405 410 415

Gly Ala Ser Thr Gly Asn Ile Gln Leu Arg Leu His Asn Asp Asp Trp
420 425 430

Ser Asn Tyr Ala Gln Ser Gly Asp Tyr Ser Phe Phe Lys Ser Asn Thr
435 440 445

Phe Lys Thr Thr Lys Lys Ile Thr Leu Tyr Asp Gln Gly Lys Leu Ile
450 455 460

Trp Gly Thr Glu Pro Asn
465 470

<210> 42
<211> 269
<212> PRT
<213> Bacillus clausii

<400> 42

Ala Gln Ser Val Pro Trp Gly Ile Ser Arg Val Gln Ala Pro Ala Ala
1 5 10 15

```
His Asn Arg Gly Leu Thr Gly Ser Gly Val Lys Val Ala Val Leu Asp
            20                  25                  30

Thr Gly Ile Ser Thr His Pro Asp Leu Asn Ile Arg Gly Gly Ala Ser
        35                  40                  45

Phe Val Pro Gly Glu Pro Ser Thr Gln Asp Gly Asn Gly His Gly Thr
    50                  55                  60

His Val Ala Gly Thr Ile Ala Ala Leu Asn Asn Ser Ile Gly Val Leu
65                  70                  75                  80

Gly Val Ala Pro Asn Ala Glu Leu Tyr Ala Val Lys Val Leu Gly Ala
                85                  90                  95

Ser Gly Gly Gly Ser Asn Ser Ser Ile Ala Gln Gly Leu Glu Trp Ala
            100                 105                 110

Gly Asn Asn Gly Met His Val Ala Asn Leu Ser Leu Gly Ser Pro Ser
        115                 120                 125

Pro Ser Ala Thr Leu Glu Gln Ala Val Asn Ser Ala Thr Ser Arg Gly
    130                 135                 140

Val Leu Val Val Ala Ala Ser Gly Asn Ser Gly Ala Gly Ser Ile Ser
145                 150                 155                 160

Tyr Pro Ala Arg Tyr Ala Asn Ala Met Ala Val Gly Ala Thr Asp Gln
                165                 170                 175

Asn Asn Asn Arg Ala Ser Phe Ser Gln Tyr Gly Ala Gly Leu Asp Ile
            180                 185                 190

Val Ala Pro Gly Val Asn Val Gln Ser Thr Tyr Pro Gly Ser Thr Tyr
        195                 200                 205

Ala Ser Leu Asn Gly Thr Ser Met Ala Thr Pro His Val Ala Gly Ala
    210                 215                 220

Ala Ala Leu Val Lys Gln Lys Asn Pro Ser Trp Ser Asn Val Gln Ile
225                 230                 235                 240

Arg Asn His Leu Lys Asn Thr Ala Thr Ser Leu Gly Ser Thr Asn Leu
                245                 250                 255

Tyr Gly Ser Gly Leu Val Asn Ala Glu Ala Ala Thr Arg
            260                 265
```

<210> 43
<211> 269
<212> PRT
<213> Bacillus amyloliquefaciens

<400> 43

```
Ala Gln Ser Val Pro Trp Gly Ile Ser Arg Val Gln Ala Pro Ala Ala
1               5                   10                  15

His Asn Arg Gly Leu Thr Gly Ser Gly Val Lys Val Ala Val Leu Asp
            20                  25                  30

Thr Gly Ile Ser Thr His Pro Asp Leu Asn Ile Arg Gly Gly Ala Ser
        35                  40                  45

Phe Val Pro Gly Glu Pro Ser Thr Gln Asp Gly Asn Gly His Gly Thr
    50                  55                  60

His Val Ala Gly Thr Ile Ala Ala Leu Asn Asn Ser Ile Gly Val Leu
65                  70                  75                  80

Gly Val Ala Pro Asn Ala Glu Leu Tyr Ala Val Lys Val Leu Gly Ala
                85                  90                  95

Ser Gly Gly Gly Ser Asn Ser Ser Ile Ala Gln Gly Leu Glu Trp Ala
            100                 105                 110

Gly Asn Asn Gly Met His Val Ala Asn Leu Ser Leu Gly Ser Pro Ser
        115                 120                 125

Pro Ser Ala Thr Leu Glu Gln Ala Val Asn Ser Ala Thr Ser Arg Gly
    130                 135                 140

Val Leu Val Val Ala Ala Ser Gly Asn Ser Gly Ala Gly Ser Ile Ser
145                 150                 155                 160

Tyr Pro Ala Arg Tyr Ala Asn Ala Met Ala Val Gly Ala Thr Asp Gln
                165                 170                 175

Asn Asn Asn Arg Ala Ser Phe Ser Gln Tyr Gly Ala Gly Leu Asp Ile
            180                 185                 190

Val Ala Pro Gly Val Asn Val Gln Ser Thr Tyr Pro Gly Ser Thr Tyr
        195                 200                 205

Ala Ser Leu Asn Gly Thr Ser Met Ala Thr Pro His Val Ala Gly Ala
    210                 215                 220
```

```
Ala Ala Leu Val Lys Gln Lys Asn Pro Ser Trp Ser Asn Val Gln Ile
225                 230                 235                 240

Arg Asn His Leu Lys Asn Thr Ala Thr Ser Leu Gly Ser Thr Asn Leu
                245                 250                 255

Tyr Gly Ser Gly Leu Val Asn Ala Glu Ala Ala Thr Arg
            260                 265
```

<210> 44
<211> 20
<212> DNA
<213> Artificial Sequence

<220>
<223> oligo

<400> 44
cgcctcatca ttacggcatc 20

<210> 45
<211> 48
<212> DNA
<213> Artificial Sequence

<220>
<223> oligo

<400> 45
cgacctgcag gcatgcaagc taccacctta tgtgagtatt gaattgac 48

<210> 46
<211> 29
<212> PRT
<213> Bacillus subtilis

<400> 46

```
Met Arg Ser Lys Lys Leu Trp Ile Ser Leu Leu Phe Ala Leu Thr Leu
1               5                   10                  15

Ile Phe Thr Met Ala Phe Ser Asn Met Ser Ala Gln Ala
            20                  25
```